# EUROPEAN PATENT APPLICATION

(11) **EP 1 266 961 A1**
(43) Date of publication of application: **18.12.2002**
(21) Application number: 01912419.7
(22) Date of filing: 16.03.2001
(51) Int. Cl.: C12N 5/12

(54) **KIDNEY REPAIRING FACTOR**

(30) Priority: 16.03.2000 JP 2000073632
(71) Applicant: Kyowa Hakko Kogyo Co., Ltd, Tokyo 100-8185 (JP)
(72) Inventor: TAKEUCHI, Kyoko, Tokyo Research Laboratories, Machida-shi, Tokyo 194-8533 (JP); KATO, Yoko, Tokyo Research Laboratories, Machida-shi, Tokyo 194-8533 (JP); SEKINE, Susumu, Tokyo Research Laboratories, Machida-shi, Tokyo 194-8533 (JP); KIKUCHI, Yasuhiro, Head Office, Chiyoda-ku, Tokyo 100-8185 (JP); SAKURADA, Kazuhiro, Tokyo Research Laboratories, Machida-shi, Tokyo 194-8533 (JP)
(74) Representative: VOSSIUS & PARTNER
(86) International application number: JP0102087
(87) International publication number: WO01068817

(57) **Abstract**

A protein [kidney regeneration factor (KRGF-1)] whose expression is increased at the time of regeneration of damaged tissues in patients with renal diseases is isolated, and a DNA encoding the protein and an antibody recognizing the protein are produced. These materials can be utilized in screening of therapeutic agents for regenerating damaged renal tissues and in diagnosis of renal damage.

## Description

### Technical Field

The present invention relates to a novel protein, a DNA encoding the protein, an antibody recognizing the protein, and a diagnostic agent, a medicine and a therapeutic agent comprising the same.

### Background Art

The kidney has a high reserve functions, and in many cases even when the remaining functions are half the normal functions, symptoms due to functional disorder are not observed. Damage of nephron composed of highly differentiated cell groups is irreversible, and degeneration of tissue structure beginning in glomerulosclerosis is accompanied by tubular disorder and stromal fibrosis and ultimately results in a serious condition of renal failure which requires kidney dialysis. It is generally thought that this process is not related to the type of the primary disease, and is roughly among the diseases. In clinical practice, for the main purpose of reducing the burden on the remaining nephron and of extending the period until the introduction of dialysis, there are employed administration of steroid agents, oral absorbents, antihypertensive agents, angiotensin converting enzyme (abbreviated hereinafter to ACE) inhibitors and the like, low-protein diet treatment method and the like. However, there are many unknown points regarding the mechanism of onset and progress of renal failure, and a method for basic remedy has not been established.

In proliferative glomerulonephritis in children and some animal models, it is known that spontaneous recovery occurs without progressing towards continuous decreasing of renal functions after the glomerulus or renal tubule is damaged, however, the mechanism of this spontaneous recovery is also not clarified. Analysis at the molecular level of the pathologic progression of proliferative glomerulonephritis or the mechanism of spontaneous recovery is considered to be important for diagnosis of renal diseases and development of therapeutic agents. An effective means for this purpose is, for example, comprehensively obtaining and analyzing of a group of genes whose expression level changes in accordance with the progress of a renal disease and recovery therefrom. It is not practical to conduct such an analysis by using actual tissues of renal disease patients in respect of obtainment of the tissues and non-uniformity of symptoms among the patients. It is presumed that by using a suitable model animal of proliferative glomerulonephritis, obtainment of comprehensive group of genes and analysis at the molecular level can be relatively easily conducted. It is considered that, in principle, genes obtained in this manner include factors which are markers for progression of pathology or recovery therefrom, as well as factors which actively promote recovery.

Several experimental models are known as model animals of nephritis, which are mainly rats . Among these models, as for Thy-1 nephritis model [Laboratory Investigation, 55, 680 (1986)] obtained by intravenously administered into a rat an antibody (anti-Thy-1 antibody) against Thy-1.1 antigen which is present as a membrane protein of mesangial cells (hereinafter, this nephritis model rat is referred to as "Thy-1 nephritis rat"), a considerable amount of analysis in the progression of pathology and in pathological findings has been conducted. In the Thy-1 nephritis rat, after mesangiolysis, renal tubular damage accompanying inflammatory cell infiltration to the stroma is observed, and then proliferation of mesangial cells and production of extracellular matrix occur. It is known that reconstitution of damaged tissue thereafter occurs, and spontaneous recovery thereof is got in days beyond 2 weeks. Therefore, the Thy-1 nephritis rat is considered to be suitable as a model of progression of symptoms of proliferative glomerulonephritis and spontaneous recovery therefrom.

There have already been several reports on analysis of the kidney condition of the Thy-1 nephritis rat at the molecular level , for example, analyses regarding genes whose expression level changes depending on the kidney condition. Specifically, there are reports regarding genes for growth factors such as transforming growth factor (abbreviated hereinafter to TGF-β), which is considered to be involved in proliferation of mesangial cells [J. Clin. Invest., 86, 453 (1990)], heparin-binding epidermal growth factor (EGF)-like growth factor [Experimental Nephrology, 4, 271 (1996)], platelet-derived growth factor (abbreviated hereinafter to PDGF) [Proc. Natl. Acad. Sci. USA, 88, 6560 (1991)] and fibroblast growth factor (abbreviated hereinafter to FGF) [J. Clin. Invest., 90, 2362 (1992)] or regarding genes for extracellular matrix-related proteins such as type IV collagen [Kidney International, 86, 453 (1990)], laminin [Kidney International, 86, 453 (1990)], tenascin [Experimental Nephrology, 5, 423 (1997)], profilin and CD44 [J. American Society of Nephrology, 7, 1006 (1996)].

Since growth factors such as TGF-β and PDGF in glomerulonephritis show high level of expression even in human glomerulonephritis such as lupus nephritis or IgA nephropathy as in experimental animals, these growth factors are considered to work as mediators of renal failure progression through proliferation response of mesangial cells, stimulation of production of extracellular matrix and the like [Pediatric Nephrology, 9, 495 (1995)]. It is reported that administration of PDGF-neutralizing antibody [J. Exp. Med., 175, 1413 (1992)] or an inhibitor of TGF-β, decorin [Nat. Med., 2, 418 (1996)] is effective in the Thy-1 nephritis rat, but the effect of these factors is not verified at the clinical level.

Development of a kidney protecting agent by using factors involved in kidney development has been studied by Creative BioMolecules Inc. in the US, and osteogenic protein-1 (abbreviated hereinafter to OP-1) [bone morphogenetic protein (abbreviated hereinafter to BMP) 7] has been provided. This protein is a factor belonging to BMP subfamily within TGF-β superfamily, which factor was first discovered as a factor which induces ectopic bone formation [EMBOJ., 9, 2085(1990)]. This factor is expressed in mesenchymal cells surrounding ureteric bud at a time of nephrogenesis in the fetal period, and is an important factor for interaction between the epithelium and mesenchyme. Further, it has recently been reported that this factor enables proliferation and differentiation of nephrogenic tissue by suppressing apoptosis in mesenchymal cells [Genes & Dev., 13, 1601 (1999)].

Results of a test in which the recombinant OP-1 protein provided by Creative Biomolecules Inc. was administered to an animal model of chronic renal failure were reported in an annual meeting of American Society of Nephrology [Nikkei Biotech, November 10, 1999].

In that preclinical test which was conducted by the Department of Medicine of Washington University (U.S.), rats model of chronic renal failure in which unilateral ureteral obstruction was induced was used. This model shows progressive fibrogenesis and renal damage, which are very similar to the cicatrisation as observed in chronic renal failure patients. The results of the test indicate that OP-1 suppresses the formation of cicatricial tissue in the kidney and decrease the tubular damage. Further, in the group administered with OP-1, approximately 30% of the filtration function of a normal kidney and 65% of the blood flow of a kidney at normal times were maintained, showing that OP-1 also has the effect of protecting renal functions. In the groups administered with a placebo or an ACE inhibitor, filtration functions and blood flow could not be determined. Tubulointerstitial lesion is more closely correlated with a decrease in kidney functions than glomerular lesion, and is also a representative tissue lesion from which prognosis of the disease is predictable. Preservation of the renal tubular structure was observed in only the OP-1-administered group. While the mechanism of tissue protection in the kidney is currently still not clear, the foregoing results suggest that cell groups that are the target of OP-1 are present even in the adult kidney, as in the nascent mesenchymal cells. However, various sites of action other than the kidney also exist for OP-1, and in particular, chondrogenic activity is thought to be one of the serious side effects of OP-1, and thus its clinical application is difficult to achieve. Accordingly, there is a need to find a factor which is expressed specifically in the kidney, and capable of inducing OP-1 or controlling the regenerative function of the kidney downstream of OP-1.

Autotaxin is known as a molecule related to cell differentiation and migration, which is induced by factors belonging to BMP or TGF-β families such as OP-1 in mesenchymal tissues. Autotaxin was isolated and cloned from a culture supernatant of a human melanoma cell line as a cytokine having a cancer cell migration activity [J. Biol. Chem., 267, 683 (1996)]. Thereafter, it has been showed that autotaxin is, in the nascent period, expressed in mesenchymal cells which are being under differentiation to bone cells and cartilage cells [Mechanisms of Dev., 84, 121 (1999)], and also that the expression is increased by simulating the precursor cells of bone and cartilage with BMP2 [Dev. Dynam., 213, 398 (1998)]. Autotaxin is classified as a plasma cell antigen PC-1 (abbreviated hereinafter to PC-1) family because of the structural homology. PC-1 family is composed of 3 kinds of type II membrane proteins: PC-1, PD-1α (autotaxin) and PD-1β (B10), each having the activity of phosphodiesterase I; EC 3.1.4.1 / nucleotide pyrophosphatase; EC 3.6.1.9 extracelluarly, and also having autophosphorylation ability. Such findings suggest that PC-1 family is expressed by stimulation of a factor belonging to BMP family, and plays an important role in cell migration, differentiation or intercellular interaction.

### Disclosure of Invention

The object of the present invention is to provide a protein useful for screening of therapeutic agents repairing damaged tissues in renal diseases, a DNA encoding the protein, an antibody recognizing the protein, and a method of using the same by obtaining a gene whose expression is varied depending on recovery from a morbid state in a rat model of proliferative glomerulonephritis or in human patients with renal diseases.

The present inventors extensively studied the problem described above, to complete the present invention.

That is, the present invention provides the following (1) to (40):
(1) A protein comprising an amino acid sequence represented by SEQ ID NO:1 or 15.
(2) A protein comprising an amino acid sequence wherein one or more amino acids are deleted, substituted or added in the amino acid sequence represented by SEQ ID NO; 1 or 15, and having a kidney-regenerating activity.
(3) A protein comprising an amino acid sequence having at least 60% identity to the amino acid sequence represented by SEQ ID NO: 1 or 15, and having a kidney-regenerating activity.
(4) A glycoprotein comprising sugar chains added to the protein according to any one of (1) to (3).
(5) A DNA encoding the protein of any one of (1) to (4).
(6) A DNA comprising a nucleotide sequence represented by SEQ ID NO:2, 3, 16 or 17.
(7) A DNA which hybridizes with the DNA according to (5) or (6) under stringent conditions and is expressed at a higher level in tissues where inflammatory diseases occur.
(8) A DNA having the same nucleotide sequence as that of consecutive 10 to 60 consecutive nucleotides in the DNA according to any one of (5) to (7).
(9) A DNA having a nucleotide sequence complementary to the DNA according to any one of (5) to (7).
(10) A method of detecting and quantifying the expression of a gene encoding the protein according to any one of (1) to (4), which comprises using the DNA according to any one of (5) to (9).
(11) A method of detecting a mutation in a gene encoding the protein according to any one of (1) to (4), which comprises using the DNA according to any one of (5) to (9).
(12) A method of obtaining a promoter region of a gene encoding the protein according to any one of (1) to (4), which comprises using the DNA according to any one of items (5) to (9).
(13) A diagnostic agent for renal diseases, which comprises the DNA according to any one of (5) to (9).
(14) A method of detecting a gene involved in progress of a renal disease or recovery therefrom, which comprises using the DNA according to any one of (5) to (9).
(15) A method of screening a substance inhibiting or promoting the transcription or translation of a gene encoding the protein according to any one of (1) to (4), which comprises using the DNA according to any one of (5) to (9).
(16) A method of screening a therapeutic agent for renal diseases, which comprises using the DNA according to any one of (5) to (9).
(17) A recombinant vector which is obtained by inserting the DNA according to any one of (5) to (9) into a vector.
(18) A recombinant vector which is obtained by inserting an RNA having a sequence homologous to the DNA according to any one of (5) to (9) into a vector.
(19) The recombinant vector according to (18), wherein the RNA is a single-stranded sense or antisense chain.
(20) A transformant obtained by introducing the recombinant vector according to (17) into a host cell.
(21) A process for producing a protein, which comprises culturing the transformant according to (20) in a medium to produce and accumulate the protein according to any one of (1) to (4) in the culture, and recovering the protein therefrom.
(22) A method of screening a therapeutic agent for renal diseases, which comprises using a culture obtained by the transformant according to (20) in a medium, said culture comprising the protein according to any one of (1) to (4).
(23) A method of screening a therapeutic agent for renal diseases, which comprises using the protein according to any one of (1) to (4).
(24) A therapeutic agent for renal diseases, which comprises the protein according to any one of (1) to (4).
(25) A therapeutic agent for renal diseases, which comprises the recombinant vector according to any one of (17) to (19).
(26) An antibody recognizing the protein according to any one of (1) to (4).
(27) A monoclonal antibody recognizing the protein according to any one of (1) to (4).
(28) The monoclonal antibody according to (27), wherein the monoclonal antibody is KM2954, KM2955, KM2956, KM2957 or KM2958.
(29) A hybridoma producing the monoclonal antibody according to (27) or (28).
(30) The hybridoma according to (29), wherein the hybridoma is hybridoma KM2955 (FERM BP-xxxx).
(31) A method of inhibiting the biological activity of the protein according to any one of (1) to (4), which comprises using the antibody according to any one of (26) to (28).
(32) A method of immunologically detecting and quantifying the protein according to any one of (1) to (4), which comprises using the antibody according to any one of (26) to (28).
(33) A method of isolating and purifying the protein according to any one of (1) to (4), which comprises using the antibody according to any one of (26) to (28).
(34) A method of obtaining cells expressing the protein according to any one of (1) to (4), which comprises using the antibody according to any one of (26) to (28).
(35) A method of screening a therapeutic agent for renal diseases, which comprises using the antibody according to any one of (26) to (28).
(36) A method of screening a substance inhibiting or promoting the transcription or translation of a gene encoding the protein according to any one of (1) to (4), which comprises using the antibody according to any one of (26) to (28).
(37) A diagnostic agent for renal diseases, which comprises the antibody according to any one of (26) to (28).
(38) A therapeutic agent for renal diseases, which comprises the antibody according to any one of (26) to (28).
(39) A drug delivery method for delivering an agent to damaged sites in the kidney, which comprises using a fusion antibody comprising the antibody according to any one of (26) to (28) bound to an agent selected from a radioisotope, a protein and a low-molecular-weight compound,.
(40) A method of screening a therapeutic agent for renal diseases, which comprises using cells obtained in the method according to (34).
(41) Amethod of treating renal diseases, which comprises using cells obtained by the method according to (34).
(42) A polypeptide comprising an amino acid sequence selected from the amino acid sequences represented by SEQ ID NOS: 1 and 15.
(43) A therapeutic agent for renal diseases, which comprises the polypeptide according to (42).
(44) A method of screening a receptor binding specifically to the protein according to any one of (1) to (4), which comprises using the protein according to any one of (1) to (4).
(45) A knockout non-human animal wherein the expression of a gene encoding the protein of any one of (1) to (4) is partially or completely suppressed.
(46) A knockout non-human animal wherein the activity of producing the protein according to any one of (1) to (4) is partially or completely suppressed.
(47) A recombinant non-human animal comprising a gene encoding the protein according to any one of (1) to (4) introduced into it.

The protein of the present invention relates to a protein (referred to hereinafter as kidney regeneration factor-1 (KRGF-1)) having an activity to regenerate a damaged kidney (referred to hereinafter as kidney regenerating activity). The protein of the present invention is expressed at a regenerative stage of a damaged kidney and is capable of acting on undifferentiated renal cells to migrate, proliferate and differentiate.

The protein of the present invention includes a protein having an amino acid sequence represented by SEQ ID NO:1 or 15, a protein having a kidney regenerating activity which comprises an amino acid sequence coding for the protein wherein one or more amino acids are deleted, substituted or added in the said amino acid sequence, and a protein which has a kidney regenerating activity and comprises an amino acid sequence having 60% or more identity with the said amino acid sequence coding for the protein, when the sequence identity is calculated using BLAST [J. Mol. Biol., 215, 403 (1990)] or FASTA [Methods in Enzymology, 183, 63 (1990)].

The protein having a kidney regenerating activity which comprises an amino acid sequence wherein one or more amino acids are deleted, substituted or added in the amino acid sequence represented by SEQ ID NO:1 or 15 can be obtained by introducing mutation in the amino acid sequence site-specifically by using site-specific mutagenesis described in Molecular Cloning, A Laboratory Manual, 2nd Edition, Cold Spring Harbor Laboratory Press (1989) (abbreviated hereinafter to Molecular Cloning, 2nd edition), Current Protocols in Molecular Biology, John Wiley & Sons (1987-1997) (abbreviated hereinafter to Current Protocols in Molecular Biology), Nucleic Acids Research, 10, 6487 (1982), Proc. Natl. Acad. Sci., USA, 79, 6409 (1982), Gene, 34, 315 (1985), Nucleic Acids Research, 13, 4431 (1985), Proc. Natl. Acad. Sci., USA, 82, 488 (1985), etc. The number of amino acids deleted, substituted or added, which is the number of amino acids capable of being deleted, substituted or added by known methods such as site-specific mutagenesis etc. described above, is not particularly limited, but it is preferably 1 to several dozens, preferably 1 to 20, more preferably 1 to 10 and most preferably 1 to 5. To allow the protein of the present invention to have functions as a kidney regeneration factor, the protein has 60% or more, usually 80% or more, particularly 95% or more identity with the amino acid sequence represented by SEQ ID NO:1, when the sequence identity is calculated using BLAST [J. Mol. Biol., 215, 403 (1990)] or FASTA [Methods in Enzymology, 183, 63 (1990)].

The glycoprotein of the present invention includes glycoproteins in which a sugar chain added to a glycosylation site of a protein having an amino acid sequence represented by SEQ ID NO:1 or 15, a protein having a kidney regenerating activity which comprises an amino acid sequence wherein one or more amino acids are deleted, substituted or added in the amino acid sequence represented by SEQ ID NO:1 or 15, and a protein having a kidney regenerating activity which comprises an amino acid sequence having 60% or more identity with the amino acid sequence represented by SEQ ID NO:1 or 15. The sugar chain bound to the protein of the present invention includes an Asn-type sugar chain wherein N-acetylglucosamine is bound via N-β-glycoside linkage to the Asn residue in an amino acid sequence Asn-X-Ser/Thr in the protein of the present invention, or a mucin-type sugar chain wherein N-acetylgalactosamine is bound via O-α-glycoside linkage to a Ser or Thr residue in the protein of the present invention.

The DNA of the present invention includes a DNA encoding the protein or glycoprotein of the present invention, for example a DNA encoding KRGF-1 derived from a patient with a renal disease (DNA having a nucleotide sequence represented by SEQ ID NO:2 or 3), and a DNA encoding KRGF-1 derived from a rat with proliferative glomerulonephritis (DNA having a nucleotide sequence represented by SEQ ID NO:16 or 17). Generally, a plurality of genetic codes occur for one amino acid, and the DNA of the present invention encompasses a DNA encoding the protein or glycoprotein of the present invention even if the DNA has a nucleotide sequence different from SEQ ID NO:2, 3, 16 or 17. Further, the DNA of the present invention also encompasses a DNA hybridizing under stringent conditions with the DNA encoding the protein and glycoprotein of the present invention, for example with the DNA having a nucleotide sequence represented by SEQ ID NO:2, 3, 16 or 17. The DNA is preferably a DNA of a gene expressed at a higher level in tissues with inflammations, for example in a damaged kidney.

The DNA being capable of hybridizing under stringent conditions refers to a DNA obtained according to colony hybridization, plaque hybridization, Southern blot hybridization or the like by using a DNA probe having a nucleotide sequence represented by SEQ ID NO:2, 3, 16 or 17, and a specific example of the DNA includes a DNA which can be identified by carrying out hybridization with a labeled DNA probe at 65°C in the presence of 0.7 to 1.0 mol/l sodium chloride using a filter on which a DNA prepared from colonies or plaques is immobilized and then washing the filter at 65°C with a 0.1 to 2-fold conc. SSC solution (1-fold conc. SSC solution is composed of 150 mmol/l sodium chloride and 15 mmol/l sodium citrate). Hybridization can be preformed according to methods described in Molecular Cloning, 2nd edition, Current Protocols in Molecular Biology, DNA Cloning 1: Core Techniques, A Practical Approach, Second Edition, Oxford University (1995) etc. Specifically, the DNA being capable of hybridizing includes a DNA having 60% or more identity, preferably 80% or more identity, and more preferably 95% or more identity with a nucleotide sequence represented by SEQ ID NO:2, 3, 16 or 17, when the sequence identity is calculated using BLAST or FASTA.

In addition, the DNA of the present invention also includes an oligonucleotide DNA having a sequence of consecutive 10 to 60 nucleotides in the nucleotide sequence of the DNA described above or a DNA having a nucleotide sequence complementary to the above-described DNA or oligonucleotide DNA.

Hereinafter, the present invention is described in detail.

### 1. Preparation of proliferative glomerulonephritis-related gene

### ① Preparation of a Thy-1 nephritis rat

A Thy-1 nephritis rat as a model of mesangial proliferative glomerulonephritis is prepared according to a literature [Laboratory Investigation, 55, 680 (1986)] in the following manner. An antibody against Thy-1.1 which is present as a membrane protein in rat mesangial cells is administered intravenously at a dose of 1 mg/kg into experimental rats such as Wister rats, whereby mesangiolytic lesions appear and hyperplasia of mesangial stroma and proliferation of mesangial cells can be induced to create a Thy-1 nephritis rats. Mesangiolysis can be detected by measuring urinary proteins or albumins.

### ② Preparation of a subtracted cDNA library from the kidney of the Thy-1 nephritis rat and selection of cDNA from the library by differential hybridization

As the DNA of the proliferative glomerulonephritis-related gene, cDNA of a gene expressed at higher levels in the kidney of the Thy-1 nephritis rat than in the kidney of a normal rat is prepared in the following manner. First, a cDNA library subjected to subtraction with normal rat kidney mRNA is prepared from the kidney of the Thy-1 nephritis rat, and cDNA clones of nephritis-related gene are concentrated. The resultant cDNA clones in the subtracted cDNA library are subjected to differential hybridization with RNAs from the kidney of the Thy-1 nephritis rat and RNA from the normal rat kidney as probes, whereby cDNA clones with increased expression levels in the kidney of the Thy-1 nephritis rat are selected, whereby the cDNA can be obtained.

### ②-1. Preparation of a subtracted cDNA library from the kidney of the Thy-1 nephritis rat

Subtraction is a method wherein single-stranded cDNA is prepared frommRNA extracted from tissues or cells under certain conditions, and then it is hybridized with mRNA in cells in a control rat, and only the cDNA hybridized with the mRNA is removed, whereby cDNA of a gene expressed at higher levels than in the control rat is selected.

There are some methods of preparing the subtracted cDNA library, but the present invention a method wherein a cDNA library from the kidney of the Thy-1 nephritis rat is prepared in a usual manner and converted into single-stranded DNA by helper phage, and then subjected to subtraction [Proc. Natl. Acad. Sci. USA, 88, 825 (1991)] is preferred. In subtraction, the cDNA is hybridized with biotinylated mRNA from a normal rat kidney, and streptoavidin is further bound to the resultant hybridized biotinylated mRNA-cDNA complex which is then separated by phenol extraction.

### ②-1-A. Preparation of a cDNA library from the kidney of the Thy-1 nephritis rat

Nephritis symptoms in the Thy-1 nephritis rat are varied depending on the number of days after intravenous injection, and it is considered that different genes are expressed at the stages of from theworsening of nephritis symptoms to spontaneous recovery, and kidneys are excised from the rats on Days 2, 4, 6, 8, and 10 after the injection, and RNA is extracted from each kidney. The RNA can be extracted by a guanidinium thiocyanate -cesium trifluoroacetate method [Methods in Enzymol., 154, 3 (1987)] and an acid guanidinium thiocyanate -phenol-chloroform method [Analytical Biochemistry, 162, 156 (1987)] or by using a kit such as FastTrack mRNA Isolation Kit (manufactured by Invitrogen). Generally, mRNA has polyA added to the 3'-terminal thereof, and mRNA can be purified from RNA by a method of using oligo-dT Sepharose (Molecular Cloning, 2nd edition).

A cDNA library can be prepared from the mRNA by using an oligo-dT primer and reverse transcriptase to prepare double-stranded cDNA and inserting it into a cloning vector according to the method described in a manual of ZAP-cDNA Preparation Kit from Stratagene.

The cloning vector needs to be capable of replication with a high copy number in Escherichia coli, have a marker gene for transformation, such as ampicillin resistance gene and kanamycin resistance gene and have a multi-cloning site into which cDNA can be inserted, and in addition to these properties as a general cloning vector, the cloning vector needs to be capable of easy conversion into single-stranded DNA. Accordingly, the cloning vector used includes phagemid vectors which are plasmids containing a replication signal IG (intergenic space) of M13 phage and can be converted into single-stranded DNA phage by infection with helper phage, for example pBluescript SK (-), pBluescript II KS(+), pBS(-), pBC (+) [all of which are manufactured by Stratagene] and pUC118 (manufactured by Takara Shuzo Co., Ltd.), and λ-phage vectors (e.g. λZAPII and ZAP Express, both of which are manufactured by Stratagene) which can be converted into phagemid by in vivo excision using helper phage. In vivo excision, conversion into single-stranded DNA phage, and purification of single-stranded DNA from phage in a culture supernatant can be carried out according to manuals attached to commercial vectors.

Escherichia coli into which the vector having the cDNA integrated therein is introduced may be any one capable of expressing the introduced gene. Specifically, Escherichia coli XL1-Blue MRF' [product of Stratagene, Strategies, 5, 81 (1992)], Escherichia coli C600 [Genetics, 39, 440 (1954)], Escherichia coli Y1088 [Science, 222, 778 (1983)], Escherichia coli Y1090 [Science, 222, 778 (1983)], Escherichia coli NM522 [J. Mol. Biol., 166, 1 (1983)], Escherichia coli K802 [J. Mol. Biol., 16, 118 (1966)] and Escherichia coli JM105 [Gene, 38, 275 (1985)] can be used.

In Subtraction, hybridization of cDNA with normal rat mRNA is carried out, and which strand of double strands is obtained as the single-stranded DNA from the phagemid depends on the type of the phagemid. Hence, in preparing the cDNA library, the preparation of the cDNA and the direction of insertion thereof into the vector need to be selected such that an antisense chain (chain having a nucleotide sequence complementary to actual mRNA) can be prepared as a single-stranded DNA from any cDNA clones. As described in e.g. a manual of a ZAP cDNA Synthesis Kit from Stratagene, synthesis of cDNA by reverse transcriptase is carried out using an oligo-dT primer having an XhoI site at the 5'-terminal thereof, and as a substrate, dNTP containing 5-methyl dCTP (whereby in cDNA after synthesis, cleavage with XhoI can be prevented) in place of dCTP, and after EcoRI adaptors are added to both ends of the synthesized cDNA, the cDNA is cleaved with XhoI and inserted into between EcoRI/XhoI sites of vector λZAPII, whereby the EcoRI site is located at the 5'-side of the cDNA, while the XhoI site at the 3'-side of the cDNA, thus permitting the cDNA to be inserted in the predetermined direction into the vector. This cDNA library is converted by in vivo excision into a cDNA library in which phagemid vector pBluescript SK(-) is used as the vector, and then infected with helper phage, to produce single-stranded DNA where the cDNA region is an antisense chain.

### ②-1-B. Subtraction with mRNA from the kidney of a control rat

The cDNA library in the phagemid vectors prepared in ②-1-A is infected with helper phage, whereby single-stranded DNA phage is released to the culture, and cDNA converted into single-stranded DNA is purified and recovered from the culture. In the case of λ-phage vector, the vector is converted into a phagemid by in vivo excision, and the same procedure is carried out (Molecular Cloning, 2nd edition).

The specific procedure of subtraction, the composition of reagents and reaction conditions can be identified in accordance with methods described in Genes to Cells, 3, 459 (1998). The control rat kidney mRNA prepared in ②-1-A is biotinylated with Photoprobe Biotin (manufactured by Vector Laboratories) or the like and then hybridized with the single-stranded cDNA from the kidney of the Thy-1 nephritis rat. Streptoavidin which strongly binds to biotin is reacted with the solution after hybridization, whereby streptoavidin is further bound to the cDNA hybridized with the biotinylated mRNA to increase the hydrophobicity thereof, followed by extraction thereof with phenol. The cDNA which has not been hybridized can be isolated from the aqueous layer. The cDNA hybridized with the biotinylated mRNA is extracted in the phenol layer.

### ②-1-C. Reverse subtraction

In the procedure of subtraction in ②-1-B, in addition to cDNA of a gene expressed at a higher level specifically in the kidney of the Thy-1 nephritis rat, clones in which expression level of the cDNA is very low in the kidneys of both the Thy-1 nephritis rat and the control rat and whose number is small and the vectors into which the cDNA has not been inserted, tend to be also concentrated, but such cDNAs and vectors do not meet the object of the present invention. Therefore, in order to select cDNA occurring at a certain level of number in the kidney of the Thy-1 nephritis rat, the cDNAs after subtraction and biotinylated mRNA from the kidney of the Thy-1 nephritis rat are subjected to hybridization and isolation in the same manner as in subtraction, and as opposed to ordinary subtraction, cDNA hybridized with the biotinylated mRNA is separated from non-hybridized cDNA and recovered in a phenol layer. The recovered, hybridized and biotinylated mRNA-cDNA is heated at 95°C and then cooled quickly whereby the conjugate is dissociated into the cDNA and the biotinylated mRNA, and by extraction with water, the cDNA hybridized with the mRNA can be isolated into the aqueous layer.

### ②-1-D. Formation of a library from the cDNA after subtraction

The cDNA after subtraction and reverse subtraction in ②-1-B and ②-1-C is converted to double-stranded by using a suitable primer having a nucleotide sequence complementary to the nucleotide sequence of the vector and DNA polymerase such as BcaBEST (manufactured by Takara Shuzo Co., Ltd.), Klenow fragments etc. and then introduced into Escherichia coli, whereby a cDNA library is constructed again. The method of introducing the cDNA into Escherichia coli is preferably electroporation in order to achieve highly efficient transformation.

### ②-2. Differential hybridization

In the subtracted cDNA library prepared in ②-1, the cDNA of proliferative glomerulonephritis-related gene expressed at a higher level in the kidney of the Thy-1 nephritis rat has been concentrated, but all cDNA clones in the library are not limited to the proliferative glomerulonephritis-related gene. For selection of the cDNA of the proliferative glomerulonephritis-related gene from these clones, Northern hybridization with each cDNA clone as a probe (Molecular Cloning, 2nd edition) and RT-PCR [PCR Protocols, Academic Press (1990)] with primers based on the nucleotide sequence of cDNA clone are used for comparing mRNA levels between the normal rat kidney and the kidney of the Thy-1 nephritis rat, whereby the cDNA of the nephritis-related gene expressed actually at a higher level in the kidney of the Thy-1 nephritis rat can be selected. Further, the cDNA clone expressed at a higher level can be selected comprehensively and efficiently by differential hybridization shown below.

First, the subtracted cDNA library obtained in the method ②-1 is diluted at such a concentration as to separate individual colonies from each other, and cultured in an agar medium, and the separated colonies are cultured separately in a liquidmedium under the same conditions. This culture is inoculated in the same volume onto 2 nylon membranes, and the membranes are placed on an agar medium and cultured under the same conditions, whereby colonies are grown in almost the same amount in the 2 membranes. Accordingly, the DNA in the colonies in almost the same amount is blotted onto each of the nylon membranes, and the DNA is denatured and neutralized by the method described in Molecular Cloning, 2nd edition and fixed on the membranes by irradiation with ultraviolet rays. One of the membranes is subjected to colony hybridization with the whole mRNA as the probe from the kidney of the Thy-1 nephritis rat, while the other membrane to colony hybridization with the whole mRNA as the probe from the kidney of the control rat, and clones expressed at higher levels in the kidney of the Thy-1 nephritis rat are selected by comparing the intensity of their hybridization signal. Colonies of the selected clones are separately cultured on a 96-well plate and then inoculated onto a nylon membrane by means of an automatic micropipetting unit Hydra 96 (manufactured by Robbins Scientific Ltd.) compatible with a 96-well plate. By this procedure, the duplicate membranes having the same amount of DNA blotted thereon can be rapidly prepared for a large number of colonies, and the correspondence of the original colonies is also clear.

As the probe, labeled cDNA, which, as similar to a conventional DNA probe, was prepared from the whole mRNA by using a reverse transcriptase and a random primer can be used, but an RNA probe is desirable because it hybridizes stronger with DNA on the membrane and gives a stronger signal than the DNA probe. mRNA is subjected to the same cDNA synthetic reaction as in ②-1-A, using a reverse transcriptase and an oligo dT primer having, at the 5'-terminal thereof, a promoter sequence specific to RNA polymerase such as T7, T3 and SP6, whereby cDNA having the promoter sequence at the terminal is synthesized. An RNA polymerase specific to the promoter sequence, together with a labeling nucleotide as the substrate, is allowed to act on the cDNA, whereby a large number of uniform RNA probes having a high degree of labeling can be easily synthesized. For labeling the probe, radioisotopes such as ³²P and ³⁵S or easily detectable non-radioisotopes such as digoxigenin (DIG) and biotin are used.

After the membrane prepared above is hybridized with each RNA from the kidney of the Thy-1 nephritis rat and the kidney of the control rat, the probe hybridized with each colony DNA is detected. For detection of the hybridized probe, a method suitable for the labeling substance is used. For example, the following method is used for highly sensitive and quantitative detection; for radioisotopes, autoradiography directly exposing an X-ray film or an imaging plate is used, and for DIG, an anti-DIG antibody labeled with alkaline phosphatase is bound thereto, and a substrate (e.g. CSPD) emitting by alkaline phosphatase is reacted therewith to sensitize an X-ray film according to a DIG System User's Guide (Roche).

When there is a gene expressed at a higher level in the kidney of the Thy-1 nephritis rat than in the kidney of the control rat, the number of mRNA molecules for the gene present in the probe is also high, so that even if the same amount of the DNA is blotted on the membrane, a larger number of probes are bound to the cDNA spot corresponding to the gene. Accordingly, by comparing the intensity of the hybridization signal on the two membranes having the DNA of the same cDNA clone blotted thereon, the cDNA of the gene expressed at a higher level in the kidney of the Thy-1 nephritis rat than in the kidney of the normal rat can be selected.

The rat cDNA thus obtained includes rat cDNA having the nucleotide sequence represented by SEQ ID NO:16.

### ③ Analysis of the nucleotide sequence of the DNA

The nucleotide sequence of the thus obtained cDNA of the gene expressed at a higher level in the kidney of the Thy-1 nephritis rat than in the kidney of the normal rat can be determined by the dideoxy method [Proc. Natl. Acad. Sci., USA, 74, 5463 (1977)] or by a DNA sequencer. By translating the resultant nucleotide sequence into an amino acid sequence, the amino acid sequence of the protein encoded by the gene can be obtained. Further, the resultant nucleotide sequence is compared with nucleotide sequences in a nucleotide sequence data base such as GenBank and EMBL by using a homology analysis program such as BLAST and FASTA, whereby to determine whether the resultant nucleotide sequence is a novel nucleotide sequence or not, and a nucleotide sequence having identity with the resultant nucleotide sequence can be searched. Further, the amino acid sequence determined from the nucleotide sequence is compared with an amino acid sequence data base such as SwissProt, PIR and GenPept, whereby a protein having identity with the protein encoded by the nucleotide sequence, for example, a protein derived from the corresponding gene in a species other than rat and a family protein presumed to have similar activity and functions can be searched.

### ④ Preparation of the full-length cDNA

The cDNA obtained in ② may contain incomplete cDNA not encoding the full-length protein because of partial degradation of mRNA or suspension of synthesis by reverse transcriptase on the way from the 3' to 5' of mRNA. By analysis of the nucleotide sequence of such incomplete cDNA, it is not possible to reveal all amino acids of the protein encoded by the cDNA. In analysis of the nucleotide sequence, there is also the case where the resultant cDNA is estimated to be not a full-length cDNA by comparison thereof with homologous nucleotide sequences or amino acid sequences, or by comparison of the length of mRNA with the length of the resultant cDNA by the Northern blotting method described in 5. When the cDNA is incomplete, the full-length cDNA can be obtained in the following manner.

### ④-1. Re-screening from the cDNA library

From the cDNA library from the kidney in the Thy-1 nephritis rat, cDNA clones hybridized with the resultant nephritis-related gene cDNA as the probe are obtained by colony hybridization or plaque hybridization (Molecular Cloning, 2nd edition). From the clones thus obtained, DNA is prepared by the method described in Molecular Cloning, 2nd edition, and then cleaved with restriction enzymes, and a clone having the longest insert is selected. As the cDNA library from the kidney of the Thy-1 nephritis rat, the subtracted cDNA library may be used again, but clones containing a longer cDNA tend to be lost in the procedure of subtraction, so that when the cDNA library prior to subtraction from the kidney of the Thy-1 nephritis rat is used, it is highly possible that the full-length cDNA can be obtained.

### ④-2. Rapid amplification of cDNA ends (RACE)

By 5'-RACE and 3'-RACE [Proc. Natl. Acad. Sci. USA, 85, 8998 (1988)] wherein adaptor nucleotide sequences having adaptor oligonucleotides added to both ends of the cDNA from the kidney of the Thy-1 nephritis rat, and primers based on the nucleotide sequence of the obtained cDNA clone, are used in PCR, cDNA fragments corresponding to external regions of the 5'-terminal and 3'-terminal of the cDNA obtained in ② can be obtained. The nucleotide sequences of the resultant cDNAs are determined in the same manner as in ③. The cDNAS obtained in the method can be ligated to the cDNA obtained in ②, to give the full-length cDNA.

The full-length cDNA of the rat proliferative glomerulonephritis-related gene, obtained in this manner, includes cDNA of the rat KRGF-1 gene having the nucleotide sequence represented by SEQ ID NO:16. Transformant Escherichia coli DH5 α/pTRDH-091KRGF harboring the cDNA clone pTRDH-091KRGF thus obtained has been deposited as FERM BP-7031 since February 16, 2000 with the National Institute of Bioscience and Human-Technology, Agency of Industrial Science and Technology (1-3, Higashi 1-chome, Tsukuba-shi, Ibaraki, Japan).

### ④-3. Data base information and use of PCR

When the nucleotide sequence of the cDNA determined in ③ is analyzed for homology with a nucleotide sequence data base, there is the case where it is identical with the nucleotide sequence of a known gene, but identical with EST (expressed sequence tag) which is the nucleotide sequence of a terminal portion of random cDNA clones. In this case, the EST, EST having a nucleotide sequence identical with the nucleotide sequence of the EST, and EST derived from the clone of such EST are collected as ESTs derived from the same gene. By merging the nucleotide sequences of ESTs derived from the same gene, the nucleotide sequence of a portion extending from the 5'- or 3'-side of the cDNA obtained in ② may be found. In this case, PCR is conducted where a forward primer having a 5'-terminal nucleotide sequence of the nucleotide sequence obtained by merging ESTs or a reverse primer having a nucleotide sequence complementary to a 3'-terminal nucleotide sequence thereof is used while cDNA from the kidney of a Thy-1 nephritis rat or a cDNA library from the kidney of a Thy-1 nephritis rat is used as the template, whereby a cDNA corresponding to the external region of the 5'- or 3'-terminal of the nucleotide sequence of the cDNA obtained in ② can be obtained. The resultant cDNAs are subjected to nucleotide sequencing in the same manner as in ③, and can be ligated to the cDNA obtained in ②, to give the full-length cDNA. When rat ESTs derived from the objective nephritis-related gene are obtained in a large number from the data base, there is also the case where without conducting RT-PCR, the nucleotide sequence of the full-length cDNA of the nephritis-related gene can be revealed by merging the nucleotide sequences of the collected ESTs.

When the nucleotide sequence of the full-length cDNA thus obtained is revealed, the full-cDNA can be obtained by PCR where primers prepared on the basis of the nucleotide sequence of the cDNA are used while cDNA or a cDNA library from the kidney of a Thy-1 nephritis rat is used as the template. On the basis of the determined nucleotide sequence of cDNA of the nephritis-related gene, the DNA of the nephritis-related gene can be chemically synthesized by a DNA synthesizer. The synthesizer includes a DNA synthesizer model 392 using the phosphoamidite method, produced by Perkin Elmer.

### ⑤ Acquisition of the human corresponding gene

For application of the KRGF-1 gene to therapy and diagnosis of human proliferative glomerulonephritis, human KRGF-1 is necessary. Generally, proteins having similar functions have a highly homologous amino acid sequence even among different species, and the nucleotide sequences of genes encoding such proteins tend to be highly homologous. Accordingly, the human cDNA can be obtained from a cDNA library of the human kidney, preferably the kidney of a patient with proliferative glomerulonephritis, by conducting screening by hybridization with the rat cDNA as the probe under slightly stringent conditions. The term "the slightly stringent conditions" As used herein, while it varies depending on homology between the human cDNA and rat cDNA, means the most stringent conditions in which clear bands can be seen, among hybridization conditions with varying stringency, in Southern blotting of restriction enzyme-cleaved human chromosomal DNA with the rat cDNA as the probe. For example, when a formamide-free hybridization solution is used, the conditions are determined by conducting hybridization under several conditions where the salt concentration in the composition of the hybridization solution is fixed at 1 mol/l while the hybridization temperature is changed stepwise between 68°C and 42°C, followed by washing with 2xSSC containing 0.5% SDS at the same temperature as in the hybridization. In the case of a formamide-containing hybridization solution, the conditions are determined by conducting hybridization under several conditions where the temperature (42°C) and the salt concentration (6×SSC) are fixed while the formamide concentration are changed stepwise between 50% and 0%, followed by washing at 50°C with 6×SSC containing 0.5% SDS.

Further, the novelty and identity of the nucleotide sequence of the rat cDNA obtained in ② or ④ is searched in the same manner as in ③, to determine whether there is a nucleotide sequence of human cDNA having high (specifically 80% or more) identity with the nucleotide sequence of the rat cDNA, particularly with the whole region coding for the protein. The human cDNA having high identity is estimated to be the cDNA of the human gene corresponding to the rat gene obtained in ② and ④. Accordingly, this human cDNA can be amplified and isolated by RT-PCR using primers corresponding to the nucleotide sequences of the 5'- and 3'-terminals of this human cDNA and RNA extracted from human cells or tissues, preferably from renal tissues or kidney-derived cells and more preferably from the kidney of a patient with proliferative glomerulonephritis is used as the template. There is the case where the human cDNA found in the data base is not a full-length one or is a nucleotide sequence of EST only, but in this case also, the full-length human cDNA can be obtained in the same manner as described in ④ for the rat cDNA.

The human cDNA thus obtained is analyzed for its nucleotide sequence in the same manner as in ③, and the amino acid sequence of the human protein encoded by the cDNA can be revealed.

For other non-human mammals, the corresponding gene can be obtained in the same manner.

### ⑥ Acquisition of the genome gene

By methods described in Molecular Cloning, 2nd edition, a genome DNA library prepared using chromosomal DNA isolated from rat or human cells and tissues is screened through techniques such as plaque hybridization with the rat or human cDNA as the probe obtained in ② or ⑤, whereby the rat or human genome DNA of the gene of the present invention can be obtained. By comparing the nucleotide sequence of the genome DNA with the nucleotide sequence of the cDNA, the exon/intron structure of the gene can be revealed. By using the 5'-end region of the cDNA as the probe, the nucleotide sequence of a genome gene region such as a promoter of the gene of the present invention regulating transcription can be revealed, and the promoter region of the gene of the present invention can be isolated and obtained. The sequence of the promoter region thus obtained is useful for analyzing the mechanism of regulating the transcription of the gene of the present invention.

Further, genome genes from other non-human mammals can be obtained by the same method.

### ⑦ Preparation of oligonucleotides

Using the information on the nucleotide sequence of the DNA of the present invention obtained in the method described above, oligonucleotides such as antisense oligonucleotide, sense oligonucleotide etc. having a partial sequence of the DNA of the present invention can be prepared by a DNA synthesizer.

The oligonucleotides include DNA having the same sequence as a sequence of consecutive 10 to 60 nucleotides in the objective DNA, or DNA having a complementary sequence to the DNA, and specific examples include DNA having the same sequence as a sequence of consecutive 10 to 60 nucleotides in the nucleotide sequence represented by SEQ ID NO:2, or DNA having a complementary sequence to the DNA. If these oligonucleotides are used as forward and reverse primers in PCR, the oligonucleotides preferably comprise 10 to 60 nucleotides, which have not so different melting temperatures (Tm) and not so different numbers of nucleotides.

Furthermore, derivatives of these oligonucleotides (also referred to hereinafter as oligonucleotide derivatives) can also be used as the oligonucleotide of the present invention.

The oligonucleotide derivatives include an oligonucleotide derivative whose phosphodiester linkage was converted into a phosphorothioate linkage, an oligonucleotide derivative whose phosphodiester linkage was converted into a N3'-P5' phosphoamidate linkage, an oligonucleotide derivative whose ribose and phosphodiester linkage was converted into a peptide nucleic acid linkage, an oligonucleotide derivative whose uracil was substituted by C-5 propinyl uracil, an oligonucleotide derivative whose uracil was substituted by C-5 thiazol uracil, an oligonucleotide derivative whose cytosine was substituted by C-5 propinyl cytosine, an oligonucleotide derivative whose cytosine was substituted by phenoxazine-modified cytosine, an oligonucleotide derivative whose ribose was substituted by 2'-O-propyl ribose, and an oligonucleotide derivative whose ribose was substituted by 2'-methoxy-ethoxyribose [SaiboKogaku (Cell Engineering), 16, 1463 (1997)].

### 2. Production of KRGF-1 protein

Hereinafter, the process for producing KRGF-1 protein is described.

On the basis of the full-length cDNA, a DNA fragment of suitable length containing a necessary coding region for the protein is prepared.

The DNA fragment or the full-length DNA is inserted into a site downstream from a promoter in an expression vector to construct a recombinant expression vector for the protein.

The recombinant expression vector is introduced into a host cell compatible with the vector.

The host cell used may be any cell which can express the objective DNA, and examples of such cells include e.g. microorganisms belonging to the genus Escherichia, Serratia, Corynebacterium, Brevibacterium, Pseudomonas, Bacillus or Microbacterium, yeasts belonging to the genus Kluyveromyces, Saccharomyces, Shizosaccharomyces, Trichosporon or Schwanniomyces, as well as mammalian cells, insect cells etc.

The expression vector used is a vector capable of autonomous replication or integration into the chromosome in the host cells and containing a promoter at a position suitable for transcription of DNA of the KRGF-1 gene.

When a bacterium is used as the host cell, recombinant expression vector for the KRGF-1 gene is preferably a recombinant expression vector capable of autonomous replication in the bacterium and comprising a promoter, a ribosome-binding sequence, KRGF-1 DNA, and a transcription termination sequence. The vector may also contain a gene for regulating the promoter.

The expression vector includes, for example, pBTrp2, pBTac1 pBTac2 (which all are commercially available from Boehringer Mannheim), pKK233-2 (manufactured by Amersham Pharmacia Biotech), pSE280 (manufactured by Invitrogen), pGEMEX-1 (manufactured by Promega), pQE-8 (manufactured by Qiagen), pKYP10 (Japanese Published Unexamined Patent Application No. 110600/1983), pKYP200 [Agricultural Biological Chemistry, 48, 669 (1984)], pLSA1 [Agric. Biol. Chem., 53, 277 (1989)], pGEL1 [Proc. Natl. Acad. Sci., USA, 82, 4306 (1985)], pBluescript II SK(-) (manufactured by Stratagene), pGEX (manufactured by Amersham Pharmacia Biotech), pET-3 (manufactured by Novagen), pTerm2 (USP4686191, USP4939094, USP5160735), pSupex, pUB110, pTP5PC-194, pEG400 [J. Bacteriol., 172, 2392 (1990)] etc.

The expression vector is preferably the one in which the distance between a ribosome-binding sequence i.e. Shine-Dalgarno sequence and an initiation codon is adjusted to an appropriate distance (for example 6 to 18 nucleotides).

The promoter may be any promoter capable of performing in hosts for expression. Examples are promoters derived from *E. coli,* phage etc., such as trp promoter (Ptrp), lac promoter (Plac), PL promoter, PR promoter, T7 promoter etc. as well as SP01 promoter, SPO2 promoter, penP promoter etc. Artificially designed and modified promoters such as a PtrpX2 promoter having two trp promoters in tandem, tac promoter, letI promoter [Gene, 44, 29 (1986)], lacT7 promoter etc. can also be used.

Productivity of the objective protein can be improved by substituting the nucleotide sequence of the protein coding region in KRGF-1 DNA of the present invention as to have the optimum codons for expression in hosts.

Although a transcription termination sequence is not necessarily required to express KRGF-1 DNA of the present invention, it is desirable to locate the transcription termination sequence just downstream from the structural gene.

The host cell includes bacterium belonging to the genus Escherichia, Serratia, Corynebacterium, Brevibacterium, Pseudomonas, Bacillus etc., for example Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No.49, Escherichia coli W3110, Escherichia coli NY49, Bacillus subtilis, Bacillus amyloliquefaciens, Brevibacterium ammoniagenes, Brevibacterium immariophilum ATCC14068, Brevibacterium saccharolyticum ATCC14066, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC15354, Pseudomonas sp. D-0110 etc.

The method of introducing the recombinant expression vector may be any method of introducing DNA into the host cell, and examples include a method of using calcium ions [Proc. Natl. Acad. Sci. USA, 69, 2110(1972)], the protoplast method (Japanese Published Unexamined Patent Application No. 248394/1988), methods described in Gene, 17, 107 (1982) and Molecular & General Genetics, 168, 111 (1979).

When yeast is used as the host cell, expression vectors such as YEp13 (ATCC37115), YEp24 (ATCC37051), YCp50 (ATCC37419), pHS19, pHS15 etc. can be exemplified.

Any promoter can be used insofar as it is capable of working for expression in yeasts, and examples include PHO5 promoter, PGK promoter, GAP promoter, ADH promoter, gal 1 promoter, gal 10 promoter, heat shock protein promoter, MF α1 promoter, CUP 1 promoter etc.

The host cell includes Saccharomyces cerevisiae, Schizosaccharomyces pombe, Kluyveromyces lactis, Trichosporon pullulans, Schwanniomyces alluvius etc.

The method of introducing the recombinant expression vector may be any method of introducing DNA into yeasts, and examples include the electroporation method [Methods in Enzymol., 194, 182 (1990)], the spheroplast method [Proc. Natl. Acad. Sci. USA, 75, 1929 (1978)], the lithium acetate method [J. Bacteriol., 153, 163 (1983)], a method described in Proc. Natl. Acad. Sci. USA, 75, 1929 (1978) and the like.

When mammalian cell are used as the host cell, examples as expression vectors include pcDNAI (manufactured by Invitrogen), pcDM8 (manufactured by Invitrogen), pAGE107 [Japanese Published Unexamined Patent Application No. 22979/1991, Cytotechnology, 3, 133 (1990)], pAS3-3 (Japanese Published Unexamined Patent Application No. 227075/1990), pCDM8 [Nature, 329, 840 (1987)], pcDNAI/Amp (manufactured by Invitrogen), pREP4 (manufactured by Invitogen), pAGE103 [J. Biochem., 101, 1307 (1987)], pAGE210 etc.

As the promoter, any promoter capable of expression in mammalian cells can be used, and examples includes a promoter of IE (immediate early) gene of cytomegalovirus (human CMV), an SV40 early promoter, a retrovirus promoter, a metallothionein promoter, a heat shock protein promoter, an SR α promoter etc. Furthermore, an enhancer of the IE gene of human CMV may be used together with the promoter.

The host cell includes Namalwa cell that is a human cell, COS cell that is a monkey cell, CHO cell that is a Chinese hamster cell, BHT5637 (Japanese Published Unexamined Patent Application No. 299/1988) etc.

As the method for introducing the recombinant expression vector, any methods capable of introducing DNA into mammalian cells can be used, and it is possible to use, for example, the electroporation method [Cytotechnology, 3, 133 (1990)], the calcium phosphate method (Japanese Published Unexamined Patent Application No. 227075/1990), the lipofection method [Proc. Natl. Acad. Sci. USA, 84, 7413 (1987), Virology, 52, 456 (1973)] etc. Acquisition and culture of the transformant can be carried out according to methods described in Japanese Published Unexamined Patent Application No. 227075/1990 or 257891/1990.

When insect cells are used as the host, the protein can be expressed by methods described in e.g. Baculovirus Expression Vectors, A Laboratory Manual, Current Protocols in Molecular Biology Supplements 1-38 (1987-1997), Bio/Technology, 6, 47 (1988) etc.

That is, the recombinant transfer vector and baculovirus are co-introduced into insect cells to obtain a recombinant virus in a culture supernatant of the insect cells, and insect cells are infected with the recombinant virus, whereby the protein can be expressed.

The transfer vector includes pVL1392, pVL1393 and pBlueBacIII (all of which are manufactured by Invitrogen).

As the baculovirus, it is possible to use for example Autographa californica nuclear polyhedrosis virus, that is, a virus infecting insects of the family Noctuidae.

As the insect cell, it is possible to use Spodoptera frugiperda ovarian cells Sf9, Sf21 [Baculovirus Expression Vectors, A Laboratory Manual, W. H. Freeman and Company, New York (1992)], Trichoplusia ni ovarian cells High 5 (manufactured by Invitrogen) etc.

As the method of co-introducing the recombinant transfer vector and the baculovirus into insect cells to prepare a recombinant virus, for example, the calcium phosphate method (Japanese Published Unexamined Patent Application No. 227075/1990), the lipofection method [Proc. Natl. Acad. Sci. USA, 84, 7413 (1987)] etc. can be exemplified.

As the method of expressing the gene, secretory production, expression of fusion protein etc. besides direct expression can be carried out according to the methods described in Molecular Cloning, 2nd edition.

In the case of expression in yeasts, mammalian cells or insect cells, a protein having sugar or sugar chains added thereto can be obtained.

The KRGF-1 protein can be produced by culturing a transformant harboring a recombinant expression vector comprising KRGF-1 DNA integrated therein in a medium, to produce and accumulate the KRGF-1 protein in the culture, and recovering the protein from the culture.

The method of culturing the transformant for production of the KRGF-1 protein of the present invention in a medium can be carried out according to conventional methods used for culturing host cells.

The medium for culturing the transformants of the present invention obtained from prokaryotes such as E. coli or eukaryotes such as yeast etc. as the host may be a natural or synthetic medium insofar as the medium contains a carbon source, a nitrogen source, inorganic salts etc. which can be assimilated by the host cells and in which the transformants can be efficiently cultured.

Any carbon sources can be used insofar as they can be assimilated by the host cells, and carbohydrates such as glucose, fructose, sucrose, molasses containing them, starch or starch hydrolyzates, organic acids such as acetic acid, propionic acid and the like, and alcohols such as ethanol, propanol and the like can be used.

As the nitrogen source, ammonia, ammonium salts of various inorganic acids and organic acids, such as ammonium chloride, ammonium sulfate, ammonium acetate, and ammonium phosphate; other nitrogen-containing compounds; and peptone, meat extracts, yeast extracts, corn steep liquor, casein hydrolyzates, soybean meal, soy beanmeal hydrolyzates, various fermented microorganisms and digests thereof and the like can be used.

As the inorganic salts, potassium dihydrogen phosphate, dipotassium hydrogen phosphate, magnesium phosphate, magnesium sulfate, sodium chloride, ferrous sulfate, manganese sulfate, copper sulfate, calcium carbonate, and the like can be used.

Culturing is conducted under aerobic conditions using for example shaking culture or submerged aeration stirring culture. The culture temperature is preferably 15 to 40°C, and the culturing time is usually 16 hours to 7 days. During culturing, pH is maintained at 3.0 to 9.0. Adjustment of the pH is conducted using an inorganic or organic acid, an alkaline solution, urea, calcium carbonate, ammonia and the like.

If necessary, antibiotics such as ampicillin and tetracycline may further be added to the medium during the culturing.

For culturing a microorganism transformed with an expression vector using an inductive promoter as a promoter, an inducer may be added to the medium if necessary. For example, for culturing a microorganism transformed with an expression vector having lac promoter, isopropyl-β-D-thiogalactopyranoside (IPTG) or the like may be added to the medium; for culturing a microorganism transformed with an expression vector having trp promoter, indole acrylic acid (IAA) or the like may be added to the medium.

As the medium for culturing a transformant obtained from a mammalian cell as the host cell, generally used media such as RPMI1640 medium [The Journal of the American Medical Association, 199, 519 (1967)], Eagle's MEM [Science, 122, 501 (1952)], Dulbecco's modified MEM medium [Virology, 8, 396 (1959)], 199 medium [Proceeding of the Society for the Biological Medicine, 73, 1 (1950)] or any of these media further supplemented with fetal calf serum can be used.

Culturing is conducted usually for 1 to 7 days under the conditions of pH 6 to 8 and 30 to 40 °C in the presence of 5% CO₂.

Further, antibiotics such as kanamycin and penicillin can be added if necessary to the medium during culturing.

As the medium for culturing the transformant obtained from insect cells as host cells, generally used TNM-FH medium (manufactured by Pharmingen), Sf-900II SFM medium (manufactured by Life Technologies), ExCell400 and ExCell405 (both are manufactured by JRH Biosciences) and Grace's Insect Medium (Grace, T. C. C., Nature, 195, 788 (1962)] can be used.

Culturing is conducted usually for 1 to 5 days under the conditions of pH 6 to 7 and 25 to 30°C.

Further, antibiotics such as gentamicin can be added if necessary to the medium during culturing.

For isolation and purification of the KRGF-1 protein from a culture of the transformant, conventional methods of isolating and purifying proteins may be used.

For example, when the protein is produced in a dissolved state in cells, the cells after culturing are recovered by centrifugation, suspended in an aqueous buffer and disrupted by a sonicator, a French press, a Manton-Gaulin homogenizer or Dynomill, to give a cell-free extract. The supernatant obtained by centrifugation of the cell-free extract is subjected to conventional protein isolation and purification methods such as solvent extraction, salting-out by sulfate ammonium, desalting, precipitation with organic solvent, diethyl aminoethyl (DEAE)-Sepharose, anion exchange chromatography using DIAIONHPA-75 (manufactured by Mitsubishi Chemical Corp.) resin, cation exchange chromatography using S-Sepharose FF (manufactured by Amersham Pharmacia Biotech) resin, hydrophobic chromatography using resin such as butyl Sepharose or phenyl Sepharose, gel filtration using a molecular sieve, affinity chromatography, chromatofocusing and electrolysis such as electrofocusing, or a combination thereof, whereby a purified preparation of the protein can be obtained.

Further, when the protein is produced as inclusion bodies in cells, the cells are recovered, disrupted and centrifuged, whereby inclusion bodies of the protein are recovered as a precipitated fraction. The recovered inclusion bodies of the protein are solubilized by a protein denaturant. The solubilized solution is diluted or dialyzed, whereby the concentration of the protein denaturant in the solubilized solution is decreased, and the protein is refolded into a normal stereostructure, and by the isolation and purification methods described above, a purified preparation of the protein is obtained.

When the protein or the glycosylated protein is extracellularly secreted, the protein or the glycosylated protein can be recovered from a culture supernatant. That is, the culture supernatant is obtained from the culture by techniques such as centrifugation, and from the culture supernatant, a purified preparation can be obtained by the isolation and purification method described above.

The protein thus obtained includes, for example, a protein having an amino acid sequence represented by SEQ ID NO:1 or 15.

The protein of the present invention can also be produced by a chemical synthesis method such as the Fmoc method (fluorenylmethyloxy carbonyl method) and t-Boc method (t-butyloxy carbonyl method). Alternatively, the protein can be synthesized by utilizing a peptide synthesizer produced by Advanced ChemTech US, Perkin-Elmer, Amersham Pharmacia Biotech, Protein Technology Instrument US, Synthecell-Vega US, PerSeptive US, and Shimadzu Corp.

### 3. Preparation of an antibody specifically recognizing KRGF-1 protein

The purified preparation of the full-length KRGF-1 protein or a partial fragment thereof, or a synthetic peptide having a partial amino acid sequence of the KRGF-1 protein is used as the antigen, whereby an antibody such as polyclonal antibody and monoclonal antibody recognizing the XRGF-1 protein can be prepared.

### (1) Preparation of a polyclonal antibody

The purified preparation of the full-length KRGF-1 protein or a partial fragment thereof, or a synthetic peptide having a partial amino acid sequence of the protein of the present invention is used as the antigen, together with suitable adjuvant (for example, complete Freund's adjuvant, aluminum hydroxide gel or pertussis vaccine), is administered subcutaneously, intravenously or intraperitoneally to an animal, whereby the polyclonal antibody can be prepared.

The animals to which the antigen is administered include rabbits, goats, rats, mice, hamsters and the like.

The preferred dosage of the antigen is 50 to 100 µg per animal.

When a peptide is used as the antigen, a conjugate of the peptide bound covalently to a carrier protein such as keyhole limpet haemocyanin, bovine thyroglobulin or the like is preferably used. The peptide used as the antigen can be synthesized by a peptide synthesizer.

Administration of the antigen is carried out 3 to 10 times at one- to two-week intervals after the first administration. A blood sample is recovered from the ocular fundus plexus venosus 3 to 7 days after each administration, and the serum is confirmed to react with the antigen used for immunization by enzyme-linked immunosorbent assay [Enzyme-linked Immunosorbent Assay (ELISA), published by Igaku Shoin (1976); Antibodies-ALaboratory Manual, Cold Spring Harbor Laboratory (1988)] as to whether it is reactive .

A polyclonal antibody can be prepared by obtaining the serum from a non-human mammal whose serums shows a sufficient antibody titer against the antigen used for immunization, then isolating and purifying it from the serum.

With regard to the method of isolation and purification, techniques such as centrifugation, salting-out with 40 to 50% saturated ammonium sulfate, caprylic acid precipitation [Antibodies, ALaboratory manual, Cold Spring Harbor Laboratory (1988)] or chromatography using a DEAE-Sepharose column, an anion exchange column, a protein A or G column, a gel filtration column and the like may be employed alone or in combination.

### (2) Preparation of a monoclonal antibody

### (a) Preparation of antibody-producing cells

The rat whose serum showed sufficient antibody titer against a partial fragment polypeptide of the protein of the present invention used in immunization is used as a source of antibody-producing cells.

On day 3 to 7 after the final administration of the antigen to a rat showing sufficient antibody titer, the spleen is excised from the rat.

The spleen is cut into pieces in MEM medium (manufactured by Nissui Pharmaceutical, Co., Ltd) and the pieces are then loosened with tweezers, followed by centrifugation at 1,200 rpm for 5 minutes, to discard the resulting supernatant.

The spleen cells in the resulting precipitated fraction are treated with Tris-ammonium chloride buffer (pH 7.65) for 1 to 2 minutes to remove erythrocytes, followed by washing 3 times with MEM medium to give spleen cells as antibody-producing cells.

### (b) Preparation of bone mallow cells

As myeloma cells, cell lines obtained from mice or rats are used. For example, 8-azaguanine-resistant mice (BALB/c)-derived myeloma cell lines P3-X63Ag8-U1 (hereinafter abbreviated to P3-U1) [Curr. Topics. Microbiol. Immunol., 81, 1 (1978), Europ. J. Immunol., 6, 511 (1976)], SP2/0-Ag14 (SP-2) [Nature, 276, 269 (1978)], P3-X63-Ag8653(653) [J. Immunol., 123, 1548 (1979)], P3-X63-Ag8(X63) [Nature, 256, 495 (1975)] etc. can be used. These cell lines are further subjected to subculture in 8-azaguanine medium [medium prepared by adding 8-azaguanine (15 µg/ml) to a medium (referred to hereinafter as normal medium) prepared by adding glutamine (1.5 mmol/l), 2-mercaptoethanol (5×10⁻⁵ mol/l), gentamicin (10 µg/ml) and fetal calf serum (FCS) (manufactured by CSL Ltd.; 10%) to RPMI-1640 medium], and 3 to 4 days before cell fusion, they are cultured in the normal medium and at least 2×10⁷ cells are used for fusion.

### (c) Preparation of hybridoma

The antibody-producing cells obtained in above (a) and the myeloma cells obtained in above(b) are washed well with MEM or PBS (1.83 g of disodium hydrogen phosphate, 0.21 g of potassium dihydrogen phosphate, 7.65 g of sodium chloride, 1 L of distilled water, pH 7.2) and mixed such that the ratio of the antibody-producing cells/myeloma cells ranges from 5/1 to 10/1, and these cells are centrifuged at 1,200 rpm for 5 minutes and the supernatant is discarded.

The cell pellet obtained as the precipitated fraction is well loosened, and a mixture of 2 g of polyethylene glycol-1000 (PEG-1000), 2 ml of MEM and 0.7 ml of dimethyl sulfoxide (DMSO) is added to the cells in a volume of 0.2 to 1 ml/10⁸ antibody-producing cells with stirring at 37°C, and 1 to 2 ml of MEM is added thereto several times at 1-to 2-minute intervals.

After the addition, MEM is added to adjust the total volume to 50 ml. The suspension thus prepared is centrifuged at 900 rpm for 5 minutes, and the supernatant is discarded. The cells obtained in the precipitated fraction are gently loosened and suspended by pipetting in 100 ml of HAT medium [medium prepared by adding hypoxanthine (10⁻⁴mol/l), thymidine (1.5×10⁻⁵ mol/l) and aminopterin (4×10⁻⁷ mol/l) to the normal medium].

The suspension is dispensed on a 96-well culture plate at 100µl/well and cultured at 37°C in a 5% CO₂ incubator for 7 to 14 days.

After the culturing, an aliquot of the supernatant is sampled and a hybridoma reacting specifically with a partial fragment polypeptide of the protein of the present invention is selected by enzyme-linked immunosorbent assay described in, for example, Antibodies, ALaboratory Manual, Cold Spring Harbor Laboratory, Chapter 14 (1988).

Specifically, enzyme-linked immunosorbent assay is conducted as follows:

A partial fragment polypeptide of the protein of the present invention, whichwas used as the antigen for immunization, is coated on an appropriate plate and then allowed to react with a culture supernatant of the hybridoma or the purified antibody obtained in (d) below as a primary antibody and then with anti-rat or anti-mouse immunoglobulin antibody as a second antibody labeled with biotin, an enzyme, a chemiluminescent substance or a radioisotope, followed by reaction depending on the labeling substance, whereby a hybridoma whose culture supernatant reacting specifically with the polypeptide of the present invention is selected as that of a hybridoma producing the monoclonal antibody of the present invention.

Using the hybridoma, cloning is repeated twice by limiting dilution [for first dilution, HT medium (aminopterin-free HAT medium) is used; for second dilution, the normal medium is used], and a hybridoma showing a stable and high antibody titer is selected as a hybridoma strain producing the monoclonal antibody of the present invention.

Examples of the hybridoma strain of the present invention include hybridoma strains KM2954, KM2955, KM2956, KM2957 and KM2958. The hybridoma strain KM2955 has been deposited as FERM BP-7500 since March 13, 2001 with the National Institute of Bioscience and Human-Technology, Agency of Industrial Science and Technology (1-3, Higashi 1-chome, Tsukuba-shi, Ibaraki, Japan).

### (d) Preparation of a monoclonal antibody

The hybridoma cells producing a monoclonal antibody against the polypeptide of the present invention, obtained in (c), are injected intraperitoneally to 8 to 10-week-old mice or nude mice previously treated with Pristane [animal raised for 2 weeks after intraperitoneal administration of 0.5 ml 2,6,10,14-tetramethylpentadecane (Pristane)] at a dose of 5 to 20×10⁶ cells/animal. The hybridoma forms ascites tumor in 10 to 21 days.

From the mouse with the ascites tumor, the ascites is collected and centrifuged at 3,000 rpm for 5 minutes, to remove the solid matters from the fluid.

From the resulting supernatant, the monoclonal antibody can be purified and obtained according to the same method as used for the polyclonal antibody.

The subclass of the antibody is determined using a mouse monoclonal antibody typing kit or a rat monoclonal antibody typing kit. The amount of the polypeptide is calculated by the Lowry method or from its absorbance at 280 nm.

Examples of the monoclonal antibody of the present invention include the anti-KRGF-1 monoclonal antibodies KM2954, KM2955, KM2956, KM2957 and KM2958.

### 4. Preparation of a recombinant virus vector for producing the KRGF-1 protein

Hereinafter, the process for preparation of a recombinant virus vector for producing the KRGF-1 protein in specific human tissues is described.

On the basis of the full-length cDNA of the KRGF-1 gene, a DNA fragment of suitable length containing a region coding for the protein is prepared.

A recombinant virus vector is constructed by inserting the full-length cDNA or a fragment of the DNA into a site downstream from a promoter in a virus vector.

When an RNA virus vector is used, cRNA homologous with the full-length cDNA of the KRGF-1 gene, or an RNA fragment homologous with a DNA fragment of suitable length containing a region coding for the protein, is prepared and inserted into a site downstream from a promoter in a virus vector, whereby a recombinant virus is prepared. As the RNA fragment, double-stranded RNA or a single-stranded sense or antisense RNA is selected depending on the type of the virus vector. For example, RNA homologous with the sense chain is selected in the case of retrovirus vector, while RNA homologous with the antisense chain is selected in the case of Sendai virus vector.

The recombinant virus vector is introduced into packaging cells compatible with the vector.

For the recombinant virus vector deficient in at least one gene encoding a protein necessary for virus packaging, any cells capable of supplying the deficient protein can be used as the packaging cells, and for example, human kidney-derived HEK293 cells, mouse fibroblast NIH3T3 etc. can be used. The protein supplied by the packaging cells includes proteins such as mouse retrovirus-derived gag, pol, env etc. in the case of retrovirus vector, proteins such as HIV virus-derived gag, pol, env, vpr, vpu, vif, tat, rev, nef etc. in the case of lentivirus vector, proteins such as adenovirus-derived E1A-E1B in the case of adenovirus vector, proteins such as Rep (p5, p19, p40), Vp (Cap) etc. in the case of adeno-associated virus, and proteins such as NP, P/C, L, M, F, HN etc. in the case of Sendai virus.

As the virus vector, a vector that can produce the recombinant virus in the packaging cells and harbors a promoter at a position capable of transcribing DNA of the KRGF-1 gene in the target cells is used. As the plasmid vector, MFG [Proc. Natl. Acad. Sci. USA, 92, 6733 (1995)], pBaberuro [NucleicAcids Res., 18, 3587 (1990)], LL-CG, CL-CG, CS-CG, CLG [Journal of Virology, 72, 8150 (1998)], pAdexl [Nucleic Acids Res., 23, 3816 (1995)] etc. are used. As the promoter, any promoter capable of expression in human tissues can be used, and for example, a cytomegalovirus (human CMV) IE .(immediate early) gene promoter, an SV40 early promoter, a retrovirus promoter,. a metallothionein promoter, a heat shock protein promoter, an SR α promoter etc. can be exemplified. Furthermore, an enhancer of the IE gene of human CMV may be used in combination with the promoter.

As the method of introducing the recombinant virus vector into packaging cells the calcium phosphate method [Japanese Published Unexamined Patent Application No. 227075/1990], the lipofection method [Proc. Natl. Acad. Sci. USA, 84, 7413 (1987)] etc. can be exemplified.

### 5. Method of detecting and quantifying mRNA for the KRGF-1 gene

Using the DNA of the present invention, a gene participating in progress of a renal disease and recovery therefrom can be detected and quantified. The gene participating in progress of a renal disease and recovery therefrom includes known and unknown splicing variants of KRGF-1.

Hereinafter, the method of detecting and quantifying the mRNA for the KRGF-1 gene by use of KRGF-1 DNA of the present invention is described.

The DNA used in the method includes, for example, DNA having a nucleotide sequence represented by SEQ ID NO:2, 3, 16 or 17 or DNA fragments obtained therefrom.

The method of detecting the expression level and structural change of the mRNA for the KRGF-1 gene includes, for example, (1) Northern blotting, (2) in situ hybridization, (3) quantitative PCR, (4) differential hybridization, (5) DNA chip and (6) RNase protection assay.

As the sample subjected to analysis by the method described above, mRNA or a total RNA prepared from biological samples such as renal tissues, serum, saliva etc. obtained from patients with renal diseases and healthy persons, or from subsequent primary culture of the cells obtained from these biological samples in a suitable medium in a test tube is used (hereinafter, the mRNA and total RNA are referred to as sample-derived RNA). Paraffin or cryostat sections containing tissues isolated from the biological samples can also be used.

In Northern blotting, the sample-derived RNA is separated by gel electrophoresis, transferred to a support such as nylon filter, hybridized with a labeled probe prepared from the DNA of the present invention and washed to detect a band specifically binding to the mRNA for the KRGF-1 gene, whereby the expression level and structural change of the mRNA for the KRGF-1 gene can be detected. In hybridization, the probe and the mRNA for the KRGF-1 gene in the sample-derived RNA are incubated under such conditions as to form a stable hybrid. To prevent false positive reaction, hybridization and washing are conducted desirably under highly stringent conditions. These conditions are determined in terms of many factors such as temperature, ionic strength, nucleotide composition, probe length and formamide concentration. These factors are described in, for example, Molecular Cloning, 2nd edition.

The labeled probe used in Northern blotting can be prepared by incorporating a radioisotope, biotin, a fluorescent group, a chemiluminescent group or the like into the DNA of the present invention or an oligonucleotide designed on the basis of the sequence of the DNA by a known method (nick translation, random priming or kinasing). The amount of the labeled probe bound reflects the expression level of the mRNA for the KRGF-1 gene, and thus the expression level of the mRNA for the KRGF-1 gene can be quantified by quantifying the amount of the labeled probe bound. Further, the structural change of the mRNA for the KRGF-1 gene can be known by analyzing the site to which the labeled probe was bound.

The expression level of the mRNA for the KRGF-1 gene can be detected by in situ hybridization wherein the labeled probe and paraffin or cryostat sections containing tissues isolated from the biological samples are subjected to hybridization and washing. To prevent false positive reaction in in situ hybridization, the hybridization and washing are conducted desirably under highly stringent conditions. These conditions are determined in terms of many factors such as temperature, ionic strength, nucleotide composition, probe length and formamide concentration. These factors are described in for example Current Protocols in Molecular Biology.

The method of detecting the mRNA for the KRGF-1 gene by the quantitative PCR, the differential hybridization or DNA chip can be carried out by a method based on synthesis of cDNA by using the sample-derived RNA, oligo-dT primers or random primers and reverse transcriptase (hereinafter, the cDNA is referred to as sample-derived cDNA). When the sample-derived RNA is mRNA, any of the above primers can be used, but when the sample-derived RNA is total RNA, the oligo-dT primers should be used.

In quantitative PCR, a DNA fragment derived from the mRNA for the KRGF-1 gene is amplified in PCR by using the sample-derived cDNA as the template, together with primers designed on the basis of the nucleotide sequence of KRGF-1 DNA of the present invention. The amount of the amplified DNA fragment reflects the expression level of the mRNA for the KRGF-1 gene, and thus the amount of the mRNA for the KRGF-1 gene can be quantified by using a DNA encoding actin or glycerardehyde-3-phosphate dehydrogenase (abbreviated hereinafter to G3PDH) as an internal control. Further, the structural change of the mRNA for the KRGF-1 gene can be detected by separating the amplified DNA fragment by gel electrophoresis. In this detection method, suitable primers for specifically and efficiently amplifying the target sequence are desirably used. The suitable primers can be designed on the basis of conditions such as the absence of hybridization between primers or intramolecular hybridization in a primer, binding to the target cDNA specifically at the annealing temperature, and dissociation from the target cDNA under denaturing conditions. Quantification of the amplified DNA fragment should be carried out during the PCR cycles where the amplified product is increased exponentially. Such PCR cycle can be monitored by recovering the amplified DNA fragment produced in each reaction cycle and analyzing it quantitatively by gel electrophoresis.

A change in the expression level of the mRNA for the KRGF-1 gene can be detected by hybridizing the sample-derived cDNA with a filter or slide glass or a base such as silicon having the DNA of the present invention immobilized as the probe thereon and subsequent washing. In the method based on this principle, there is a method called differential hybridization [Trends in Genetics, 7, 314 (1991)] or DNA chip [Genome Research, 6, 639 (1996)]. In either method, a difference in expression of the mRNA for the KRGF-1 gene between the control sample and the target sample can be accurately detected by immobilizing an internal control such as actin or G3PDH on a filter or base. Further, labeled cDNAs are synthesized by using different labeled dNTP, on the basis of RNAs derived from the control sample and the target sample respectively, and the two labeled cDNA probes are hybridized simultaneously with one filter or one substrate thereby enabling the accurate quantification of the expression level of the mRNA for the KRGF-1 gene.

In the RNase protection assay, a promoter sequence such as T7 promoter or SP6 promoter is bound to the 3'-end of the DNA of the present invention, and labeled antisense RNA is synthesized by using labeled rNTP in an in vitro transcriptional system using RNA polymerase. The labeled antisense RNA is bound to the sample-derived RNA, to form an RNA-RNA hybrid which is then digested with RNase, and the RNA fragment protected from digestion is detected by forming a band in gel electrophoresis. By quantifying the resultant band, the expression level of the mRNA for the KRGF-1 gene can be quantified.

### 6. Method of detecting a gene causing a renal disease

Hereinafter, the method of detecting a gene causing a renal disease by use of KRGF-1 DNA of the present invention is described.

The DNA used in the method includes, for example, DNA having a nucleotide sequence represented by SEQ ID NO:2, 3, 16 or 17 or DNA fragments obtained therefrom.

The most evident test for evaluating the presence or absence of a renal disease-causing mutation occurring in a locus of the KRGF-1 gene is to compare a gene from a control group directly with a gene from a patient with a renal disease.

Specifically, a human biological sample such as renal tissues, serum, saliva etc. from patients with renal diseases and healthy persons or a sample derived from primary-cultured cells established from the biological sample is collected, and DNA is extracted from the biological sample and the sample derived from the primary-cultured cells (hereinafter, the DNA is referred to as sample-derived DNA). The sample-derived DNA, or KRGF-1 DNA amplified by primers designed on the basis of the nucleotide sequence of the DNA of the present invention, can be used as a sample DNA. In an alternative method, PCR is conducted where the sample-derived cDNA is used as the template, together with primers designed on the basis of the nucleotide sequence of the DNA of the present invention, whereby a DNA fragment containing the DNA sequence of the KRGF-1 gene can be amplified and used as a sample DNA.

As the method of detecting whether a mutation causing a renal disease is present in KRGF-1 DNA, a method of detecting heteroduplex formed by hybridizing a DNA chain having a wild-type allele with a DNA chain having a mutant allele can be used.

The method of detecting heteroduplex includes (1) a method of detecting heteroduplex by polyacrylamide gel electrophoresis [Trends Genet., 7, 5 (1991)], (2) a method of analyzing single strand conformation polymorphism [Genomics, 16, 325 (1993)], (3) a method of chemical cleavage of mismatches (CCM) [Human Molecular Genetics (1996), Tom Strachan and Andrew P. Read (BIOS Scientific Publishers Limited)], (4) a method of enzymatic cleavage of mismatches [Nature Genetics, 9, 103 (1996)], and (5) denaturing gel electrophoresis [Mutat. Res., 288, 103 (1993)] etc.

KRGF-1 DNA is amplified as a smaller fragment than 200 bp by using the sample-derived DNA or sample-derived cDNA as the template, along with primers designed on the basis of a nucleotide sequence represented by SEQ ID NO:2, 3, 16 or 17, and then subjected to polyacrylamide gel electrophoresis. When heteroduplex is formed due to a mutation in KRGF-1 DNA, the migration thereof is shorter than that of mutation-free homo double strands, so the hetero double strands can be detected as an excess band. Special gel (Hydro-link, MDE etc.) is used for higher separation. In examination of fragments smaller than 200 bp, it is possible to detect insertions, deletions and almost all one-base substitutions. Analysis of the hetero double strands is conducted desirably in one gel by combination with single strand conformation polymorphism analysis described below.

In single strand conformation polymorphism analysis (SSCP analysis), KRGF-1 DNA is amplified as a smaller fragment than 200 bp by using the sample-derived DNA or sample-derived cDNA as the template, along with primers designed on the basis of a nucleotide sequence represented by SEQ ID NO:2, 3, 16 or 17, and then denatured and electrophoresed in non-denaturing polyacrylamide gel. For amplification of the DNA, the primers are labeled with a radioisotope or a fluorescent coloring matter, or the unlabeled amplification product is silver-stained, whereby the amplified KRGF-1 gene DNA can be detected as a band. To clarify the difference thereof from the wild-type pattern, the control sample is also simultaneously electrophoresed whereby the fragment having a mutation can be detected owing to a difference in migration.

In chemical cleavage of mismatches (CCM), a DNA fragment obtained by amplifying KRGF-1 DNA by using the sample-derived DNA or sample-derived cDNA as the template, together with primers designed on the basis of a nucleotide sequence represented by SEQ ID NO:2, 3, 16 or 17 is hybridized with labeled DNA prepared by incorporating a radioisotope or a fluorescent dye into the DNA of the present invention, and then treated with osmium tetraoxide, whereby either chain of double strands is cleaved at a mismatched site, to enable detection of a mutation. CCM is one of the most sensitive methods and can be applied to a sample of kilo base length.

In place of osmium tetraoxide mentioned above, an enzyme (e.g. T4 phage resolvase or endonuclease VII) involving in repairing mismatches in cells may be combined with RNase A, to cleave mismatches enzymatically.

In the denaturing gradient gel electrophoresis (DGGE), a DNA fragment obtained by amplifying KRGF-1 DNA by using the sample-derived DNA or sample-derived cDNA as the template, together with primers designed on the basis of a nucleotide sequence represented by SEQ ID NO:2, 3, 16 or 17 is electrophoresed on gel having a concentration gradient of a chemical denaturant and a temperature gradient. The amplified DNA fragment migrates in gel at a position where it is denatured into a single-stranded chain, and after this denaturation, the DNA fragment does not migrate. Depending on whether a mutation is present or absent inKRGF-1 DNA, the migration of the amplified DNA in the gel is different, thus enabling detection of the presence of a mutation. For improving detection sensitivity, a poly (G:C) tail may be added to each primer.

As an alternative method of detecting a gene causing a renal disease, there is a protein truncation test (PTT) [Genomics, 20, 1 (1994)]. By this test, a frame shift mutation, a splicing site mutation and a nonsense mutation generating deficiency in protein can be specifically detected. In PTT, a special primer having a T7 promoter sequence and an eukaryote translation initiation sequence linked with the 5'-end of DNA having a nucleotide sequence represented by SEQ ID NO:2, 3, 16 or 17 is designed, and the sample-derived RNA is subjected to reverse transcription-PCR (RT-PCR) with the above primer, to prepare cDNA. Using this cDNA, in vitro transcription and translation are carried out to produce a protein. The protein is electrophoresed in gel, and when the protein migrates to a position corresponding to the full-length protein, there is no mutation generating deficiency, while when there is deficiency in the protein, the protein is electrophoresed at a shorter position than the full-length protein, so the degree of deficiency can be monitored from the position.

To determine the nucleotide sequences of the sample-derived DNA and sample-derived cDNA, primers designed on the basis of the nucleotide sequence of the DNA of the present invention can be used. By analyzing the determined nucleotide sequence, whether a mutation causing a renal disease occurs in the sample-derived DNA or sample-derived cDNA can be judged.

A mutation in the region other than the coding region in the KRGF-1 gene can be detected by examining a non-coding region such as intron and regulatory sequence around or in the gene. Renal diseases attributable to mutations in the non-coding region can be confirmed by detecting mRNA with abnormal size or abnormal expression levels in patients with renal diseases as compared with the control sample in the method described above.

The gene thus suggested to have a mutation in the non-coding region can be cloned by using DNA having a nucleotide sequence represented by SEQ ID NO:2, 3, 16 or 17 as the hybridization probe. A mutation in the non-coding region can be examined according to any of the methods described above.

The mutation thus found is processed statistically according to a method described in Handbook of Human Genetics Linkage, The John Hopkins University Press, Baltimore (1994), whereby the mutation can be identified as SNP (single nucleotide polymorphism) related to renal diseases. Further, DNA is obtained from a family having hospital records of a renal disease by the method described above, and by detecting its mutation, the gene causing the renal disease can be identified.

### 7. Method of diagnosing the possible occurrence and prognosis of renal diseases by use of KRGF-1 DNA

The DNA used in the method includes, for example, DNA having a nucleotide sequence represented by SEQ ID NO:2, 3, 16 or 17 or DNA fragments obtained therefrom.

The cause of the renal disease can be confirmed by detecting a mutation in the gene from any human tissues. For example, when the mutation occurs in the generative cell line, it is possible that a person who has inherited the mutation tends to easily have the onset of the renal disease. This mutation can be detected by examining DNA from any tissues in that person. For example, blood is collected, DNA is extracted from cells in the blood, and this DNA is used to examine the gene, whereby the renal disease can be diagnosed. Using fetal cells, placental cells or amnion cells, a mutation in the gene can be examined in prenatal diagnosis.

Further, biological tissues in lesions are obtained from a patient with a renal disease, and the type of the renal disease is diagnosed by examining the DNA, and medicines to be administered can be selected on the basis of the type of the disease. For detection of a mutation in the gene in tissues, isolation of tissues from lesions released from surrounding normal tissues is useful. Renal tissues in a patient with a renal disease can be excised by biopsy. The tissues thus obtained are treated with trypsin, and the resultant cells are cultured in a suitable medium. From the cultured cells, chromosomal DNA and mRNA can be extracted.

Hereinafter, the DNA obtained for diagnostic purposes from human samples by any one of the methods described above is referred to as diagnosis sample-derived DNA. In addition, cDNA synthesized from RNA obtained for diagnostic purposes from human samples by any one of the methods described above is referred to hereinafter as diagnosis sample-derived cDNA.

KRGF-1 DNA and the diagnosis sample-derived DNA or diagnosis sample-derived cDNA can be used in diagnosis of renal diseases by a method which is in accordance with the above-described method of detecting the gene causing a renal disease.

In diagnosis of renal diseases by utilizing KRGF-1 DNA and the diagnosis sample-derived DNA or diagnosis sample-derived cDNA, methods such as (1) detection of restriction enzyme sites, (2) a method of utilizing an allele specific oligonucleotide probe (ASO: allele specific oligonucleotide hybridization), (3) PCR using an allele specific oligonucleotide (ARMS: amplification refractory mutation system), (4) oligonucleotide ligation assay (OLA), (5) PCR-PHFA assay (PCR-preferential homoduplex formation assay), and (6) a method of using an oligo DNA array ["Tanpakushitsu Kakusan Koso" (Protein, Nucleic Acid and Enzyme), 43, 2004 (1998)] can be used.

In the case where a restriction enzyme site is deleted or produced by a change in one base, the diagnosis sample-derived DNA or diagnosis sample-derived cDNA is amplified by primers designed on the basis of the sequence of the DNA of the present invention, and then digested with the restriction enzyme, and the resultant DNA fragment cleaved with the restriction enzyme is compared with that from a healthy person, whereby the mutation can be easily detected. However, the change in one base rarely occurs, so for diagnostic purposes the mutation is detected by reverse dot blotting wherein hybridization is carried out after an oligonucleotide probe is designed by combining the information on the sequence of the DNA of the present invention with the information on the separately identified mutation, and the oligonucleotide probe is bound to a filter for hybridization.

A short synthetic DNA probe hybridizes with a completely pairing sequence only. By utilizing this property, one-base mutation can be easily detected using an allele specific oligonucleotide probe (ASO). For diagnostic purposes, reverse dot blotting is often conducted wherein hybridization with a probe prepared by PCR using labeled dNTP and primers designed using the sequence of the DNA of the present invention from the diagnostic sample-derived DNA or diagnostic sample-derived cDNA is carried out after an oligonucleotide designed on the basis of the sequence of the DNA of the present invention and the identified mutation is bound to a filter for hybridization. The DNA chip method wherein an oligonucleotide designed on the basis of the mutation and the sequence of the DNA of the present invention is synthesized directly on a slide glass or a base such as silicon to form high-density arrays thereon is a mutation-detecting method suitable for large-scale diagnostic purposes because various mutations can be easily detected using a small amount of the diagnostic sample-derived DNA or diagnostic sample-derived cDNA.

A mutation in nucleotides can also be detected by the following oligonucleotide ligation assay (OLA).

Two oligonucleotides each having about 20 nucleotides, between which a mutation is to be sandwiched, are designed and prepared on the basis of the sequence of the DNA of the present invention. KRGF-1 DNA fragment is amplified by PCR wherein the diagnostic sample-derived DNA or diagnostic sample-derived cDNA is used as the template, together with primers designed from the sequence of KRGF-1 DNA. The amplified fragment is hybridized with the above oligonucleotides. After the hybridization, the two oligonucleotides are ligated by DNA ligase. For example, if one oligonucleotide is labeled with biotin while the other oligonucleotide is labeled with another label such as digoxigenin, whether the ligation reaction occurs or not can be rapidly detected. OLA is a mutation-detecting method suitable for efficient diagnosis of a large number of samples in a short time without requiring electrophoresis or centrifugation.

Further, following PCR-PHFA can quantitatively and easily detect a very small amount of a mutant gene.

PCR-PHFA is a combination of 3 techniques i.e. polymerase chain reaction (PCR), hybridization in a liquid phase showing very high specificity, and ED-PCR (enzymatic detection of PCR product) for detecting a PCR product in the same manner as in ELISA. A pair of dinitrophenyl (DNP)-labeled and biotinylated primers are used in PCR amplification using the DNA of the present invention as the template, to prepare an amplification product labeled at both ends thereof. An unlabeled amplification product is obtained by amplification using a pair of label-free primers having sequences identical with the labeled primers, together with the diagnostic sample-derived DNA or diagnostic sample-derived cDNA as the template, and then mixed in 20- to 100-fold large excess to the above labeled amplification product. After thermal denaturation, the sample is cooled in a gentle temperature gradient of about 1°C/5 minutes to 10 minutes, whereby a completely complementary chain is preferentially formed. The labeled DNA formed again in this manner is adsorbed via biotin onto a streptoavidin-immobilized well and bound via DNP to an enzyme-labeled anti-DNP antibody to permit detection thereof by chromogenic reaction with the enzyme. When a gene having the same sequence as the labeled DNA is not present in the sample, the original double-stranded labeled DNA is preferentially formed again to exhibit coloration. On the other hand, when a gene having the same sequence is present, substitutions of the complementary chain occur at random, and thus the labeled DNA formed again is reduced, thus significantly reducing coloration. Accordingly, detection and quantification of known mutation and polymorphisms of a gene became possible.

### 8. Method of detecting and quantifying the KRGF-1 protein immunologically by an antibody specifically recognizing the KRGF-1 protein

The method of immunologically detecting and quantifying microorganisms, mammalian cells, insect cells or tissues expressing the KRGF-1 protein intracellularly or extracellularly by using an antibody (polyclonal or monoclonal antibody) specifically recognizing the KRGF-1 protein of the present invention includes immunofluorescence technique, enzyme-linked immunosorbent assay (ELISA), radioimmunoassay (RIA), immunohistochemistry (ABS, CSA etc.) such as tissue immunostaining and cell immunostaining, Western blotting, dot blotting, immunoprecipitation, and sandwich ELISA techniques ["Tankuron Kotai Jikken Manual" (Monoclonal Antibody Experiment Manual) (Kodansha Scientific) (1987), "Zoku Seikagaku Jikken Koza" (Sequel to Lecture of Experiments in Biochemistry), Vol. 5, "Meneki Seikagaku Kenkyuho" (Method for Study in Immunological Biochemistry) (Tokyo Kagaku Dojin) (1986)].

Immunofluorescence technique is a method wherein the antibody of the present invention is reacted with microorganisms, mammalian cells, insect cells or tissues expressing the KRGF-1 protein intracellularly or extracellularly, then an anti-mouse IgG antibody or a fragment thereof labeled with a fluorescent material such as fluorescein isothiocyanate (FITC) is reacted therewith, and the fluorescent dye is measured by a flow cytometer.

Enzyme-linked immunosorbent assay (ELISA) is a method wherein the antibody of the present invention is reacted with microorganisms, mammalian cells, insect cells or tissues expressing the KRGF-1 protein intracellularly or extracellularly, then an anti-mouse IgG antibody or a binding fragment thereof labeled with an enzyme such as peroxidase and biotin is reacted therewith, and the coloring matter is measured by a spectrophotometer.

Radio immunoassay (RIA) is a method wherein the antibody of the present invention is reacted with microorganisms, mammalian cells, insect cells or tissues expressing the KRGF-1 protein intracellularly or extracellularly and then an anti-mouse IgG antibody or a fragment thereof labeled with a radioisotope is reacted therewith, followed by measurement with a scintillation counter or the like.

Cell immunostaining or tissue immunostaining is a method wherein the antibody specifically recognizing the KRGF-1 protein is reacted with microorganisms, mammalian cells, insect cells or tissues expressing the KRGF-1 protein intracellularly or extracellularly and then an anti-mouse IgG antibody or a fragment thereof labeled with a fluorescent material such as FITC or an enzyme such as peroxidase or biotin is reacted therewith, followed by observation under a microscope.

Western blotting is a method wherein an extract of microorganisms, mammalian cells, insect cells or tissues expressing the KRGF-1 protein intracellularly or extracellularly is fractionated by SDS-polyacrylamide gel electrophoresis [Antibodies - A Laboratory Manual, Cold Spring Harbor Laboratory (1988)], then the gel is blotted onto a PVDF membrane or nitrocellulose membrane, the antibody of the present invention specifically recognizing the KRGF-1 protein is reacted with the membrane, an anti-mouse IgG antibody or a fragment thereof labeled with a fluorescent material such as FITC or an enzyme such as peroxidase or biotin is reacted therewith for confirmation.

Dot blotting is a method wherein an extract of microorganisms, mammalian cells, insect cells or tissues expressing the KRGF-1 protein intracellularly or extracellularly is blotted onto a nitrocellulose membrane, the antibody of the present invention is reacted with the membrane, and an anti-mouse IgG antibody or a binding fragment thereof labeled with a fluorescent material such as FITC or an enzyme such as peroxidase or biotin is reacted therewith for confirmation.

Immunoprecipitation is a method wherein an extract of microorganisms, mammalian cells, insect cells or tissues expressing the KRGF-1 protein intracellularly or extracellularly is reacted with the antibody of the present invention specifically recognizing the KRGF-1 protein, and a carrier (e.g. protein G-Sepharose) having an ability to specifically bind to immunoglobulins is added to precipitate the antigen-antibody complex.

Sandwich ELISA is a method wherein one of two monoclonal antibodies, each specifically recognizing the KRGF-1 protein of the present invention and having a different recognition site, is adsorbed onto a plate while the other is labeled with a fluorescent material such as FITC or an enzyme such as peroxidase or biotin, and an extract of microorganisms, mammalian cells, insect cells or tissues expressing the KRGF-1 protein intracellularly or extracellularly is reacted with the antibody-adsorbed plate, and the labeled antibody is reacted therewith, followed by reaction depending on the labeling substance.

By utilizing the fact that the monoclonal antibody of the present invention specifically recognizes the KRGF-1 protein, the protein of the present invention can be isolated and purified by techniques such as affinity chromatography using the monoclonal antibody.

### 9. Method of separating KRGF-1-expressing cells by the antibody specifically recognizing the KRGF-1 protein

The monoclonal antibody specifically recognizing the KRGF-1 protein, whichwas obtained in the method described above, can be used for isolating KRGF-expressing cells from the kidney by a panning method or FACS (fluorescence activated cell sorter) method. In either method, excised renal tissues should be physically cut into thin pieces and incubated in the presence of trypsin or neutral protease to separate the cells.

In the FACS method, separated cells derived from the kidney are reacted with the monoclonal antibody specifically recognizing the KRGF-1 protein of the present invention and then reacted with a fluorescence-labeled secondary antibody specifically recognizing the monoclonal antibody, followed by sorting the cells by fluorescence intensity with FACS. The resultant positive cells are proliferated again in vitro to remove false-positive cells followed by repeating sorting. From the cells thus concentrated, the total RNA is recovered, and whether the objective cells could be obtained or not is confirmed by the RT-PCR method described above.

The panning method is a method wherein separated cells derived from the kidney are reacted with the monoclonal antibody, and cells expressing the objective surface antigen are collected specifically by means of a plate coated with a secondary antibody specifically recognizing the monoclonal antibody. After panning is repeated twice to remove false-positive cells, whether the objective cells could be obtained or not is confirmed in the same manner as in the FACS method.

### 10. Method of diagnosing renal diseases by using the antibody specifically recognizing the KRGF-1 protein

Identification of the expression level of the KRGF-1 protein and the structural change of the expressed protein in human biological samples and human primary-cultured cells is useful for examining the possibility of the onset of renal diseases in the future or the cause of previously occurring renal diseases.

The method of diagnosis by detecting the expression level and structural change of the KRGF-1 protein includes the above-mentioned immunofluorescence technique, enzyme-linked immunosorbent assay (ELISA), radioimmunoassay (RIA), immunohistochemistry (ABC method, CSA method etc.) such as tissue immunostaining and cell immunostaining, Western blotting, dot blotting, immunoprecipitation and sandwich ELISA.

As the sample subjected to analysis by themethod described above, biological samples such as tissues from lesions of renal diseases, blood, serum, urine, feces, saliva etc. obtained from patients, or cells and cell extracts obtained from the biological samples are used. Paraffin or cryostat sections containing tissues isolated from the biological samples can also be used.

### 11. Method of screening a therapeutic agent for renal diseases by using the KRGF-1 protein, the DNA encoding the protein, or the antibody recognizing the protein

The DNA used in this screening method includes, for example, a DNA having a nucleotide sequence represented by SEQ ID NO:2, 3, 16 or 17; the protein includes a protein having an amino acid sequence selected from an amino acid sequence represented by SEQ ID NO:1, or a protein having an amino acid sequence wherein one or more amino acids are deleted, substituted or added in the amino acid sequence represented by SEQ ID NO:1 and having an activity participating in formation of lesions in renal diseases and recovery therefrom; and the antibody includes an antibody recognizing the protein.

The microorganisms, mammalian cells, or insect cells transformed with KRGF-1 DNA of the present invention to produce the KRGF-1 protein of the present invention or a polypeptide constituting a part of the KRGF-1 protein, as well as the purified KRGF-1 protein or KRGF-1 polypeptide, are useful for screening agents acting specifically on the KRGF-1 protein. The agents obtained by screening are useful for treatment of renal diseases.

One method of screening is to select a target compound specifically binding to the microorganisms, mammalian cells or insect cells transformed to produce the KRGF-1 protein of the present invention or a polypeptide constituting a part of the KRGF-1 protein (referred to hereinafter as screening transformant). By comparison with non-transformed microorganisms, mammalian cells or insect cells as the control group, the specific target compound can be detected. Further, the target compound can be competitively screened by using its inhibitory action on the binding, to the screening transformant, of another compound or protein which specifically binds to the screening transformant as an index.
the purified KRGF-1 protein of the present invention or the purified polypeptide constituting a part of the KRGF-1 protein can be used for selecting the target compound specifically binding to the KRGF-1 protein. The target compound can be quantified in the immunological method by using the antibody specifically recognizing the KRGF-1 protein of the present invention. Further, the target compound can be competitively screened by using its inhibitory action as an indicator on the binding, to the KRGF-1 protein or the KRGF-1 polypeptide, of another compound specifically binding to the KRGF-1 protein or the KRGF-1 polypeptide.

Another method of screening is a method wherein a large number of peptides each constituting a part of the KRGF-1 protein are synthesized at high density on plastic pins or a certain kind of solid support, and a compound or protein binding specifically to the peptides can be efficiently screened (WO84/03564).

An expression-regulating agent which promotes expression of the mRNA for the KRGF-1 gene or the KRGF-1 protein in a cell line derived from the kidney is also useful for treatment of renal diseases.

A substance inhibiting or promoting transcription or translation of the KRGF-1 gene can be screened by adding various test compounds to a cell line derived from the kidney and then examining a change in the expression level of the mRNA for the KRGF-1 gene by means of KRGF-1 DNA of the present invention. A change in the expression level of the mRNA for the KRGF-1 gene can be detected by the above-described PCR, Northern blotting, and RNase protection assay.

A substance promoting transcription or translation of the KRGF-1 gene can be screened by adding various test compounds to a cell line derived from the kidney and then examining a change in the expression level of the KRGF-1 protein by means of the antibody specifically recognizing the KRGF-1 protein of the present invention. A change in the expression level of the KRGF-1 protein can be detected by the above-described immunofluorescence technique, enzyme-linked immunosorbent assay (ELISA), radioimmunoassay (RIA), immunolohistochemistry (ABC method, CSA method etc.) such as tissue immunostaining and cell immunostaining, Western blotting, dot blotting, immunoprecipitation and sandwich ELISA.

The therapeutic effect of the compound obtained in the above-described method on renal diseases can be evaluated by administering it as an agent into animal models of renal diseases, such as Thy-1 nephritis rats, anti-GBM nephritis, serum sickness-type nephritis, PAN nephritis, daunomycin nephritis, 5/6 kidney-excised rats, spontaneous occurring loops nephritis etc. and then measuring urinary proteins and albumins in the animals.

### 12. Method of delivering a drug specifically to the kidney by using the antibody specifically recognizing the KRGF-1 protein (drug delivery method)

The antibody used in the drug delivery method may be any antibody recognizing the KRGF-1 protein of the present invention, and in particular, a humanized antibody is preferably used.

The humanized antibody includes, for example, CDR (complementary determining region, abbreviated hereinafter to CDR)-grafted humanized antibody and human chimeric antibody.

The human chimeric antibody refers to an antibody consisting of a non-human antibody heavy-chain variable region (also referred to hereinafter as HV or VH wherein H is a heavy chain and V is a variable region), a non-human antibody light-chain variable region (also referred to hereinafter as LV or VL wherein L is a light chain), a human antibody heavy-chain constant region (also referred to hereinafter as CH wherein C is a constant region) and a human antibody light-chain constant region (also referred to hereinafter as CL). As the non-human animal, use can be any animals such as mice, rats, hamsters, rabbits etc. insofar as they can produce a monoclonal antibody-producing hybridoma.

The human chimeric antibody of the present invention can be produced by obtaining cDNA encoding VH and VL from a hybridoma producing a monoclonal antibody binding to the KRGF-1 protein and neutralizing the function of the protein of the present invention, then inserting an mammalian expression vectors harboring genes encoding human antibody CH and human antibody CL respectively to construct a human chimeric antibody expression vector, and introducing the vector into mammalian cells thereby expressing and producing the desired human chimeric antibody.

The CH of the human chimeric antibody may be any one belonging to human immunoglobulin (referred to hereinafter as hIg), preferably the one of hIgG class, and further any subclasses such as hIgG1, hIgG2, hIgG3 and hIgG4 belonging to the hIgG class can be used. The CL of the human chimeric antibody may be any one belonging to hIg, and those of κ class or λ class can be used.

The CDR-grafted humanized antibody means an antibody wherein the amino acid sequences of CDRs in VH and VL of the non-human antibody are transplanted at suitable positions in VH and VL of the human antibody respectively.

The CDR-grafted humanized antibody of the present invention can be produced by constructing cDNAs encoding V regions wherein CDR sequences in VH and VL of an arbitrary human antibody are replaced by CDR sequences in VH and VL of a non-human antibody reacting with the KRGF-1 protein of the present invention, binding to the KRGF-1 protein of the present invention and neutralizing the function of the KRGF-1 protein of the present invention, then inserting the cDNAs into an mammalian expression vector harboring genes encoding human antibody CH and human antibody CL respectively to construct a CDR-grafted humanized antibody expression vector, and introducing the vector into mammalian cells thereby expressing and producing the desired CDR-grafted antibody.

The CH of the CDR-grafted humanized antibody may be any one belonging to hIg, preferably the one of hIgG class, and further any subclasses such as hIgG1, hIgG2, hIgG3 and hIgG4 belonging to the hIgG class can be used. The CL of the CDR-grafted humanized antibody may be any one belonging to hIg, and those of κ class or λ class can be used.

The human antibody originally means an antibody naturally occurring in the human body, but also includes antibodies obtained from a human antibody phage library and a human antibody-producing transgenic animal prepared by recent progress in genetic engineering, cell engineering and developmental engineering.

The antibody occurring in the human body can be obtained for example in the following method.

Lymphocytes are isolated from human peripheral blood, immortalized by infection with, for example, EB virus, and cloned. The resulting lymphocytes producing the objective antibody are cultured, and the antibody can be obtained from the culture.

The human antibody phage library is a library wherein antibody fragments such as Fab, single-chain antibody etc. are expressed on phages by inserting the antibody gene prepared from human B cells into a phage gene. The phage expressing an antibody fragment having the desired antigen-binding activity can be recovered by using, as an index, the activity of binding to a base having the antigen immobilized thereon. The antibody fragment can be converted into a complete human antibody by genetic engineering means.

The human antibody-producing transgenic animal means an animal having the human antibody gene integrated in its cells. Specifically, the human antibody-producing transgenic animal can be created by introducing the human antibody gene into mouse ES cells and transplanting the ES cells into mouse early embryos followed by development thereof. The method of creating the human antibody from the human antibody-producing transgenic animal includes a method wherein the human antibody-producing hybridoma is obtained by a conventional method of preparing a hybridoma for non-human mammals , and the hybridoma is cultured to thereby produce and accumulate the human antibody in the culture.

The antibody fragments include Fab, Fab', F(ab')2, single-chain antibody, a disulfide-stabilized V region fragment (also referred to hereinafter as dsFv), and a CDR-containing peptide.

Among the fragments (cleaved at an amino acid residue at the 224 position in the H chain) obtained by treating IgG with a proteinase papain, Fab is an antibody fragment with a molecular weight of about 50,000 having an antigen binding activity, which comprises an N-terminal half of the H chain bound via a disulfide linkage to the whole of the L chain.

The Fab of the present invention can be obtained by treating the antibody specifically reacting the protein of the present invention with a proteinase papain. Alternatively, the Fab can be obtained by inserting a DNA encoding the Fab of the antibody into a prokaryotic expression vector or eukaryotic expression vector, then introducing the vector into prokaryotes or eukaryotes and expressing the DNA.

Among the fragments (cleaved at an amino acid residue at the 234 position in the H chain) obtained by treating IgG with a proteinase pepsin, F(ab')2 is an antibody fragment with a molecular weight of about 100,000 having an antigen binding activity, which is slightly larger than that of Fab bound via a disulfide linkage in the hinge region.

The F(ab')2 of the present invention can be obtained by treating the antibody reacting specifically with the protein of the present invention with a proteinase pepsin. Alternatively, the F(ab')2 can be obtained by inserting a DNA encoding F(ab' )2 of the antibody into a prokaryotic expression vector or eukaryotic expression vector, then introducing the vector into prokaryotes or eukaryotes and expressing the DNA.

Fab' is an antibody fragment with a molecular weight of about 50,000 having an antigen binding activity, obtained by cleaving the F(ab')2 at the position of a disulfide linkage in the hinge region.

The Fab' of the present invention can be obtained by treating the antibody reacting specifically with the protein of the present invention with a reducing agent dithiothreitol. Alternatively, the Fab' can be obtained by inserting DNA encoding the Fab' fragment of the antibody into a prokaryotic expression vector or eukaryotic expression vector, then introducing the vector into prokaryotes or eukaryotes and expressing the DNA.

The single-chain antibody (also referred to hereinafter as scFv) is a VH-P-VL or VL-P-VH polypeptide having one VH linked with one VL via a suitable peptide linker (referred to hereinafter as P). The VH and VL contained in scFv used in the present invention can make use of those derived from the antibody (for example, the humanized antibody or the human antibody) reacting specifically with the protein of the present invention.

The single-chain antibody of the present invention can be obtained in the following method.

After cDNAs encoding VH and VL of the antibody reacting specifically with the protein of the present invention are obtained, a DNA encoding the single-chain antibody is constructed. The DNA is inserted into a prokaryotic expression vector or eukaryotic expression vector, and the expression vector is introduced into prokaryotes or eukaryotes, and the DNA is expressed therein, whereby the single-chain antibody can be obtained.

The disulfide-stabilized V region fragment (dsFv) refers to a fragment comprising VH and VL polypeptides wherein one amino acid residue in each polypeptide is substituted by a cysteine residue between which both the polypeptides are bound to one another via the disulfide-binding. The amino acid residue substituted by the cysteine residue can be selected on the basis of the predicted stereostructure of the antibody according to a method shown by Reiter et al. [Protein Engineering, 7, 697 (1994)]. The VH and VL contained in dsFv used in the present invention can make use of those derived from the antibody (for example, the humanized antibody or the human antibody) reacting specifically with the protein of the present invention.

The disulfide-stabilized V region fragment (dsFv) of the present invention can be obtained in the following method.

After cDNAs encoding VH and VL of the antibody specifically reacting with the protein of the present invention is obtained, a DNA encoding dsFv is constructed. The DNA is inserted into a prokaryotic expression vector or eukaryotic expression vector, the vector is introduced into prokaryotes or eukaryotes, and the DNA is expressed, whereby dsFv can be obtained.

The CDR-containing peptide can be produced by chemical synthesis methods such as Fmoc method, tBoc method etc.

The following fusion antibody prepared from the antibody of the present invention can be used in drug delivery for delivering an agent or protein to lesions in the kidney.

The fusion antibody refers to an antibody comprising an agent such as a radioisotope, a protein, a low-molecular-weight compound etc. bound chemically or by genetic engineering to the antibody reacting specifically with the protein of the present invention, for example to the humanized antibody, the human antibody or an antibody fragment thereof.

The fusion antibody of the present invention can be produced by binding an agent such as a radioisotope, a protein, a low-molecular-weight compound etc. chemically or by genetic engineering to the N- or C-terminal of H or L chain of the antibody reacting specifically with the protein of the present invention, or of an antibody fragment thereof, to a suitable substituent group or a side chain of the antibody or an antibody fragment thereof, or to a sugar chain of the antibody or an antibody fragment thereof.

The radioisotopes include ¹³¹I, ¹²⁵I etc., which can be bound to the antibody or antigen fragments thereof by, for example, the chloramine T method.

The low-molecular-weight compound includes anticancer agents, for example alkylating agents such as nitrogen mustard and cyclophosphamide, antimetabolites such as 5-fluorouracil and mesotrexate, antibiotics such as daunomycin, bleomycin, mitomycin C, daunorubicin and doxsorubicin, plant alkaloids such as vincristine, vinblastine and vindecine, and hormones such as tamoxyphene and dexamethasone [Clinical Oncology (edited by Japanese Society for Study in Clinical Oncology, published in 1996 by "Gan To Kagakuryoho Sha"], or antiinflammatory agents for example steroids such as hydrocortisone and prednisone, non-steroids such as aspirin and indomethacin, immune regulators such as aurothiomalate and penicillamine, immuosuppresants such as cyclophosphamide and azathiopurine, and antihistamine agents such as chlorpheniramine maleate and clemastine ("Ensho To Koensho Ryoho" ("Inflammations and Antiinflammatory. Therapy") published in 1982 by Ishiyaku Shuppan).

The low-molecular-weight compound can be bound to the antibody in a usual manner, and for example, the method of binding daunomycin to the antibody includes a method of binding an amino group of daunomycin via glutaraldehyde to an amino group of the antibody and a method of binding an amino group of daunomycin via water-soluble carbodiimide to a carboxyl group of the antibody.

The protein includes cytokines which activates immunocompetent cells and growth-regulating factors in vascular endothelium and vascular smooth muscles, and for example, human interleukin 2, human granulocyte-macrophage-colony stimulating factor, human macrophage colony stimulating factor, human interleukin 12, FGF-2, PDGF etc.

The fusion antibody with the protein can be obtained in the following method.

A cDNA encoding a desired protein is ligated to the cDNA encoding the antibody or an antibody fragment thereof, to construct a DNA encoding the fusion antibody. The DNA is inserted into a prokaryotic or eukaryotic expression vector, the expression vector is introduced into prokaryotes or eukaryotes, and the DNA is expressed, whereby the fusion antibody can be obtained.

### 13. Therapeutic agent for renal diseases comprising the KRGF-1 protein

The KRGF-1 protein of the present invention can be used for re-constructing the structure and functions of the kidney in a patient with a renal disease including nephritis.

The therapeutic agent for renal diseases, comprising the KRGF-1 protein, may comprise the protein only as the active ingredient, but preferably the protein is mixed with one or more pharmacologically acceptable carriers and provided as a pharmaceutical preparation produced by a well-known arbitrary method in the technical field of pharmaceutical manufacturing.

The administration route is preferably the most effective route for treatment, and includes oral administration and parenteral administration such as intra-oral cavity administration, intratracheal administration, intrarectal administration, subcutaneous administration, intramuscular administration and intravenous administration. The administration form includes sprays, capsules, tablets, granules, syrups, emulsions, suppositories, injections, ointments, tapes etc.

The pharmaceutical preparation suitable for oral administration includes emulsions, syrups, capsules, tablets, powders and granules. For example, liquid preparations such as emulsions and syrups can be produced by using water, saccharides such as sucrose, sorbitol and fructose, glycols such as polyethylene glycol and propylene glycol, oils such as sesame oil, olive oil and soybean oil, preservatives such as p-hydroxybenzoate and flavors such as strawberry flavor and peppermint as additives. The capsules, tablets, powders and granules can be produced by using excipients such as lactose, glucose, sucrose and mannitol, disintegrating agents such as starch and sodium alginate, lubricants such as magnesium stearate and talc, binders such as polyvinyl alcohol, hydroxypropyl cellulose and gelatin, surfactants such as fatty acid esters and plasticizers such as glycerin as additives.

The pharmaceutical preparation suitable for parenteral administration includes injections, suppositories, sprays etc. For example, the injections are prepared by using carriers such as saline solution, sucrose solution or a mixture of the two. The suppositories are produced by using carriers such as cocoa butter, hydrogenated fats or carboxylic acids. The sprays are prepared from the protein of the present invention as such or by using carriers for dispersing the protein as fine particles to facilitate absorption thereof without stimulating the oral cavity or tracheal mucous membrane of a recipient. Specific examples of such carriers include lactose, glycerin etc. Depending on the properties of the protein and the carriers used, preparations such as aerosols, dry powders etc. can be manufactured. In these parenteral preparations, the ingredients exemplified as additives in the oral preparations can also be added.

The dose and administration frequency are varied depending on the type of the disease, administration method, period of treatment, age, body weight etc., but the dose is usually 10 mg/kg to 8 mg/kg per day in an adult.

### 14. Gene therapeutic agent comprising the KRGF-1 gene

The gene therapeutic agent using a virus vector comprising the KRGF-1 gene of the present invention can be prepared by mixing the recombinant vector prepared in 4. with a base material to be used in the gene therapeutic agent [Nature Genet., 8, 42 (1994)].

The base material used in the gene therapeutic agent may be any base material used usually in injections, and examples thereof include distilled water, salt solutions of sodium chloride or a mixture of sodium chloride and an inorganic salt, sugar solutions of mannitol, lactose, dextran, glucose etc., amino acid solutions of glycine, arginine etc., and mixed solutions such as organic acid solution or a mixed solution of a salt solution and a glucose solution. Further, additives such as osmotic pressure regulators, pH adjusters, vegetable oils such as sesame oil, soybean oil etc., lecithin and surfactants such as nonionic surfactants may be used in these base materials to prepare injections in the form of solution, suspension or dispersion. By powdering and lyophilizing procedures, these injections can also. be prepared as preparations dissolved just before use. The gene therapeutic agent of the present invention can be used in therapy as it is in the case of a solution or just after dissolution thereof in the optionally sterilized base materials in the case of a solid. The method of administering the gene therapeutic agent of the present invention includes a method of topical administration to allow it to be adsorbed into lesions in the kidney of the patient.

As a system of delivering the virus vector more specifically to lesions in the kidney, there is a method of using a fusion protein between a single-chain antibody specifically recognizing BMP-7 receptor and an Env protein of retrovirus vector [Proc. Natl. Acad. Sci. USA, 92, 7570 (1995)]. This system is not limited to retrovirus vector, and can also be adapted to rentivirus vector etc.

The KRGF-1 DNA of the present invention of suitable size is combined with an adenovirus hexon protein-specific polylysine-conjugate antibody to prepare a complex, and the resultant complex is bound to an adenovirus vector, whereby a virus vector can be prepared. The virus vector stably reaches the target cell and can be incorporated via endosomes into the cell and decomposed in the cell to express the gene efficiently.

A virus vector based on Sendai virus which is a (-) chain RNA virus has also been developed (Japanese Patent Application Nos. 517213/1997 and 517214/1997), and for the purpose of gene therapy, a Sendai virus vector comprising the KRGF-1 gene integrated therein can be prepared.

KRGF-1 DNA can also be delivered to kidney lesions by a non-viral gene transfer method.

The known method of transferring anon-virus gene includes a calcium phosphate co-precipitation method [Virology, 52, 456 (1973); Science, 209, 1414 (1980)], a microinjection method [Proc. Natl. Acad. Sci. USA, 77, 5399 (1980); Proc. Natl. Acad. Sci. USA, 77, 7380 (1980); Cell, 27, 223 (1981); Nature, 294, 92 (1981)], a method of transfer through membrane fusion via liposomes [Proc. Natl. Acad. Sci. USA, 84, 7413 (1987); Biochemistry, 28, 9508 (1989); J. Biol. Chem., 264, 12126 (1989); Hum. Gene Ther., 3, 267 (1992); Science, 249, 1285 (1990); Circulation, 83, 2007 (1992)] or a method of direct incorporation of DNA and receptor-mediated DNA transfer [Science, 247, 1465 (1990); J. Biol. Chem., 266, 14338 (1991); Proc. Natl. Acad. Sci. USA, 87, 3655 (1991); J. Biol. Chem., 264, 16985 (1989); BioTechniques, 11, 474 (1991); Proc. Natl. Acad. Sci. USA, 87, 3410 (1990); Proc. Natl. Acad. Sci. USA, 88, 4255 (1991); Proc. Natl. Acad. Sci. USA, 87, 4033 (1990); Proc. Natl. Acad. Sci. USA, 88, 8850 (1991); Hum. Gene Ther., 3, 147 (1991)] etc.

In the method of transfer through membrane fusion via liposomes, it has been reported in a study on tumors that a liposome preparation is directly administered into target tissues to enable topical incorporation of a gene into the tissues and expression thereof [Hum. Gene Ther. 3, 399 (1992)]. Accordingly, the same effect can be expected in kidney lesions. For direct targeting of the DNA at kidney lesions, the method of direct incorporation of the DNA is preferred. The receptor-mediated DNA transfer is conducted for example by conjugating the DNA (usually in the form of a covalently closed-circular supercoiled plasmid) with a protein ligand via polylysine. The ligand is selected depending on its corresponding ligand receptor occurring on the surface of the target cells or tissues. The combination of a receptor and its ligand includes, for example, a combination of BMP-7 receptor and BMP-7. The ligand-DNA conjugate can be directly injected into vessels if desired, to allow it to be directed toward the target tissues where receptor binding and DNA-protein complex are made inherent. For preventing destruction of the DNA in the cells, endosome functions can be destroyed by simultaneous infection with an adenovirus.

### 15. Therapeutic agent for renal diseases comprising the antibody specifically recognizing the KRGP-1 protein

The antibody specifically recognizing the KRGF-1 protein of the present invention can inhibit and regulate functions such as biological activity of the protein, and can be directly used to treat diseases such as renal cancers promoting neoplasia in the kidney.

The therapeutic agent comprising the antibody specifically recognizing the KRGF-1 protein of the present invention may comprise the antibody only as the active ingredient, but desirably the therapeutic agent of the present invention is usually provided as a pharmaceutical preparation produced by an arbitrary method known in the field of pharmacology by mixing the protein with one or more pharmaceutically acceptable carriers. Preparation of the therapeutic agent and administration thereof can be carried out according to the agent comprising the KRGF-1 protein in item 13 above.

### 16. Creation of knockout non-human animals by using KRGF-1 DNA

Using a recombinant vector comprising the DNA of the present invention, mutant clones in which the DNA encoding the protein of the present invention on the chromosome is inactivated or replaced by an arbitrary sequence by known homologous recombination techniques [e.g., Nature, 326, 6110, 295 (1987), Cell, 51, 3, 503 (1987), etc.] are produced from embryonic stem cells in objective non-human animals such as cattle, sheep, goats, pigs, horses, mice, chickens etc. [e.g., Nature, 350, 6315, 243 (1991)]. The embryonic stem cells thus produced and blastocyst of fertilized eggs of animals can be used for producing chimeras composed of embryonic stem cell clones and normal cells by techniques such as injection chimera method, aggregation chimera method etc. By crossing the chimeras with normal individuals, chimeras individuals in which the DNA encoding the protein of the present invention on the chromosome in cells in the whole body was arbitrarily mutated can be obtained, and by crossing the individuals, knockout non-human animals wherein expression of the DNA encoding the protein of the present invention is partially or completely inhibited can be obtained from homozygotes having the mutation on both homologous chromosomes.

Alternatively, knockout non-human animals can be prepared by introducing a mutation into an arbitrary site in the DNA encoding the protein of the present invention on the chromosome. For example, the translated region of the DNA encoding the protein of the present invention on the chromosome can be mutated by nucleotide substitutions, deletions, insertions etc. to alter the activity of its product. Further, by similar mutation of its expression-regulating region, the level of expression, stage, tissue specificity etc. can also be modified. Furthermore, by combination with the Cre-loxP system, the expression stage, expression site, expression level etc. can also be regulated efficiently. By way of example, it is known that by using a promoter to be expressed in a specific region in the brain, an objective gene is deleted in only that region [Cell, 87, 7, 1317 (1996)], or by using adenovirus expressing Cre, an objective gene is deleted in an organ-specific manner at a desired stage [Science, 278, 5335 (1997)].

It is therefore possible to create knockout non-human animals in which expression of the DNA encoding the protein of the present invention on the chromosome can be regulated at an arbitrary stage or in a specific organ as described above, or the translated region or expression-regulating region has arbitrary insertions, deletions or substitutions.

In such knockout non-human animals, various diseases attributable to the protein of the present invention can be inducted at an arbitrary stage, at an arbitrary level or in an arbitrary site.

Accordingly, the knockout non-human animal of the present invention can serve as a very useful animal model in treating or preventing various diseases attributable to the protein of the present invention. In particular, the animal is very useful as a model for evaluation of agents for treating or preventing the diseases or functional foods, health foods etc.

### Brief Description of the Drawings

Fig. 1 is a profile in analysis by Northern blotting showing distribution of rat KRGF-1 expression in several tissues.
Fig. 2 is a profile in Western blotting analysis of the reactivity, with rat KRGF-1, of antiserums prepared by using 3 peptide antigens TRDH091-1, TRDH091-2 and TRDH091-3, which were derived from partial peptides in KRGF-1.
Fig. 3 is a profile in analysis of the reactivity of monoclonal antibodies KM2954, KM2955, KM2956, KM2957 and KM2958 with KRGF-1 by Western blotting.
Fig. 4 is a profile in analysis of immunoprecipitation of KRGF-1 secreted and expressed in CHO cells, with monoclonal antibodies KM2954, KM2955, KM2956, KM2957 and KM2958.
Fig. 5 is a profile in analysis of the expression of a MBP-KRGF-1 fusion protein consisting of MBP and KRGF-1 in Escherichia coli.
Fig. 6 is a profile of the MBP-KRGF-1 fusion protein expressed in Escherichia coli and purified by amylose.
Fig. 7 is a profile in analysis of intracellular location of KRGF-1 expressed in CHO cells.
Fig. 8 is a profile of a purified preparation of KRGF-1 expressed in CHO cells, which was obtained from CHO cells (A) and a culture supernatant (B).
Fig. 9 is a profile of a change, upon treatment with N-glycanase, in the molecular weight of KRGF-1 expressed in CHO cells, which was obtained from the CHO cells and a culture supernatant thereof.

### Best Mode for Carrying Out the Invention

Hereinafter, the present invention is described in more detail by reference to the Examples, which are not intended to limit the present invention.

### Example 1: Preparation of Thy-1 nephritis rat kidney cDNA library

Anti-rat Thy-1 monoclonal antibody OX-7 (manufactured by Cedarlane) was administered by tail vein injection to 20 Wistarrats (males) (manufactured by Japan SLC Inc., body weight; approximately 200 g) at a dose of 1mg/kg, thereby inducing nephritis. Physiological saline was administered to a control group. For these rats, the polypeptide concentration in urine was measured using urine analysis stick pretest 6B (manufactured by wako Pure Chemical Industries), and was used as an index of nephritis condition.

On each of days 2, 4, 6, 8, 10, 13 and 16 after administration of OX-7, kidney was extracted from 3 rats (on day 16 only, from 2 rats), as well as from rats of the control group, and total RNA was extracted from each individual by a guanidinium thiocyanate-cesium trifluoroacetate method [Methods in Enzymology, 154, 3 (1987)]. For the control group, total RNA was not extracted from each individual, and instead total RNA was extracted from a mixture comprising equal amounts of renal tissue lysate prepared by treating the kidneys of 3 individuals on day 2 after administration of physiological saline and renal tissue lysate prepared by treating the kidneys of 3 individuals on day 10 after administration. Of these total RNAs, the total RNA of each individual rat kidney of days 2, 4, 6, 8 and 10 after OX-7 administration were mixed together in equal amounts, and poly (A) RNA was prepared by using oligo(dT) cellulose. Then, using ZAP-cDNA Synthesis Kit (manufactured by Stratagene), a cDNA library of total independent plaques of 1.0 x 10⁶ was prepared (Thy-1 nephritis rat kidney cDNA library). Details of the method for preparing the cDNA are as described in the kit manual. This cDNA library is inserted between the Xho I/EcoR I sites of the vector in such a way that the 5' end of cDNA was on the EcoR I site side, with using λ phage vector λ ZAP II (manufactured by Stratagene) as a vector.

### Example 2: Preparation of subtracted library

### (1) Preparation of single stranded DNA

By infecting host cell Escherichia coli XL1-Blue MRF' (manufactured by Stratagene) with the Thy-1 nephritis rat kidney cDNA library prepared in Example 1 together with helper phage ExAssist (manufactured by Stratagene) and performing in vivo excision, a phagemid pBluescript SK(-) region containing cDNA was excised from the vector as a single stranded DNA phage, and was released into a culture supernatant. The method of in vivo excision was performed in accordance with Strategene's manual. 200 µl of this culture supernatant (titer: 8.5 x 10⁵ cfu/µl) was added to 7ml of 10 mmol MgSO₄ containing 1.8 x 10¹⁰ Escherichia coli SORL (manufactured by Stratagene) as a host cell which cannot be infected with ExAssist, and incubated at 37°C for 15 minutes. The total volume was added to 200 ml of 2x YT culture medium (1.6% Bacto-tryptone, 1% yeast extract), and the mixture was cultured with shaking for 1 hour at 37°C, and was infected with single stranded DNA phage containing cDNA. Ampicillin was added thereto at a concentration of 50 µg/ml, the mixture was again subjected to shaking culture for 1 hour at 37°C, and only phage-infected Escherichia coli was proliferated. The number of the cells was measured by an absorbance of 600 nm, and the result was 4 x 10¹⁰ cells. Helper phage R408 (manufactured by Stratagene) was added at multiplicity of infection (moi) = 13 (5.3 x 10¹¹ pfu), and the mixture was cultured with shaking for 7 hours at 37°C to release single stranded DNA again in the supernatant. The culture medium was transferred to a sterile tube, centrifuged for 10 minutes at 10,000 rpm at 4°C, and only supernatant containing phage was transferred and recovered in a new sterile tube. After centrifugation of this supernatant under the same conditions, the cells were completely removed by passing through a sterile filter of 0.22 mm pore size (manufactured by Millipore). 20 ml of 10x buffer [100 mmol/l Tris-HCl (pH 7.5), 100 mmol/l MgCl₂] and 140 units of deoxyribonuclease I (manufactured by Nippon Gene) were added, and the mixture was reacted at 37°C for 30 minutes. 2.5 mol/l NaCl solution containing 1/4 volume of 20% polyethyleneglycol (molecular weight 6,000) was added thereto, and the mixture was mixed well at room temperature and allowed to stand for 20 minutes, and then centrifuged for 10 minutes at 10,000 rpm at 4°C to precipitate phage. The supernatant was completely removed, and the obtained phage precipitate was dissolved in 400 µl of TE [10 mmol/l Tris-HCl (pH 8.0), 1 mmol/l EDTA (pH 8.0)]. Then, 4 µl of 10% SDS and 625 µg (25 µl) of proteinase K were added thereto, and the mixture was allowed to react for 1 hour at 42°C. After phenol extraction, phenol-chloroform extraction and chloroform extraction, the aqueous layer was precipitated by ethanol, and 77.6 µg of single stranded DNA [vector pBluescript SK(-)] of Thy-1 nephritis rat kidney cDNA library was obtained.

### (2) Biotinylation of RNA

From 1.2 mg of total RNA prepared from kidney of control group rat of Example 1, 20 µg of poly(A) RNA was prepared by using oligo(dT) cellulose. To 10 µg of it was added distilled water to bring to 20 µl in a test tube, and 30 µg (30 µl) of 1 mg/ml PHOTOPROBE biotin (manufactured by Vector Laboratories) was added thereto in a dark place. The cap of the test tube was opened and the tube was placed on ice, and RNA was biotinylated by light irradiation for 20 minutes using a mercury lamp from a height of about 10 cm. After addition of 50 µl of a solution of 100 mmol/l 1 Tris-HCl (pH 9.5) and 1 mmol/l EDTA (pH 8.0) to the reaction solution, extraction with water saturated butanol was performed 3 times, and chloroform extraction was performed twice, and then the aqueous layer was precipitated with ethanol. The recovered RNA precipitate was dissolved in 20 µl of distilled water, the above-described biotinylation reaction (procedure from addition of PHOTOPROBE biotin to precipitation with ethanol) was performed once more to obtain biotinylated RNA.

### (3) Subtraction

To 0.5 µg (1 µl) of single stranded DNA of the Thy-1 nephritis rat kidney cDNA library prepared in (1) was added 12.5 µl of 2x hybridization buffer [80% formamide, 100 mmol/l HEPES (pH 7.5), 2 mmol/l EDTA (pH 8.0), 0.2% SDS], 2.5 µl of 2.5 mol/l NaCl, and 1 µg (1 µl) of poly(A) (manufactured by Amersham Pharmacia Biotech), and then 8 µl of biotinylated RNA (RNA 10µg) prepared in (2) was dissolved in distilled water and added. After the mixture was heated at 65°C for 10 minutes, hybridization was performed at 42°C for 63 hours.

To the solution after hybridization reaction was added 400 µl of buffer [500 mmol/l NaCl, 50 mmol/l HEPES (pH 7.5), 2 mmol/l EDTA (pH 8.0)], and 10 µg (5 µl) of streptavidin (manufactured by Life Technologies) was then added thereto, and the mixture was allowed to react at room temperature for 5 minutes. Phenol-chloroform extraction was performed and a complex of streptavidin-biotinylated RNA-hybridized cDNA was removed from the aqueous layer. 10 µg of streptavidin was again added to the aqueous layer and allowed to react at room temperature for 5 minutes, and phenol-chloroform extraction was performed twice, after which chloroform extraction was performed and the aqueous layer recovered. After passing the aqueous layer through Unit Filter Ultra. Free C3 Plus TK (manufactured by Millipore) to allowed cDNA adsorbed in the filter and washing, concentration and desalting of cDNA was performed by recovering in 30 µl of 1/10 TE [1 mmol/l Tris-HCl (pH8.0), 0.1 mmol/l EDTA (pH 8.0)]. Procedures using this filter were performed in accordance with Millipore's manual. By this subtraction, cDNA of a gene whose expression amount was large in both Thy-1 nephritis rat kidney and control group rat kidney was removed from the cDNA library, and cDNA of a gene which was expressed in Thy-1 nephritis rat kidney but almost not expressed in control group rat kidney was concentrated. However, use of only the above subtraction would result also in the concentration of cDNA of a gene which was expressed at a very low level in Thy-1 nephritis rat kidney but almost not expressed in control group rat kidney. Therefore, reverse subtraction as described in (5) below was performed, and a library was prepared that did not include cDNA of a gene expressing at a very low level in Thy-1 nephritis rat kidney.

### (4) Amplification of cDNA after subtraction

Since it was considered that the quantity of the cDNA had decreased considerably following the subtraction of (3), in order to perform the reverse subtraction described in (5) below, the quantity was increased in the following manner. 14 µl of distilled water and 2 µg (1 µl) of 5'-AP primer was added to a half amount (15 µl) of the cDNA (single-stranded DNA) after subtraction. After heating for 10 minutes at 65°C, the mixture was left to stand at room temperature for 5 minutes to anneal the primer to the single-stranded DNA. 5 µl of 10x BcaBEST reaction buffer [which is attached with BcaBEST Dideoxy Sequencing Kit (manufactured by Takara Shuzo)], 10 µl of 1 mmol/l dNTP (mixture of 1 mmol/l each of dATP, dGTP, dCTP, and TTP), 1.5 µg (0.5 µl) of single-stranded DNA binding polypeptide (manufactured by USB), 4 units (2 µl) of BcaBEST DNA polymerase (manufactured by Takara Shuzo) and 2.5 µl of distilled water were added thereto, and the solution was allowed to react for 1 hour at 65°C to synthesize double-stranded DNA. 50 µl of distilled water was added to the reaction solution, and phenol-chloroform extraction and chloroform extraction were performed. Then, double stranded DNA was finally recovered in 20 µl of TE by using a Unit Filter Ultra Free C3 Plus TK in the same manner as described in (3).

The total amount (4 µl) of the recovered double-stranded DNA was introduced into Escherichia coli DH12S (manufactured by Life Technologies) by electroporation. To the Escherichia coli DH12S after the electroporation was added 1.5 ml of SOC culture medium, and this was then inoculated into 42.5 ml of LB-Ap culture medium (1% Bacto-tryptone 0.5% yeast extract, 1% NaCl, 50 µg/ml ampicillin). The titer at this stage was 4.3 x 10⁶ cfu. After culturing at 37°C for 4 hours, the number of the cells were measured by absorbance of 600nm, and the result was 1-1.5 x 10⁸ cells/ml. Dimethyl sulfoxide was added to the half amount of the culture at a concentration of 7%, and this was stored at -80°C. The remaining half amount of the culture was infected with helper phage R408 of moi = 14-20 (5 x 10⁸ pfu). After culturing at 37°C for 15 minutes, each 5 ml of the mixture was inoculated into 5 x 45 ml of 2x YT culture medium, and then cultured at 37°C. 2 hours and 30 minutes after the start of culturing, ampicillin was added at a concentration of 100 µg/ml, and this was then cultured for a further 5 hours and 30 minutes to release single-stranded DNA phage in the culture solution. In the same manner as (1), 30.8 µg of single strand DNA was purified from this culture solution.

### (5) Reverse subtraction

2.5 µg of poly(A) RNA of Thy-1 nephritis rat kidney prepared in Example 1 was biotinylated in the same manner as in (2). This biotinylated RNA was added to 2.5 µg of the single strand DNA after subtraction prepared in (4), and distilled water was added thereto to bring to 9 µl. Added thereto was 12.5 µl of 2x hybridization buffer which was the same as that used in the subtraction of (3), 2.5 µl of 2.5 mol/l NaCl, and 1 µg (1 µl) of poly(A). After heating the mixture at 65°C for 10 minutes, hybridization was performed at 42°C for 59 hours.

Streptavidin was reacted with the solution after hybridization reaction in the same manner as in the subtraction of (3). Phenol-chloroform extraction was performed to remove the aqueous layer, and a phenol-chloroform layer containing a complex of biotinylated RNA of Thy-1 nephritis rat kidney and hybridized cDNA was recovered. After repeating 3 times the operation of addition of TE and extraction, TE was again added to the phenol-chloroform layer, and the mixture was heated at 95°C for 5 minutes, to thereby dissociate the biotinylated RNA and cDNA. After the reaction layer was cooled quickly by immersion in ice water, the solution was stirred vigorously and dissociated cDNAwas extracted into an aqueous layer. After heating this solution once more for 5 minutes at 95°C, the cooling quickly and extraction were repeated, and an aqueous layer containing dissociated cDNA was recovered by centrifugation. After performing phenol-chloroform extraction and chloroform extraction for the aqueous layer, the aqueous layer was passed through Unit Filter Ultra Free C3 Plus TK in the same manner as in (3). cDNA was adsorbed in the filter and washed, and concentration and desalting of cDNA was performed by recovering cDNA in 30 µl of 1/10 TE.

### (6) Preparation of cDNA library

For the single-stranded cDNA after reverse subtraction obtained in (5), after making half amount into double stranded DNA in the same manner as in (4), 1/8 of the amount was introduced into Escherichia coli DH12S by electroporation to prepare a reverse subtraction cDNA library. From the analysis using one portion of the library, it was estimated that the number of independent colonies of the library was 2.5 x 10⁴ cfu and that the ratio of cDNA insertion was 98%.

### Example 3: Differential Hybridization

### (1) Preparation of array filters

Using the reverse-subtraction cDNA library prepared in (6) of Example 2, colonies were formed on LB-Ap agar medium, and 9,600 colonies among them were inoculated onto one hundred 96-well plates in which 100 µl of LB-Ap culture medium had been added, at 1 colony/well. After each colony was cultured in the 96-well plates at 37°C, 50 µl of 50% glycerol was added thereto and the colonies were then stored at -80°C (this storage culture solution is hereinafter referred to as "glycerol stock").

Onto 96-well plates, each well of which contains 100 µ l of LB-Ap culture medium, glycerol stock was again inoculated using 96 pin replicators, and the plates were left to stand for culturing overnight at 37°C. Using an automatic microdispenser, Hydra 96, a culture solution containing this Escherichia coli was spotted in spots of 0.5 µl each on nylon membranes in the same lattice formation as the 96-well plate (8 vertically x 12 horizontally). On one nylon membrane were spotted 384 colonies in a lattice formation (16 vertically x 24 horizontally), which corresponded to the total amount of four 96-well plates, and one colony was spotted in the same position on 2 membranes so that 2 of the same membranes could be prepared. Membranes spotted with culture solution were placed on LB-Ap agar medium, with the spotted surface upward, and were cultured overnight at 37°C.

After the membranes on which colonies of Escherichia coli had grown sufficiently were stripped from the culture medium, the membranes were placed on paper soaked with DNA denaturing solution (0.5 mol/l NaOH, 1.5 mol/l NaCl), and left at room temperature for 10 minutes to denature DNA. The membranes were then transferred to paper soaked with neutralizing solution [1.0 mol/l Tris-HCl (pH 7.5), 1.5 mol/l NaCl] and left at room temperature for 10 minutes. After abrasively washing the cell clusters on the membrane in a sufficient amount of 2 x SSC (0.3 mol/l sodium chloride, 30 mmol/l sodium citrate) containing 0.5% SDS which was prepared in a bath, washing was performed by replacing the same buffer two times. Membranes were transferred to polyethylene bags, a reaction buffer [50 mol/l tris-hydrochloric acid (pH8.5), 50 mol/l EDTA, 100 mol/l sodium chloride, 1% sodium lauroyl sarcosinate] in which proteinase Kwas dissolved at a concentration of 250 µg/ml was added thereto, and the bags were sealed, and reaction was carried out for 2 hours at 37°C. After the membranes were removed from the bags and washed with 2 x SSC, the membranes were once again put into polyethylene bags, 2 x SSC containing proteinase inhibitor Pefabloc (manufactured by Roche) at a concentration of 400 µ g/ml was added thereto, and the bags were sealed and treated at room temperature for 1 hour. The membranes were removed from the bags and washed with 2 x SSC. Finally, DNA was immobilized on the membranes by ultraviolet irradiation using crosslinker Optimal Link (manufactured by Funakoshi). The thus obtained membranes are referred to as "array filters."

### (2) Preparation of riboprobes

From poly(A) RNA of Thy-1 nephritis rat kidney and control group rat kidney prepared in Example 1, digoxigenin (DIG) labeled riboprobes were prepared in the following manner. Since the number of membranes is large and a large quantity of probes is required, 150 µg of probes is necessary for performing hybridization of 50 membranes of 100 cm². Firstly, double stranded cDNA was prepared from poly(A) RNA, and T7 RNA polymerase reaction was carried out with employing this cDNA as a template, to thereby obtain riboprobes incorporated with DIG.

### (2)-1 Preparation of double stranded cDNA

Five µg of each poly(A) RNA was mixed with 8 µg of T7(dT) primer (SEQ ID NO: 161; having T7 promoter sequence at 5' end), and distilled water (pure water that was distilled a further 2 times, the same applies hereinafter) was added to bring the mixture to 7.8 µl. The mixture was heated at 70°C for 10 minutes and was quenched on ice. Four µl of 5x hybridization buffer (a buffer attached with commercially available enzyme), 2 µ l of 100 mmol/l DTT and 1.2 µl of 10 mmol/l dNTP was added thereto, and the mixture was mixed well by pipetting. After incubating at 37°C for 2 minutes to perform annealing, 5 µl of Superscript II reverse transcriptase (manufactured by Life Technologies) was added, and the reaction was carried out at 42°C for 1 hour to synthesize single strand cDNA, and then the mixture was cooled with ice.

To the solution after reaction were added 92.3 µl of distilled water, 32 µl of 5 x hybridization buffer [94 mmol/l tris-hydrochloric acid (pH 6.9), 453 mmol/l potassium chloride, 23 mmol/l magnesium chloride, 750 µmol/1β-NAD, 50 mmol/l ammonium sulfate], 3 µl of 10 mmol/l dNTP, 6 µl of 100 mmol/l DTT, 15 units (2.5 µl) of Escherichia coli DNA ligase (manufactured by Takara Shuzo), 40 units (11.5 µl) of Escherichia coli DNA polymerase I (manufactured by Takara Shuzo), and 1.2 units (2 µl) of Escherichia coli ribonuclease H (manufactured by Takara Shuzo), in that order. After mixing well by pipetting on ice, the mixture was allowed to react at 16°C for 2 hours and 30 minutes to synthesize double stranded cDNA. The Escherichia coli DNA ligase and Escherichia coli ribonuclease H were used after diluting with a 1x reaction buffer immediately before the reaction to dilute the stock solutions to 6 units/µl and 0.6 units/µl, respectively. After reaction, 2 µl of 0.5 mol/l EDTA and 2 µl of 10% SDS were added to stop the reaction, and an aqueous layer was recovered by phenol-chloroform extraction. Then, 70 µl of TE was further added to the phenol-chloroform layer excluding the aqueous layer, and extraction was carried out, and the aqueous layer was combined with the aqueous layer recovered earlier. To this aqueous layer was added 12 µl of proteinase K (2mg/ml), and this was allowed to react at 42°C for 1 hour. After recovering an aqueous layer by phenol-chloroform extraction in the solution after reaction, 70 µl of TE was added to the phenol-chloroform layer excluding the aqueous layer and extracted, and the aqueous layer was combined with the aqueous layer recovered earlier.

Using Unit Filter Ultra Free C3LTK (manufactured by Millipore), the aqueous layer was subjected to concentration and desalting. Specifically, the aqueous layer was placed in a filter cup and centrifuged at 8,000 rpm for 5 minutes, thereby adsorbing DNA in the filter. Solution which moved to the lower part was removed, and 300 µl of distilled water was placed in the filter cup, which was again centrifuged at 8,000 rpm for 5 minutes, thereby washing the filter. After repeating this washing operation once more, 25 µl of distilled water was placed in the filter cup, and suspended by pipetting to extract DNA. The filter cup was taken out and inserted upside-down into a tube for centrifugation (Falcon 2059), which was then centrifuged to collect the suspension in the bottom of the tube. 25 µl of distilled water was placed in the filter cup once more, and the suspension was recovered in the same manner (total of 50 µl).

### (2)-2 Synthesis and labeling of RNA

From double stranded cDNA obtained in the above-described manner, DIG labeled riboprobes were prepared using DIG RNA Labeling Kit (manufactured by Roche). The method was in accordance with Roche's DIG System Users Guide. Specifically, 20 µl of a mixture of 1 µg of cDNA (prepared to 14 µl with distilled water), 2 µl of 10x reaction buffer (buffer included in kit), 2 µl of NTP labeling mix (included in kit, and containing DIG-11-UTP) and 2 µl of T7 RNA polymerase (manufactured by Roche) was mixed by pipetting, and then allowed to react at 37°C for 2 hours. After stopping the reaction by adding 0.8 µl of 0.5 mol/l EDTA, 2.3 µl (1/9 volume of reaction solution) 4mol/l lithium chloride and 65 µl ethanol (2.5-3 times volume of reaction solution) were added, and the mixture was left at -80°C for 30 minutes (alternatively, at -20°C overnight) to precipitate RNA. After centrifugation at 4°C, the supernatant was removed, and the precipitate was washed with 70% ethanol, and air-dried in a clean bench, and was then dissolved in 100 µl distilled water. The yield of synthesized riboprobe was assayed in accordance with Roche's DIG System Users Guide.

### (3) Hybridization

The method of hybridization and detection of hybridized spots as well as the reagents were adopted in accordance with Roche's DIG System Users Guide.

To 20 ml of hybridization buffer [5x SSC, 0.1% sodium lauroyl sarcosinate, 0.02% SDS, 2% blocking agent (manufactured by Roche), 50% formamide] heated to 50°C, was added 1 mg (final concentration 50 µg/ml) of Poly(U) (manufactured by Amersham Pharmacia Biotech) which had been cooled quickly after heating at 95°C for 5 minutes. This was sealed together with a membrane in hybridization bag, and pre-hybridization was performed at 50°C for 2 hours. The hybridization buffer was transferred to a tube, and 5-6 µg of riboprobe (final concentration 0.25-0.3 µg/ml) which was cooled quickly after heating for 5 minutes at 95°C was added to the hybridization buffer and mixed. Then, the mixture was returned to a polyethylene bag, and the bag was sealed again. Hybridization was performed at 50°C for 1 night to 3 days, while shaking in such way that the filter moved within the bag (approximately 12 rpm). Since 2 membranes spotted with the same DNA in (1) were prepared, 1 membrane was hybridized with a riboprobe of Thy-1 nephritis rat kidney and 1 membrane was hybridized with a riboprobe of control group rat kidney.

### (4) Detection of spots

The membranes were removed from the hybridization bags and washed with 2x SSC containing 0.1% SDS at 68°C for 10 minutes. Then, the membranes were washed again in the same condition with using a fresh washing solution. Then, the washing at 68°C for 15 minutes with 2x SSC containing 0.1% SDS was repeated two times.

After equilibrating the membranes by soaking them for 1 minutes in a small amount of buffer 1 [0.1-5 mol/l NaCl, 0.1 mol/l maleic acid, (pH 7.5)], the membranes were sealed in a polyethylene bag together with buffer 2 [buffer prepared by dissolving the blocking agent (manufactured by Roche) in buffer 1 at a final concentration of 1%] of an amount such that the membranes could move, and gently shaken at room temperature for 1 hour or more to perform blocking. After transferring buffer 2 in the polyethylene bags into a tube and adding alkaline phosphatase labeled anti-DIG antibody Anti-DIG-AP (manufactured by Roche) in a volume of 1/10,000 and mixing, the mixture was returned to the bag and the bag was resealed. Then, the reaction was carried out while shaking gently at room temperature for 30 minutes to 1 hour. The membranes were removed from the bag, and was then subjected to 2 repetitions of washing while shaking for 15 minutes using buffer 1 added with 0.3% Tween 20. After equilibrating the membranes on opened bag by soaking them for 2 minutes in a small amount of buffer 3 [0.1 mol/l Tris (pH9.5), 0.1 mol/ NaCl, 50 mmol/l MgCl₂], luminous alkaline phosphatase substrate CSPD (manufactured by Roche) diluted to 100 times with buffer 3 was applied on the membrane surface at 0.5-1.0ml per 100 cm². After the membrane was covered with a bag from the top, and the substrates were spread uniformly over the membrane surface, and reaction was allowed for 5 minutes. After excess moisture was removed, the bags were sealed, and reaction was allowed at 37°C for 15 minutes. Then, X-ray film, Hyperfilm ECL (manufactured by Amersham Pharmacia Biotech), was exposed, and the film was developed. Exposure time was adjusted in such a way that the background concentration was the same level for those hybridized with riboprobe of Thy-1 nephritis rat kidney or riboprobe of control group rat kidney.

454 clones which had strongly hybridized with riboprobe of Thy-1 nephritis rat kidney in comparison with riboprobe of control group rat kidney were selected. Clones were identified from their array position, and each clone was cultured from the respective glycerol stock prepared in Example 3 (1), and plasmid DNA was prepared.

### Example 4: Analysis of nucleotide sequence and expression

### (1) Analysis of nucleotide sequence

The nucleotide sequence of cDNA from each of the 454 clones selected by differential hybridization in Example 3 was analyzed from the terminal of the nucleotide sequence by a DNA sequencer. These nucleotide sequences were examined for homology with sequences in a nucleotide sequence data base GenBank, EMBL or GeneSeq (Derwent) by an analysis program BlastN, to select cDNA which might code for a novel secretory protein.

### (2) Analysis of expression

### (2)-1. Northern hybridization

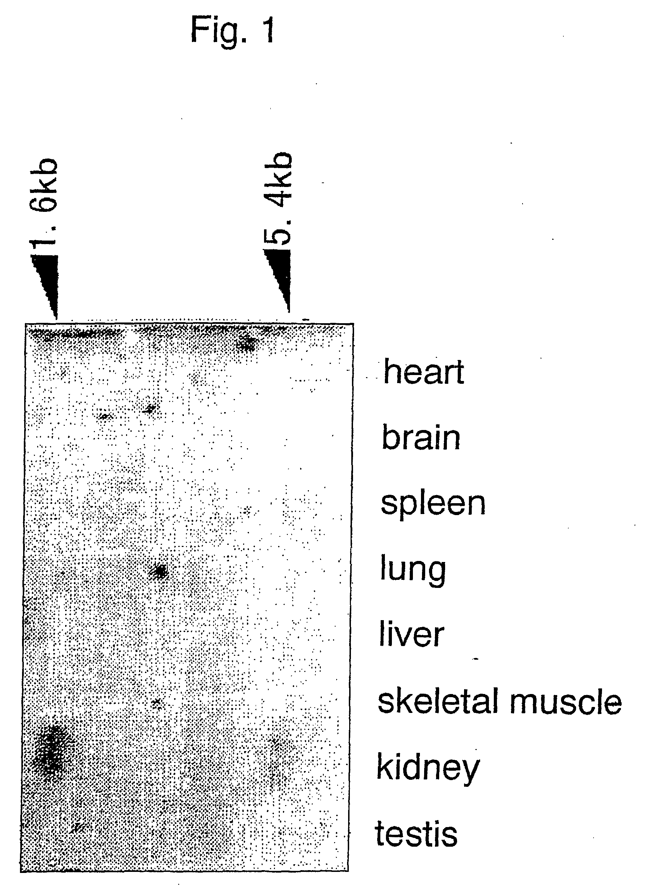

The cDNA clone selected in (1) was used as the probe in Northern hybridization with the whole RNA of rat organs (Rat Multiple Tissue Northern, manufactured by Clontech), indicating that expression of mRNA hybridizing with a cDNA clone designated TRDH-091 was limited almost to the kidney, and the presence of about 1.6 kb and about 5.4 kb molecular species was revealed. The result is shown in Fig. 1.

### (2)-2. Semi-quantitative RT-PCR

Time dependency change in the expression level of mRNA hybridizing with the cDNA from the TRDH-091 clone, in the kidney of the Thy-1 nephritis rat, was examined by RT-PCR comparing it with the expression level in the control rat. As the template, 5 µg each of the whole RNAs prepared in Example 1 from the kidney of each rat on Days 2, 4, 6, 8, 10, 13 and 16 after administration of anti-Thy-1 antibody OX-7, a mixture of these whole RNAs (abbreviated to Thy-1 mix), and the whole RNA from the kidney of the control rat administered with physiological saline were used to synthesize single-stranded cDNAs by using SUPERSCRIPT Preamplification System for First Strand cDNA Synthesis Kit (manufactured by Life Technologies) according to a manual of the kit, and each cDNA was dissolved finally in 250 µl of a distilled water. On the basis of the nucleotide sequence of the TRDH-091 clone, a pair of forward and reverse PCR primers (SEQ ID NOS:4 and 5) having a nucleotide sequence specific to the cDNA and permitting amplification of the 19-to 520-positions in the nucleotide sequence of SEQ ID NO:16 were designed and synthesized. In PCR, 1 µl of single-stranded cDNA (corresponding to 20 ng total RNA) serving as the template, 2 µl of 10×reaction buffer (attached to rTaq), 2 µl of 2.5 mmol/l dNTP, 1 µl of 10 µmol/l forward primer, 1 µl of 10 µmol/l reverse primer, 12.8 µl of a distilled water, and Taq DNA polymerase rTaq (manufactured by Takara Shuzo Co., Ltd.) were mixed, heated at 94°C for 5 minutes, and subjected to 20 reaction cycles each consisting of 94°C for 1 minute (denaturation)/64°C for 1 minute (annealing)/72°C for 1 minute (extension reaction) using a PCR apparatus and stored at 4°C. After the reaction, an aliquot of the solution was electrophoresed, and the amplified DNA fragment was stained with a fluorescent dye Cyber Green (manufactured by FMC BioProducts) and the amplified fragment was quantified by a FluorImager (manufactured by Molecular Dynamics). As the control, G3PDH gene which is a house keeping gene and considered to be expressed at almost the same level both in the kidney of the Thy-1 nephritis rat and in the kidney of the control rat at each stage was subjected to RT-PCR (annealing temperature, 58°C) with each template and primers having nucleotide sequences represented by SEQ ID NOS:6 and 7, and the amplified fragment was quantified. Comparison between the control rat and the Thy-1 nephritis rat at each stage was conducted on the basis of the expression level corrected by dividing the amount of the amplified fragment of each gene by the amount of the amplified fragment of G3PDH gene with the same template.

As a result, it was found that on Day 2 to 13 after administration of the antibody, the amplification level of mRNA hybridizing with the cDNA of TRDH-091 clone was as slightly high (1.1 to 1.4 times) as that of the control group, but the amplification level on Day 16 at the stage of recovering from the morbid state was about 2.5-times as high as that of the control group.

When a change in the expression level of a known protein OP-1 (BMP7) gene was examined in the same manner (annealing temperature, 63°C) by using PCR primers having nucleotide sequences shown in SEQ ID NO:8 and 9, it was found that the expression level was gradually increased after administration of the antibody, to indicate an expression pattern similar to that of the TRDH-091 clone. It was revealed that the development stage-specific protein regarded as effective for treatment of renal insufficiency is also expressed at a higher level even in the process of recovery from nephritis.

The change with time in the expression level of the gene in the kidney of the Thy-1 nephritis rat, relative to the expression level ( = 1) thereof in the kidney in the control rat, is shown in Table 1.

**Table 1.**

| Change with time in the relative expression levels of KRGF-1 and OP-1 in the kidney of Thy-1 rat | | | | | | | | |
|---|---|---|---|---|---|---|---|---|
| Gene | Total | Day 2 | Day 4 | Day 6 | Day 8 | Day 10 | Day 13 | Day 16 |
| TRDH-091 | 1.36 | 1.12 | 1.21 | 1.28 | 1.34 | 1.16 | 1.40 | 2.53 |
| Rat OP-1 | 1.13 | 1.04 | 1.81 | 1.61 | 1.63 | 2.11 | 2.33 | 2.31 |

### (3) Analysis of the sequence

As a result of the nucleotide sequence of the cDNA in TRDH-091 clone, its length was 728 bp. When homology thereof with sequences in data bases was examined, there was no sequence completely identical thereto, indicating that it is a novel cDNA. In the sequences of ESTs, there were human cDNA-derived, two fragments having high homology with TRDH-091 clone, that is, SEQ ID NO:13 (GenBank Accession No. W96209) and SEQ ID NO:14 (GenBank Accession No. W96302).

By searching for an open reading frame and comparison with an estimated length of mRNA obtained as a result of Northern hybridization, the cDNA clone TRDH-091 of the length of 728 bp described above could be considered to have deleted the 5'-or 3'-region from the full-length cDNA. Accordingly, cDNA fragments in the external region of the 5'- and 3'-terminals of the clone cDNA were amplified and isolated by PCR wherein the cDNA library from the kidney of the Thy-1 nephritis rat prepared by using λZAPII as the vector in Example 1 was used as the template, together with a pair of primers shown in SEQ ID NOS:5 and 10 and a pair of primers shown in SEQ ID NOS:4 and 11. Several clones isolated as cDNA 5'fragments had almost the same length as that of the original TRDH-091 clone, but clones longer by at least 700 bp than the TRDH-091 clone were obtained by amplification at the 3'. After-determination of the nucleotide sequences of these cDNA fragments, they were combined with the nucleotide sequence of the original TRDH-091 clone cDNA, whereby the nucleotide sequence of the rat KRGF-1 gene shown in SEQ ID NO:16 was obtained.

When the whole RNA from the kidney of the Thy-1 nephritis rat was subjected to Northern blotting with the cDNA clone of the rat KRGF-1 gene as the probe, the length of mRNA binding to rat KRGF-1 was about 1.6 kb, and in the nucleotide sequence shown in SEQ ID NO:16, there was an open reading frame (ORF) consisting of 395 amino acids shown in SEQ ID NO:15, and the rat KRGF-1 DNA was considered to encode a novel protein having this amino acid sequence. Further, signal P score in analysis for estimation of a secretory signal sequence on the basis of this amino acid sequence was 10.0, suggesting that this protein is a secretory protein. The nucleotide sequence of DNA in a region encoding the rat KRGF-1 protein is shown in SEQ ID NO: 17.

Further, this amino acid sequence was examined for its homologous protein in amino acid sequence data bases SwissProt, PIR, GenPept, TREMBL and GeneSeq by analysis program BLAST, and as a result, it showed 30% identity with an amino acid sequence described as human secretory protein in WO98/33916. Further, it showed 30 to 40% identity with an extracellular region of a family of nucleotide pyrophosphatases such as membrane protein PC-1 [J. Biol. Chem. 265, 17506 (1990)].

### Example 5: Analysis of expression distribution by in situ hybridization

According to a conventional method [Kazuho Nakane: In Situ Hybridization Techniques, revised edition, Gakusai Kikaku (1992)], frozen sections of kidneys from normal rats and Thy-1 nephritis rats were hybridized with antisense RNA labeled with a radioisotope ³⁵S. As a result, the tubulointerstitium in the outer medulla in normal tissues showed reaction in a band, while in the kidney from the Thy-1 nephritis rat, not only the tubulointerstitium but also a part of the Bowman's capsule epithelium showed reaction. This result indicates that the KRGF-1 protein is expressed in cells present in the renal tubular basement membrane, and at the time of repairing damages, the cells migrate to damaged sites and work for recovery therefrom.

### Example 6: Acquisition of human gene by PCR techniques

The sequence (SEQ ID NO:16) of the rat KRGF-1 gene was compared with the sequences (SEQ ID NOS:13 and 14) of the human cDNA fragments described above, and primers (SEQ ID NOS:18 and 19) having high homology therebetween were prepared. The kidney was excised from a patient with a renal cancer, followed by removing tumor tissues to obtain a pathologically normal renal tissue. Then by using the normal renal tissue, a human kidney cDNA library was prepared in the same manner as in Example 1 by using λZAPII as the vector, and PCR performed by using this human kidney cDNA library as the template generates an amplified cDNA fragment of about 0.6 kb matched to the length deduced from the sequence of the rat gene, and the sequence of this cDNA had 85% identity with its corresponding region in the rat gene. Accordingly, for the purpose of obtaining its full-length human cDNA, the 5'- and 3'- fragments of the cDNA were amplified and isolated by performing PCR in the same manner as in (3) in Example 4 except that a human kidney cDNA library using λZAPII as the vector was used as the template, together with a pair of primers shown in SEQ ID NOS: 10 and 19 and a pair of primers shown in SEQ ID NOS: 11 and 18. The nucleotide sequences of these cDNA fragments were determined and combined to give the nucleotide sequence shown in SEQ ID NO:2.

In the nucleotide sequence shown in SEQ ID NO:2, there was an open reading frame (ORF) consisting of 409 amino acids shown in SEQ ID NO:1 and having 84% identity with the amino acid sequence (SEQ ID NO:15) of rat KRGF-1. Accordingly, the amino acid sequence shown in SEQ ID NO:1 was identified as human KRGF-1 protein, and the DNA sequence shown in SEQ ID NO:2 as human KRGF-1 gene. A transformant Escherichia coli DH5 α/phKRGF-1 harboring the cDNA clone phKRGF-1 thus obtained has been deposited under FERM BP-7057 since February 25, 2000 with the National Institute of Bioscience and Human-Technology, Agency of Industrial Science and Technology (1-3, Higashi 1 chome, Tsukuba-shi, Ibaraki-ken, Japan).

### Example 7: Preparation of antiserum (polyclonal antibody) against rat KRGF-1

### (1) Preparation of antigen

A partial peptide of rat KRGF-1 was used as the antigen. To obtain a specific antigen capable of recognizing KRGF-1 having a naturally occurring normal stereostructure and not reacting with PC-1, the amino acids 126 to 142, 298 to 316, and 341 to 359 in the amino acid sequence of rat KRGF-1, which were regions poor in identity with the amino acid sequence of PC-1 and estimated to be highly hydrophilic and likely to occur on the surface of the protein in consideration of its stereostructure were selected as the sequence regions of the partial peptides for antigens. The hydrophilicity thereof was estimated from the amino acid sequence by the method of Kyte and Doolittle [J. Mol. Biol., 157, 105 (1982)]. On the basis of these amino acid sequences, TRDH091-1 (SEQ ID NO:20), TRDH091-2 (SEQ ID NO:21) and TRDH091-3 (SEQ ID NO:22) were synthesized by a peptide synthesizer. The selected two peptides other than TRDH091-1 containing a cysteine residue at the C-terminal thereof were modified by adding, to the N-terminal thereof, a cysteine residue necessary for forming a conjugate with keyhole limpet hemocyanin (abbreviated hereinafter to KLH) for increasing immunogenicity. Taking into consideration that the terminals of regions corresponding to these partial peptides constitute a peptide linkage, the N-terminal of TRDH091-1 was acetylated while the C-terminals of TRDH091-2 and TRDH091-3 were amidated.

10 mg/ml KLH (manufactured by Calbiochem) solution in PBS was prepared, and 25 mg/ml N-(m-maleimide benzoyloxy)succinimide (MBS, manufactured by Nacalai Tesque) in an volume of 1/10 relative to the above solution was added dropwise thereto and reacted for 30 minutes under stirring. The reaction solution was passed through a Sephadex G-25 column (manufactured by Amersham Pharmacia Biotech) previously equilibrated with PBS, to remove free MBS. The resultant KLH-MBS, 2.5 mg, was mixed with 1 mg of each peptide dissolved in 0.1 mol/l sodium phosphate buffer (pH 7.0) and reacted at room temperature for 3 hours under stirring, whereby the peptide was bound to KLH to give a peptide-KLH conjugate. This reaction solution was dialyzed against PBS and used in the following immunization.

### (2) Preparation of antiserum

100 µg of each peptide-KLH conjugate prepared in (1), together with 2 mg of aluminum hydroxide adjuvant [Antibodies - A Laboratory Manual, Cold Spring Harbor Laboratory (1988)] and 1×10⁹ pertussis vaccine cells (manufactured by Chiba Serum Institute), was administered into each of three 5-week-old BALB/c mice. Two weeks after the administration, 100 µg of each peptide-KLH conjugate was administered 4 times at 1 week intervals. Blood was collected from the ocular fundus plexus venosus of the mouse, and the serum titer value was examined by enzyme immunoassay (EIA), and 3 days after final immunization, blood was collected and the spleen was excised from a mouse showing a sufficient antibody titer. From the collected blood, serum was collected and used as antiserum, and the spleen was used for preparation of monoclonal antibody described in Example 8. From all 9 mice subjected to immunization, antisera which were capable of reacting specifically with the peptide used as the antigen in EIA, but were not capable of reacting with the 2 other peptides not used as the antigen were obtained.

EIA was conducted in the following manner. First, each peptide used as the antigen was bound to thyroglobulin (hereinafter referred to as THY) in the same manner as for the peptide-KLH conjugate in (1), to prepare a peptide-TYH conjugate. However, the crosslinking agent used was SMCC [4-(N-maleimide methyl)-cyclohexane-1-carboxylic acid, N-hydroxysuccinimido ester, produced by Sigma Aldrich] in place of MBS. Then, each peptide-THY conjugate prepared at 10 µg/ml was pipetted in a volume of 50 µl/well in a 96-well EIA plate (manufactured by Greiner) and adsorbed thereon by leaving it overnight at 4 °C. After the plate was washed, 1% bovine serum albumin (BSA)-containing PBS (Dulbecco's phosphate buffered saline) (hereinafter referred to as BSA/PBS) was added in a volume of 100 µl/well and left for 1 hour at room temperature, and the remaining active sites were blocked. After the plate was left, BSA/PBS was discarded, and the immunized mouse antiserum was pipetted in a volume of 50 µl/well onto the plate and left for 2 hours, whereby the antiserum was bound to the peptide on the plate. The plate was washed with PBS containing 0.05% polyoxyethylene (20) sorbitan monolaurate (manufactured by Wako Pure Chemical Industries, Ltd.) (hereinafter referred to as Tween-PBS), and peroxidase-labeled rabbit anti-mouse immunoglobulin (manufactured by DAKO) diluted at 200-fold was added in a volume of 50 µl/well and left at room temperature for 1 hour, whereby it was bound to the antiserum. The plate was washed with Tween-PBS and colored by adding ABTS [ammonium 2,2-azinobis(3-ethylbenzothiozole-6-sulfonate)] substrate solution [0.1 mol/l citrate buffer (pH 4.2) containing 1 mol/l ABTS], and the absorbance at 415 nm was measured by a plate reader (Emax, manufactured by Molecular Devices).

### (3) Reactivity of the antiserum with KRGF-1

Each antiserum obtained in (2) was subjected to EIA wherein KRGF-1 obtained in Example 9, denatured KRGF-1 prepared by dissolving KRGF-1 in 0.5% SDS and denaturing it by heating at 100 °C for 5 minutes, and similarly denatured maltose-binding protein (MBP) from Escherichia coli transformed with MBP expression plasmid pMAL-p2X as the negative control were used in place of the peptide-THY conjugate. As a result, every antisera showed specific reactivity with KRGF-1 although the degree of reactivity was varied.

Further, the MBP-KRGF-1 fusion protein-expressing E. coli JM109/pMALpKR obtained in Example 9 was cultured, and the microorganism was subjected to SDS-polyacrylamide gel electrophoresis (SDS-PAGE) and then to Western blotting with these antisera as primary antibody, to examine the reactivity of the antisera with KRGF-1. The SDS-PAGE sample used was obtained by adding a sample buffer [60 mmol/l Tris-HCl, pH 6.8, 2% SDS, 10% glycerol, 5% 2-mercaptoethanol] to the microorganism and then lyzing the microorganism by heating at 100°C for 5 minutes. Western blotting was carried out in the following manner.

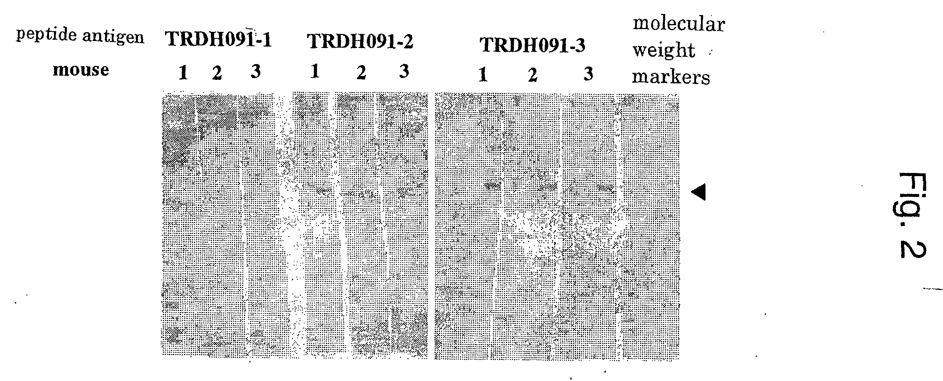

The protein was blotted from the gel after SDS-PAGE onto a polyvinylidene fluoride (PVDF) membrane (manufactured by Millipore), and the membrane was blocked by BSA/PBS, and each antiserum was added thereto as primary antibody and left at room temperature for 2 hours, whereby KRGF-1 on the membrane was reacted with the antiserum. After the membrane was washed well with Tween-PBS, peroxidase-labeled rabbit anti-mouse immunoglobulin antibody (manufactured by DAKO) diluted at a concentration of 1/2000 as second antibody was added thereto and left at room temperature for 1 hour, whereby the second antibody was bound thereto. The membrane was washed well with Tween-PBS and detected by an ECL Western Blotting System (manufactured by Amersham Pharmacia Biotech). The results are shown in Fig. 2. The results of 9 antisera are shown wherein antisera from 3 mice immunized with peptide TRDH091-1 as the antigen, antisera from 3 mice immunized with peptide TRDH091-2 as the antigen and antisera from 3 mice immunized with peptide TRDH091-3 as the antigen were used from the left. In the respective lanes, the left lane shows MBP-expressing Escherichia coli as the control, the right lane shows MBP-KRGF-1 fusion protein-expressing Escherichia coli as the samples, and the arrow indicates the position of a detected band of the MBP-KRGF-1 fusion protein. The rightmost lane shows SDS-PAGE of molecular-weight markers. As shown in Fig. 2, the 6 antiserums obtained by using TRDH091-2 and TRDH091-3 as the immunogen could specifically detect KRGF-1 expressed by JM109/pMALpKR, but none of the 3 antiserums with TRDH091-1 as the immunogen could detect KRGF-1 expressed by JM109/pMALpKR.

### Example 8: Preparation of monoclonal antibody against rat KRGF-1

### (1) Preparation of monoclonal antibody

From the rat obtained in Example 7 whose serum showed adequate antibody titer after immunization with a partial fragment of KRGF-1 as the antigen, the spleen was excised on Day 3 after the final immunization. The spleen is cut into pieces in MEM medium (manufactured by Nissui Pharmaceutical, Co.) and the pieces are then loosened with tweezers and centrifuged at 250×g for 5 minutes. Tris-ammonium chloride buffer (pH 7.6) was added to the resultant precipitated fraction, followed by treatment for 1 to 2 minutes to remove erythrocytes. The resultant precipitated fraction (spleen cells) was washed 3 times with MEM. Separately, 8-azaguanine-resistant mice myeloma cell line P3-X63Ag8-U1 (P3-U1, purchased from ATCC) was cultured in a normal medium (RPMI1640 medium containing 10% BSA) and proliferated until the number of cells was increased to 2×10⁷ or more.

The mouse spleen cells and the myeloma cells prepared as described above were mixed at the ratio of 10 : 1 and centrifuged at 250xg for 5 minutes, and the cells were recovered as the precipitates. The cells were loosed well, and a mixture of 2 g polyethylene glycol 1000, 2 ml MEM and 0.7 ml dimethyl sulfoxide was added in an volume of 0.5 ml/10⁸ mouse spleen cells, and 1 ml MEM was added to the suspension several times at 1- to 2-minute intervals while stirring at 37°C, and MEM was added thereto to adjust the total volume to 50 ml. The suspension was centrifuged at 900 rpm for 5 minutes and the cells were recovered as precipitates. The cells were gently loosened and suspended by pipetting in 100 ml HAT medium [medium prepared by adding HAT media supplement (manufactured by Boehringer Mannheim) to the normal medium]. The suspension was put to each well on a 96-well culture plate in a volume of 200 µl/well and cultured at 37°C in a 5% CO₂ incubator for 10 to 14 days.

After culture, the antibody titer of the culture supernatant in each well was examined by EIA described in Example 7. However, denatured KRGF-1 was used in place of the antigen peptide, and denatured MBP was used as negative control. As a result, a culture supernatant reacting with denatured KRGF-1 but not with denatured MBP was selected, and cloning of the cells contained in the culture by limiting dilution was repeated twice, and finally 5 strains of anti-KRGF-1 monoclonal antibody-producing hybridoma were established.

The hybridoma strain was injected intraperitoneally at a dose of 5 to 20×10⁶ cells/animal of 8-week-old nude male mice (BALB/c) treated with pristane. From the mouse with the ascites tumor after 10 to 21 days, the ascites was collected in a volume of 1 to 8 ml/mouse.

The ascites was centrifuged at 1,200 rpm for 5 minutes, to recover a supernatant. The subclass of the monoclonal antibody contained in the supernatant was determined by using a subclass typing kit, and the ascites was subjected to salting-out with 50% ammonium sulfate, dialyzed against PBS containing 0.5 mol/l sodium chloride and passed at a flow rate of 15 ml/hour through a column of Sellurofine GSL2000 (volume of 750 ml, produced by Seikagaku Kogyo), whereby the monoclonal antibodies whose subclass was IgM were purified and obtained, while the monoclonal antibodies whose subclass was IgG were isolated and purified by a method of caprylic acid precipitation [Antibodies-A Laboratory Manual, Cold Spring Harbor Laboratory (1988)]. In this manner, 5 types of anti-KRGF-1 monoclonal antibodies, KM2954, KM2955, KM2956, KM2957 and KM2958 were obtained.

### (2) Reactivity of the monoclonal antibodies

### (2-1) Western blotting

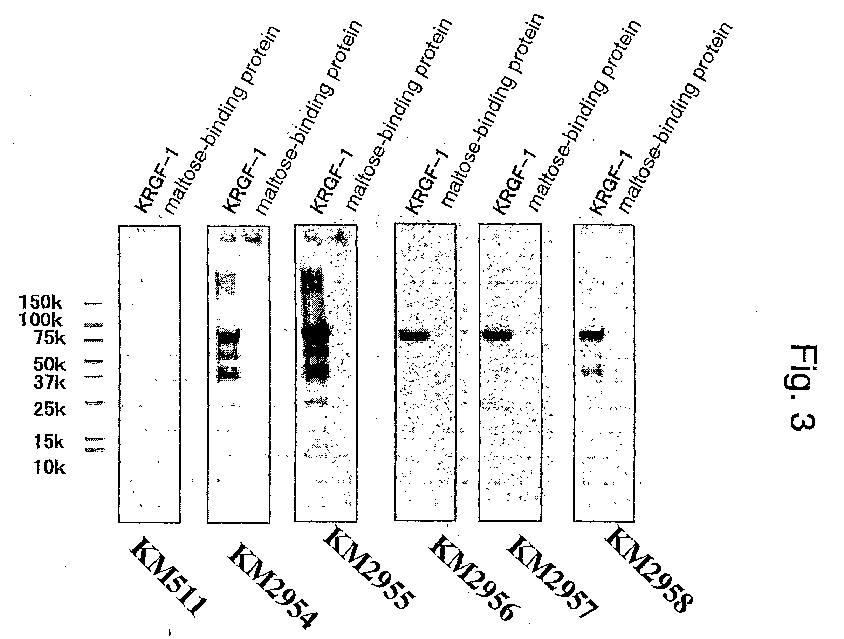

KRGF-1-expressing E. coli JM109/pMALpKR and the negative control E. coli transformed with maltose-binding protein expression plasmid pMAL-p2X, obtained in Example 9, were subjected after SDS-PAGE to Western blotting with the 5 monoclonal antibodies as the primary antibody obtained in (1). The results are shown in Fig. 3. From the left, monoclonal antibody KM511 against a human granulocyte colony stimulating factor (G-CSF) derivative as the negative control, and monoclonal antibodies KM2954, KM2955, KM2956, KM2957 and KM2958 against KRGF-1 were used. In the respective monoclonal antibodies, as the samples, the left lane shows MBP-KRGF-1 fusion protein-expressing E. coli (expressed as KRGF-1 in the figure), and the right lane shows MBP-expressing E. coli (expressed as maltose-binding protein in the figure), and the leftmost numbers indicate the positions of molecular-weight markers (unit, Da). As shown in Fig. 3, all the 5 monoclonal antibodies against KRGF-1 reacted specifically with KRGF-1, but did not react with the maltose-binding protein-expressing E. coli. Accordingly, it was found that these monoclonal antibodies can be used for detection of KRGF-1 by Western blotting.

### (2-2) Immunoprecipitation

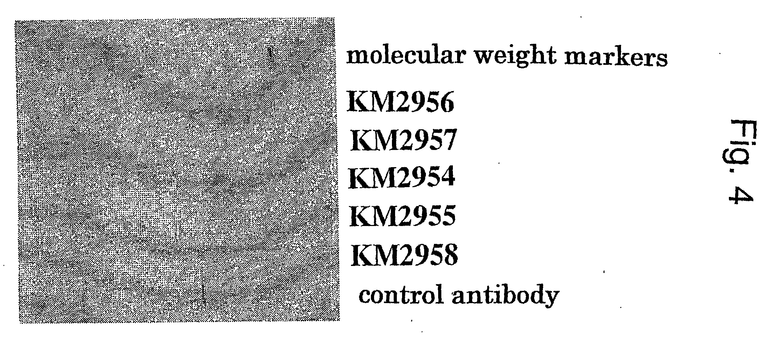

50 µl of a culture supernatant obtained by culturing each of hybridoma strains producing the 5 monoclonal antibodies in the normal medium was mixed with 25 µl of KRGF-1 purified from a culture supernatant of the KRGF-1-expressing CHO cells obtained in Example 10 and reacted for 1 hour on ice, whereby KRGF-1 was bound to the monoclonal antibody. 50 µl of a protein G-Sepharose (manufactured by Amersham Pharmacia Biotech) equilibrated with buffer B (20 mmol/l sodium phosphate, 0.5 mol/l NaCl, pH 7.4) was added thereto and incubated at 4°C for 1 hour, whereby the monoclonal antibody was bound to protein G. Thereafter, the protein G-Sepharose was recovered by centrifugation and washed 3 times with buffer B. 50 µl of a sample buffer [60 mmol/l Tris-HCl, pH 6.8, 2% SDS, 10% glycerol, 5% 2-mercaptoethanol] was added thereto, and the protein G-Sepharose was heated at 100°C, whereby the monoclonal antibody bound to protein G was dissolved in the sample buffer. 20 µl of this solution was subjected to SDS-PAGE and then to Western blotting with KM2955 as the primary antibody. The results are shown in Fig. 4. From the left, SDS-PAGE of molecular-weight markers, samples immunoprecipitated with the anti-KRFG-1 monoclonal antibodies KM2965, KM2957, KM2954, KM2955 and KM2958, and monoclonal antibody KM2957 only as the control are shown. The right line indicates the position of KRGF-1 detected in the immunoprecipitates. As shown in Fig. 4, all the 5 monoclonal antibodies against KRGF-1 indicated smear bands, and KRGF-1 was detected in the immunoprecipitates bound to protein G. Accordingly, it was found that these monoclonal antibodies are bound to KRGF-1 and usable for immunoprecipitates.

### Example 9: Expression of rat KRGF-1 in E. coli

### (1) Construction of an expression plasmid for a fusion protein of rat KRGF-1 with a maltose-binding protein

A DNA fragment(referred to hereinafter as EKR fragment) encoding mature rat KRGF-1 (corresponding to the 113- to 1241-positions in SEQ ID NO:16) excluding N-terminal 22 amino acids estimated to be a signal peptide, and having an EcoRI site at the 5'-terminal thereof and the stop codon TGA and HindIII site at the 3'-terminal thereof, was amplified by PCR wherein, by use of plasmid p091D2X containing rat KRGF-1 cDNA as the template, oligonucleotide primers 5' EKR and 3'EKR whose sequences are shown in SEQ ID NOS:23 and 24 respectively were synthesized. In PCR, Pfu buffer (manufactured by Stratagene) containing 1 ng/ml template DNA, 0.5 µmol/l of each primer, 0.25 mmol/l dNTP, 10% dimethyl sulfoxide and 25 mU/µl Pfu Turbo Polymerase (manufactured by Stratagene) was used as the reaction solution and subjected to 25 cycles of PCR, each consisting of denaturation at 98°C for 1 minute, annealing at 60°C for 1 minute and extension at 72°C for 1 minute. The amplified DNA fragment was digested with restriction enzymes EcoRI and HindIII and purified by GENE CLEAN SPIN Kit (manufactured by Qbiogene).

Vector pBluescript II SK(-) (manufactured by Stratagene) was cleaved with EcoRI and HindIII and then dephosphorylated at the 5'-terminal cleaved site thereof with bacterial alkaline phosphatase, followed by ligation thereof with the purified EKR fragment, to prepare pBS-EKR. The nucleotide sequence of the inserted fragment was determined by a DNA sequencer, and it was confirmed that there was no mutation in the nucleotide sequence of the EKR fragment inserted into pBS-EKR. The MBP-fusion-protein expression vector pMAL-p2X (manufactured by New England BioLabs) was cleaved with EcoRI and HindIII and then dephosphorylated at the 5'-terminal cleaved site thereof with bacterial alkaline phosphatase, followed by ligation thereof with a 1.1 kb DNA fragment obtained by cleaving pBS-EKR with EcoRI and HindIII, whereby plasmid pMALpKR for expression of MBP-KRGF-1 fusion protein in Escherichia coli was constructed.

### (2) Expression of the fusion protein in Escherichia coli

pMALpKR was introduced into Escherichia coli JM109 to give a transformant JM109/pMALpKR. Culture of JM109/pMALpKR and purification of MBP-KRGF-1 fusion protein from the culture were carried out in the following manner according to a manual of New England BioLabs.

JM109/pMALpKR was inoculated into an ampicillin-containing LB medium (1% Bactotrypton, 0.5% yeast extract, 1% NaCl, 50 mg/l ampicillin, pH 7.2) and cultured at 37°C overnight. 10 ml of this culture was inoculated into 1 L of ampicillin-containing LB medium and cultured at 37°C during which the absorbance of the culture at 600 nm (A₆₀₀) was monitored. When the culture reached A₆₀₀ = 0.5, isopropyl thiogalactoside (IPTG) was added at a final concentration of 1 mmol/l to induce the promoter, and culturing was further continued at 37°C.

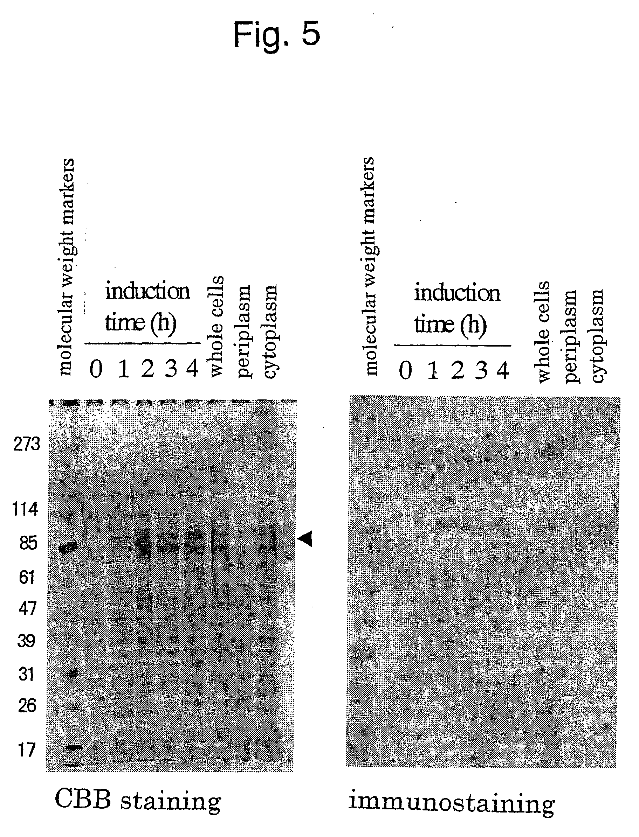

The microorganism before addition of IPTG and the microorganism which was cultured for 1 to 4 hours after addition of IPTG were subjected to SDS-PAGE, and the MBP-KRGF-1 fusion protein in the microorganism was detected by Coomassie Brilliant Blue staining. The results are shown in Fig. 5. The left gel shows the result of Coomassie Brilliant Blue staining after SDS-PAGE (expressed as CBB staining in the figure), and the right gel shows Western blotting with anti-TRDH091-2 antiserum as the primary antibody (expressed as immunostaining in the figure), and the leftmost lane shows molecular markers, and the others lanes are MBP-MRGF-1 fusion-expressing E. coli cultured in the presence of IPTG, wherein the microorganism before addition of IPTG (expressed as induction time (h) 0 in the figure), the microorganism cultured for 1, 2, 3 and 4 hours after addition of IPTG (expressed as induction time (h) 1, 2, 3 and 4), the whole of the microorganism after addition of IPTG (expressed as whole cells in the figure), a periplasm fraction (expressed as periplasm in the figure) and a cytoplasm fraction (expressed as cytoplasm in the figure) are indicated from the left as the samples. The arrow indicates the position of a band of the MBP-KRGF-1 fusion protein.

As shown in Fig. 5, after addition of IPTG a band was detected at the position of the estimated molecular weight (about 90 kDa) of the MBP-KRGF-1 fusion protein, and it was thus confirmed that the MBP-KRGF-1 fusion protein are formed and accumulated after induction of the promoter with IPTG. The band of the MBP-KRGF-1 fusion protein was not detected before addition of IPTG, and the density of the band was increased with time for 3 hours after the addition.

The band at the position of about 90 kDa was also confirmed to be that of the MBP-KRGF-1 fusion protein because the band at the position of about 90 kDa reacted specifically in Western blotting wherein for the transformant JM109/pMALpKR after SDS-PAGE, the antiserum to the rat KRGF-1 partial peptide TRDH091-2 was used as the primary antibody (Fig. 5).

Further, pMALpKR is an expression plasmid containing a signal sequence for MBP, so the microorganism cultured after addition of IPTG was separated by osmotic pressure shock into an intracellular fraction and a periplasm fraction, and each fraction was subjected to SDS-PAGE, to determine whether the MBP-KRGF-1 fusion protein after secretion and expression was present in the cells or in the periplasm. The result indicated that the MBP-KRGF-1 fusion protein was present in the intracellular fraction as shown in Fig. 5.

### (3) Purification of MBP-KRGF-1 fusion protein by an affinity column

1 L of a culture obtained by culturing the JM109/pMALpKR at 37°C for 3 hours after addition of IPTG was centrifuged to recovery the microorganism which was then stored at -20°C.

The microorganisms were thawed, and 50 ml of buffer A (20 mmol/l Tris-HCl, pH 7.4, 0.2 mol/l NaCl, 1 mmol/l ethylene diamine tetraacetate) was added thereto, and 80% of the microorganisms were disrupted by sonication. The microbial lysate was centrifuged at 9,000×g for 30 minutes, and the supernatant was separated and recovered. The supernatant was diluted to a concentration of 1/5 with buffer A, then passed through a 0.45 µm filter and applied onto an affinity column.

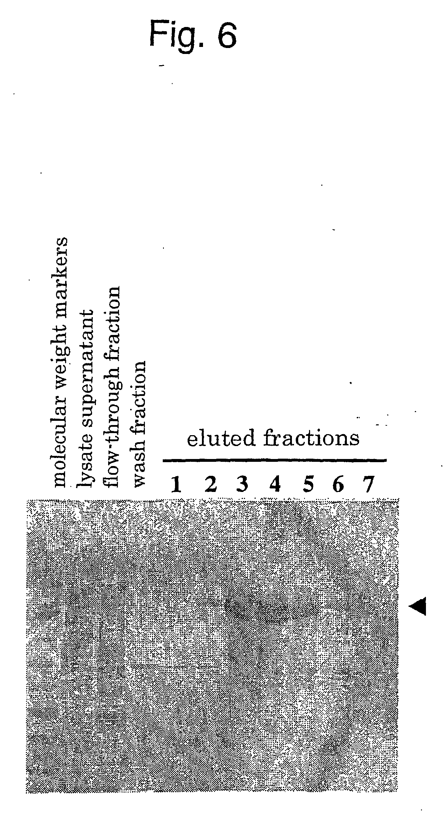

An amylose resin (volume of 4 ml, New England BioLabs) was introduced into a column and equilibrated with 32 ml of buffer A to prepare an affinity column. 250 ml of the microbial lysate supernatant was applied to this column, whereby the MBP-KRGF-1 fusion protein was adsorbed onto the column. The column was washed by passing 80 ml of buffer A therethrough, and 7 ml of buffer A containing 10 mmol/l maltose was passed through the column to elute the MBP-KRGF-1 fusion protein. The eluate was fractionated in a volume of 1 ml/fraction, and aliquots from the respective fractions, non-adsorbed fractions obtained by passing the supernatant, and fractions eluted during washing were subjected to SDS-PAGE, to detect the MBP-KRGF-1 fusion protein in each fraction. The results are shown in Fig. 6. The results of SDS-PAGE (Coomassie Brilliant Blue staining) of the following samples are shown from the left: molecular-weight markers, the microbial lysate supernatant of the MBP-KRGF-1 fusion protein-expressing Escherichia coli before passing through the column (expressed as lysate supernatant in the figure), a fraction passed through the column (expressed as flow-through fraction), a fraction during washing with buffer A (expressed as wash fraction) and eluted fractions 1 to 7 (expressed as eluted fractions 1 to 7 in the figure). The arrow shows the position of a band of the MBP-KRGF-1 fusion protein. As shown in Fig. 6, the MBP-KRGF-1 fusion protein of about 90 kDa was detected in the respective fractions (eluted fractions 2 to 7) excluding 1 ml of the first eluate. Accordingly, the fractions, 6 ml in total, excluding 1 ml of the first eluate were combined and stored at -20 °C as purified MBP-KRGF-1 fusion protein. The purity of the purified MBP-KRGF-1 fusion protein was about 70% in SDS-PAGE.

### Example 10: Expression of rat KRGF-1 in CHO cells

### (1) Construction of myc-His-tagged rat KRGF-1 expression plasmid

A DNA fragment (referred to hereinafter as CKR fragment) encoding a signal peptide-containing rat KRGF-1 (corresponding to SEQ ID NO:17), and having the Kozac sequence and an EcoRI site at the 5'-terminal thereof and a HindIII site at the 3'-terminal thereof, was amplified by carrying out PCR in the same manner as in (1) in Example 8 by using p091D2X as the template, and synthesizing oligonucleotide primers 5' CKR and 3' CKR whose sequences are shown in SEQ ID NOS:25 and 26 respectively. The CKR fragment was digested with EcoRI and HindIII and purified with GENE CLEAN SPIN Kit (manufactured by Qbiogene).

As the cloning vector for adding a myc-His tag sequence to the 3'-side of the CKR fragment, pBSmycHis was prepared in the following manner. pcDNA3.1/Myc-His(-)C (manufactured by Invitrogen) was digested with PmeI and separated by agarose gelelectrophoresis, and a DNA fragment (referred to hereinafter as insert DNA fragment) containing an about 170-bp myc-His tag sequence was excised and extracted. Separately, pBluescript II SK(+) was digested with XbaI and KpnI and then blunt-ended with T4 polymerase (manufactured by Takara Shuzo), and an about 2.9-kpb DNA fragment (referred to hereinafter as vector DNA fragment) in agarose gel electrophoresis was excised and extracted. 100 ng of the vector DNA fragment and 3.2 µg of the insert DNA fragment, in a volume of 10 µl, were subjected to ligation reaction by DNA Ligation Kit Ver. 2 (manufactured by Takara Shuzo Co., Ltd.). The reaction solution was used to transform Escherichia coli DH5 α strain (Library Efficiency DH5 α Competent Cell, manufactured by Life Technologies) to give transformants. A plasmid was isolated from several transformant strains by a known method (Molecular Cloning, 2nd edition). If the insert DNA fragment is inserted into the vector DNA fragment, two XbaI sites are produced, and by cleavage with XbaI, an about 2.9-kbp fragment and an about 160-bp fragment are generated, so a plasmid generating an about 2.9-kbp fragment and an about 160-bp fragment, upon cleavage with XbaI, was selected as a plasmid having the desired structure and named pBSmycHis.

The pBSmycHis was cleaved with EcoRI and HindIII and then dephosphorylated at the 5'-terminal cleaved site thereof with bacterial alkaline phosphatase, followed by ligation thereof with the purified CKR fragment, to prepare pBS-CKRmH. The nucleotide sequence of the inserted fragment was determined by a DNA sequencer, and it was confirmed that there was no mutation in the nucleotide sequence of the EKR fragment inserted into pBSmycHis.

The animal cell expression vector pcDNA3.1 (+) (manufactured by Stratagene) was cleaved with EcoRI and XbaI and then dephosphorylated at the 5'-terminal cleaved site thereof with bacterial alkaline phosphatase, followed by ligation thereof with a 1.3 kb DNA fragment obtained by cleaving pBS-CKRmH with EcoRI and XbaI, whereby plasmid pcKKRmH for expression of rat KRGF-1 having the myc-His tag added to the C-terminal thereof in animal cells was constructed.

### (2) Expression in CHO cells and selection of high-expression strain

CHO cells were cultured and proliferated in a medium (CHO medium) prepared by adding 5% bovine fetal albumin (Catalog No. 10099-141, inactivated for 1 hour, manufactured by Life Technologies) and a 1/100 volume of a penicillin-streptomycin solution (Catalog No. 15140-122, manufactured by Life Technologies) to the minimum essential medium (MEM, Catalog No. 11900-024, manufactured by Life Technologies).

The plasmid pcKKRmH was linearized by cleavage with SacI and adjusted at a concentration of 1 mg/ml, and 4 µl of the resulting matter was mixed with 1.6×10⁷ CHO cells suspended in 200 µl of K-PBS, then transferred to an electroporation cuvette having an electrode distance of 0.2 mm, and pulsated at 0.35 kV, 250 µF. The time constant was 3.8 millisecond. A half of the cell suspension after pulsing was suspended in 10 ml of CHO cell medium and pipetted onto a 96-well culture plate (manufactured by Greiner) in a volume of 100 µl/well and subjected to stationary culture at 37°C in a CO₂ incubator (5% CO₂).

On the next day, the cells were cultured in the presence of G418 at a concentration of 0.3 mg/ml, and a colony of cells appearing 7 days later was selected as G418 resistance clone. After the medium was exchanged with EX-CELL301 (manufactured by JRH Biosciences) containing 0.3 mg/ml G418, culture of each clone was continued to adapt the clone to serum-free suspension culture.

KRGF-1 was predicted to be expressed by secretion because a sequence estimated to be a signal peptide is present in the N-terminal of KRGF-1, but because the culture scale was small, a cell extract of each clone was prepared and subjected to dot blotting assay with KM2954 as the primary antibody, whereby a high-expression clone of KRGF-1 was selected. Serum-free suspension culture of the selected high-expression clone was continued, and finally culture was carried out in a roller bottle.

### (3) Intracellular location of KRGF-1 expressed in CHO cells

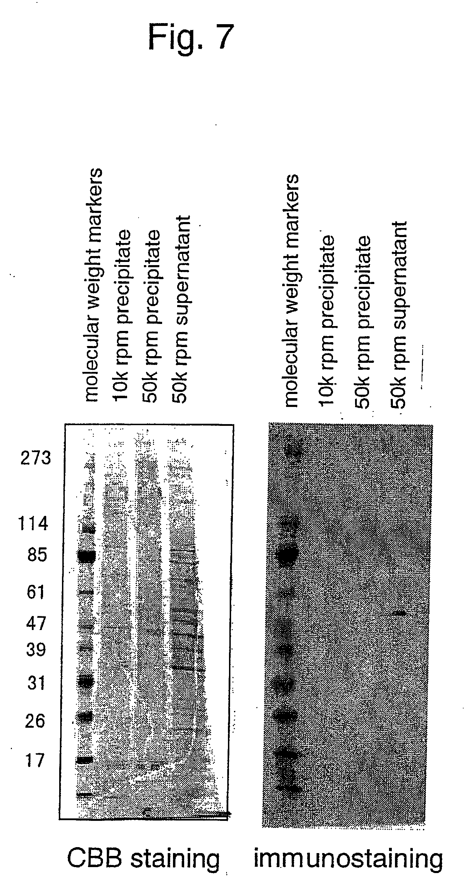

Whether KRGF-1 present in CHO cells was located in the cytosol in the cells or in the cell membrane was examined in the following manner. Cells of the high-expression clone cultured in (2) were recovered and disrupted by a homogenizer to prepare a cell extract. The cell extract was first centrifuged at 10,000 rpm, and a supernatant containing the cell membrane and cytosol was isolated from the precipitate. The supernatant was further centrifuged at 50,000 rpm, whereby it was separated into a supernatant fraction containing the cytosol and a precipitated fraction containing the cell membrane. Each fraction was subjected to SDS-PAGE, and KRGF-1 was detected by Western blotting with KM2954 as the primary antibody. The results are shown in Fig. 7. The left gel shows the result of Coomassie Brilliant Blue staining in SDS-PAGE, and the right gel shows the result of Western blotting with KM2954 as the primary antibody, wherein the following samples were used from the left: molecular markers, a precipitated fraction (precipitated at 10k rpm in the figure) after centrifugation of the cell extract (10,000 rpm), a precipitated fraction (cell membrane fraction, precipitated at 50 k rpm in the figure) after centrifugation of the supernatant (50,000 rpm), and a supernatant fraction (cytosol fraction, a supernatant at 50 k rpm in the figure) after centrifugation of the supernatant (50,000 rpm). As shown in Fig. 7, it was found that in the cells, KRGF-1 occurs not in the cell membrane but in the cytosol.

### (4) Purification of KRGF-1 expressed in CHO cells.

By using the metal-chelating ability of the His tag (polyhistidine) added to the C-terminal of KRGF-1 expressed in CHO in (2), purification thereof was conducted in the following manner.

100 ml suspension culture of the clone highly expressing KRGF-1 selected in (2) was separated by centrifugation into CHO cells and a culture supernatant, and the CHO cells were suspended in 5 ml buffer B (20 mmol/l sodium phosphate, 0.5 mol/l NaCl pH 7.4) and disrupted by sonication. The resultant cell-disrupted solution was centrifuged to isolate a supernatant which was then passed through a filter with pore size of 0.22 µm, and the filtrate was purified by His Trap Kit (manufactured by Amersham Pharmacia Biotech) as described later. On the other hand, the culture supernatant, 30 ml, was concentrated into 1.5 ml by ultrafiltration and diluted to a concentration of 1/2 by adding an equal volume of buffer B and then passed through a filter with pore size of 0.22 µm, and the filtrate was subjected to purification by His Trap Kit as described below.

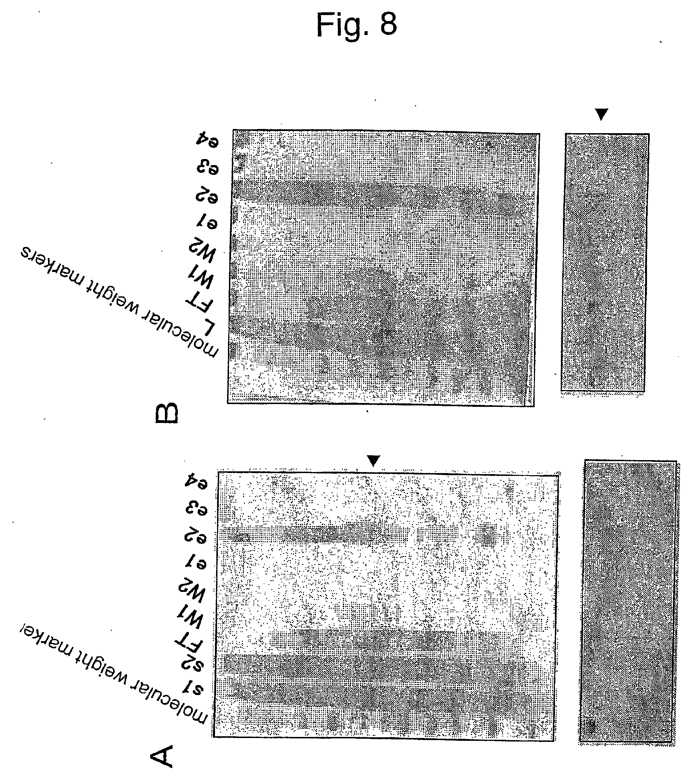

The purification followed a manual attached to the kit, and the supernatant of the cell-disrupted solution or the supernatant of the culture was passed through a HiTrap Chelating HP (with a volume of 1 ml, manufactured by Amersham Pharmacia Biotech) column, the myc-His-tagged rat KRGF-1 was bound to the column, washed with 20 ml buffer B and eluted with 5 ml buffer B containing 0.5 mol/l imidazole, and the eluate was collected in a volume of 1 ml per fraction. Each fraction was subjected to SDS-PAGE, and KRFG-1 was detected by Western blotting with Coomassie Brilliant Blue staining and anti-KRGF-1 antiserum as the primary antibody. The results are shown in Fig. 8. Gel A shows KRGF-1 purified from the CHO cells, while gel B shows KRGF-1 purified from the culture supernatant, wherein the leftmost lane indicates molecular-weight markers; s1, 2 in the figure indicate a supernatant from the cell-disrupted solution before application to the column; L in the figure, a culture supernatant; FT in the figure, a fraction not retained on the column; W1, 2 in the figure, a fraction eluted during washing with buffer B; and el to 4 in the figure, eluted fractions 1 to 4; in the upper gel, the samples were subjected to staining with Coomassie Brilliant Blue for detection of KRGF-1 by SDS-PAGE, while in the lower gel, the samples were subjected to Western blotting for detection of KRGF-1. As shown in Fig. 8, KRGF-1 in both the CHO cell-disrupted solution and the culture supernatant was eluted into fraction 2, and this fraction was regarded as purified KRGF-1 fraction. In the purified fraction from the CHO cell disrupted solution, KRGF-1 could be detected as a band not only by Western blotting but also by Coomassie Brilliant Blue staining. KRGF-1 in the culture supernatant was detected as a smear band indicating a higher molecular weight than that of KRGF-1 in CHO cells, thus suggesting addition of sugar chains thereto.

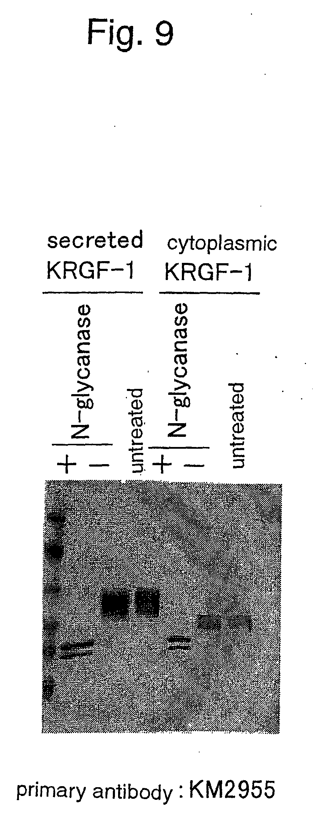

For confirmation of addition of sugar chains, SDS and 2-mercaptoethanol were added at final concentrations of 0.5% and 50 mmol/l respectively to the purified KRGF-1 fraction prepared from the CHO cells and the culture supernatant respectively, and heated at 100°C for 5 minutes, followed by adding Nonidet P-40 (manufactured by Nacalai Tesque) thereto at a final concentration of 1.5%. N-glicanase (manufactured by Genzyme) was added thereto in an amount of 0.01 unit/µl and reacted at 37°C for 15 hours, whereby N-type sugar chains were digested. To the control was added the same volume of sterilized water in place of N-glycanase, and the same reaction was carried out. KRGF-1 after the reaction, together with KRGF-1 before the reaction, was subjected to SDS-PAGE and Western blotting with KM2955 as the primary antibody, whereby a change in the molecular weight of KRGF-1 was detected. The results are shown in Fig. 9. From the left, KRGF-1 in the following samples were detected by Western blotting: molecular markers; the secreted KRGF-1 purified from the culture supernatant (expressed as secreted KRGF-1 in the figure), that is, the secreted KRGF-1 treated with N-glycanase (expressed as + in the figure), the secreted KRGF-1 subjected to reaction such as incubation in the absence of N-glycanase (expressed as - in the figure) and the untreated secreted KRGF-1 (expressed as untreated in the figure); and the cytoplasmic KRGF-1 purified from the CHO cells, that is, the cytoplasmic KRGF-1 treated with N-glycanase (expressed as + in the figure), the cytoplasmic KRGF-1 subjected to reaction such as incubation in the absence of N-glycanase (expressed as - in the figure) and the untreated cytoplasmic KRGF-1 (expressed as untreated in the figure). Both the secreted KRGF-1 from the culture supernatant and the cytoplasmic KRGF-1 from the CHO cells had a similar molecular weight decreased by treatment with N-glycanase. It was thus found that KRGF-1 undergoes addition of sugar chains at multiple stages in the process from synthesis to secretion of the protein, in particular in the step of secretion of the protein from the cells.

### Example 11: Measurement of the phosphodiesterase activity of KRGF-1

PC-1 having about 30% homology with KRGF-1 was reported to have a phosphodiesterase activity [Proc Natl Acad Sci USA, 88, 5192 (1991)], so the phosphodiesterase activity of KRGF-1 expressed in Escherichia coli and CHO cells was measured in a partially modified method of the Lee et al.'s method [J. Biol. Chem., 271, 24408 (1996)]. That is, 80 µl substrate solution [50 mmol/l Tris-HCl, pH 8.0, containing 5 mmol/l pNP-TMP (manufactured by Sigma Aldrich)] was mixed with 20 µl of the sample (solution containing the purified KRGF-1 prepared from KRGF-1-expressing Escherichia coli or KRGF-1-expressing CHO cells described in Examples 7 and 8) on a 96-well ELISA plate (manufactured by Iwaki Glass Co., Ltd.) and incubated at 37°C for 90 minutes, and the absorbance at 415 nm was measured by a Bench Mark Microplate Reader (manufactured by Bio-Rad Laboratories). However, there was no difference in phosphodiesterase activity between the sample and the buffer only as the negative control, and the phosphodiesterase activity of KRGF-1 could not be detected.

### Industrial Applicability

According to this invention, there can be provided a protein useful for screening and development of a therapeutic agent actively repairing damaged tissues in renal diseases, a DNA encoding the protein, an antibody recognizing the protein, and a method of using the same.

SEQUENCE LISTING FREE TEXT
Description of Artificial Sequence SEQ ID NO:4: Synthetic DNA
Description of Artificial Sequence SEQ ID NO:5: Synthetic DNA
Description of Artificial Sequence SEQ ID NO:6: Synthetic DNA
Description of Artificial Sequence SEQ ID NO:7: Synthetic DNA
Description of Artificial Sequence SEQ ID NO:8: Synthetic DNA
Description of Artificial Sequence SEQ ID NO:9: Synthetic DNA
Description of Artificial Sequence SEQ ID NO:10: Synthetic
DNA
Description of Artificial Sequence SEQ ID NO:11: Synthetic
DNA
Description of Artificial Sequence SEQ ID NO:12: Synthetic
DNA
Description of Artificial Sequence SEQ ID NO:18: Synthetic
DNA
Description of Artificial Sequence SEQ ID NO:19: Synthetic

DNA
Description of Artificial Sequence SEQ ID NO:20: Synthetic
peptide
Description of Artificial Sequence SEQ ID NO:21: Synthetic
peptide
Description of Artificial Sequence SEQ ID NO:22: Synthetic
peptide
Description of Artificial Sequence SEQ ID NO:23: Synthetic
DNA
Description of Artificial Sequence SEQ ID NO:24: Synthetic
DNA
Description of Artificial Sequence SEQ ID NO:25: Synthetic
DNA
Description of Artificial Sequence SEQ ID NO:26: Synthetic
DNA

## Claims

1. A protein comprising an amino acid sequence represented by SEQ ID NO:1 or 15.

2. A protein comprising an amino acid sequence wherein one or more amino acids are deleted, substituted or added in the amino acid sequence represented by SEQ ID NO: 1 or 15, and having a kidney-regenerating activity.

3. A protein comprising an amino acid sequence having at least 60% identity with the amino acid sequence represented by SEQ ID NO: 1 or 15, and having a kidney-regenerating activity.

4. A glycoprotein comprising sugar chains added to the protein according to any one of claims 1 to 3.

5. A DNA encoding the protein according to any one of claims 1 to 4.

6. A DNA comprising a nucleotide sequence represented by SEQ ID NO:2, 3, 16 or 17.

7. A DNA which hybridizes with the DNA according to claim 5 or 6 under stringent conditions and is expressed at a higher level in tissues where inflammatory diseases occur.

8. A DNA having the same nucleotide sequence as that of consecutive 10 to 60 nucleotides in the DNA according to any one of claims 5 to 7.

9. A DNA having a nucleotide sequence complementary to the DNA according to any one of claims 5 to 7.

10. A method of detecting and quantifying the expression of a gene encoding the protein according to any one of claims 1 to 4, which comprises using the DNA according to any one of claims 5 to 9.

11. A method of detecting a mutation in a gene encoding the protein according to any one of claims 1 to 4, which comprises using the DNA according to any of claims 5 to 9.

12. Amethod of obtaining a promoter region of a gene encoding the protein according to any one of claims 1 to 4, which comprises using the DNA according to any one of claims 5 to 9.

13. A diagnostic agent for a renal disease, which comprises the DNA according to any one of claims 5 to 9.

14. A method of detecting a gene involved in progress of a renal disease or recovery therefrom, which comprises using the DNA according to any one of claims 5 to 9.

15. A method of screening a substance inhibiting or promoting the transcription or translation of a gene encoding the protein according to any one of claims 1 to 4, which comprises using the DNA according to any one of claims 5 to 9.

16. A method of screening a therapeutic agent for a renal disease, which comprises using the DNA according to any one of claims 5 to 9.

17. A recombinant vector which is obtained by inserting the DNA according to any one of claims 5 to 9 into a vector.

18. A recombinant vector which is obtained by inserting an RNA having a sequence homologous to the DNA according to any one of claims 5 to 9 into a vector.

19. The recombinant vector according to claim 18, wherein the RNA is a double-stranded sense or antisense chain.

20. A transformant obtained by introducing the recombinant vector according to claim 17 into a host cell.

21. A process for producing a protein, which comprises culturing the transformant according to claim 20 in a medium to produce and accumulate the protein according to any one of claims 1 to 4 in the culture, and recovering the protein therefrom.

22. A method of screening a therapeutic agent for a renal disease, which comprises using a culture obtained by culturing the transformant according to claim 20 in a medium, said culture comprising the protein according to any one of claims 1 to 4.

23. A method of screening a therapeutic agent for a renal disease, which comprises using the protein according to any one of claims 1 to 4.

24. A therapeutic agent for a renal disease, which comprises the protein according to any one of claims 1 to 4.

25. A therapeutic agent for a renal disease, which comprises the recombinant vector according to any one of claims 17 to 19.

26. An antibody which recognizes the protein according to any one of claims 1 to 4.

27. A monoclonal antibody which recognizes the protein according to any one of claims 1 to 4.

28. The monoclonal antibody according to claim 27, wherein the monoclonal antibody is KM2954, KM2955, KM2956, KM2957 or KM2958.

29. A hybridoma which produces the monoclonal antibody according to claim 27 or 28.

30. The hybridoma according to claim 29, wherein the hybridoma is a hybridoma KM2955 (FERM BP-xxxx).

31. A method of inhibiting the biological activity of the protein according to any one of claims 1 to 4, which comprises using the antibody according to any one of claims 26 to 28.

32. A method of immunologically detecting and quantifying the protein according to any one of claims 1 to 4, which comprises using the antibody according to any one of claims 26 to 28.

33. A method of isolating and purifying the protein according to any one of claims 1 to 4, which comprises using the antibody according to any one of claims 26 to 28.

34. A method of obtaining cells being capable of expressing the protein of any one of claims 1 to 4, which comprises using the antibody according to any one of claims 26 to 28.

35. A method of screening a therapeutic agent for a renal disease, which comprises using the antibody according to any one of claims 26 to 28.

36. A method of screening a substance being capable of inhibiting or promoting the transcription or translation of a gene encoding the protein according to any one of claims 1 to 4, which comprises using the antibody according to claims 26 to 28.

37. A diagnostic agent for a renal disease, which comprises the antibody according to any one of claims 26 to 28.

38. A therapeutic agent for a renal disease, which comprises the antibody according to any one of claims 26 to 28.

39. A drug delivery method for delivering an agent to damaged lesions in the kidney, which comprises using a fusion antibody comprising the antibody according to any one of claims 26 to 28 bound to the agent selected from a radioisotope, a protein and a low-molecular weight compound.

40. A method of screening a therapeutic agent for a renal disease, which comprises using cells obtained by the method according to claim 34.

41. A method of treating a renal disease, which comprises using cells obtained by the method according to claim 34.

42. A polypeptide comprising an amino acid sequence selected from the amino acid sequences represented by SEQ ID NOS: 1 and 15.

43. A therapeutic agent for a renal disease, which comprises the polypeptide according to claim 42.

44. A method of screening a receptor binding specifically to the protein according to any one of claims 1 to 4, which comprises using the protein according to any one of claims 1 to 4.

45. A knockout non-human animal wherein the expression of a gene encoding the protein according to any one of claims 1 to 4 is partially or completely suppressed.

46. A knockout non-human animal wherein the activity of producing the protein according to any one of claims 1 to 4 is partially or completely suppressed.

47. A recombinant non-human animal in which a gene encoding the protein according to any one of claims 1 to 4 has been introduced.


SEQUENCE LISTING
<110> KYOWA HAKKO KOGYO CO., LTD
<120> novel protein
<130> H11-1951J5
<140>
<141>
<150> JP 2000-73632
<151> 2000-03-16
<160> 19
<170>PatentIn Ver. 2.0

<210> 1
<211> 409
<212> PRT
<213> Homo sapiens
<400> 1
Met Ala Val Lys Leu Gly Thr Leu Leu Leu Ala Leu Ala Leu Gly Leu
1               5                   10                  15
Ala Gln Pro Ala Ser Ala Arg Arg Lys Leu Leu Val Phe Leu Leu Asp
            20                  25                  30
Gly Phe Arg Ser Asp Tyr Ile Ser Asp Glu Ala Leu Glu Ser Leu Pro
        35                  40                  45
Gly Phe Lys Glu Ile Val Ser Arg Gly Val Lys Val Asp Tyr Leu Thr
    50                  55                  60
Pro Asp Phe Pro Ser Leu Ser Tyr Pro Asn Tyr Tyr Thr Leu Met Thr
65                  70                  75                  80
Gly Arg His Cys Glu Val His Gln Met Ile Gly Asn Tyr Met Trp Asp
                85                  90                  95
Pro Thr Thr Asn Lys Ser Phe Asp Ile Gly Val Asn Lys Asp Ser Leu
            100                 105                 110
Met Pro Leu Trp Trp Asn Gly Ser Glu Pro Leu Trp Val Thr Leu Thr
        115                 120                 125

Lys Ala Lys Arg Lys Val Tyr Met Tyr Tyr Trp Pro Gly Cys Glu Val
    130                 135                 140
Glu Ile Leu Gly Val Arg Pro Thr Tyr Cys Leu Glu Tyr Lys Asn Val
145                 150                 155                 160
Pro Thr Asp Ile Asn Phe Ala Asn Ala Val Ser Asp Ala Leu Asp Ser
                165                 170                 175
Phe Lys Ser Gly Arg Ala Asp Leu Ala Ala Ile Tyr His Glu Arg Ile
            180                 185                 190
Asp Val Glu Gly His His Tyr Gly Pro Ala Ser Pro Gln Arg Lys Asp
        195                 200                 205
Ala Leu Lys Ala Val Asp Thr Val Leu Lys Tyr Met Thr Lys Trp Ile
    210                 215                 220
Gln Glu Arg Gly Leu Gln Asp Arg Leu Asn Val Ile Ile Phe Ser Asp
225                 230                 235                 240
His Gly Met Thr Asp Ile Phe Trp Met Asp Lys Val Ile Glu Leu Asn
                245                 250                 255
Lys Tyr Ile Ser Leu Asn Asp Leu Gln Gln Val Lys Asp Arg Gly Pro
            260                 265                 270
Val Val Ser Leu Trp Pro Ala Pro Gly Lys His Ser Glu Ile Tyr Asn
        275                 280                 285
Lys Leu Ser Thr Val Glu His Met Thr Val Tyr Glu Lys Glu Ala Ile
    290                 295                 300
Pro Ser Arg Phe Tyr Tyr Lys Lys Gly Lys Phe Val Ser Pro Leu Thr
305                 310                 315                 320
Leu Val Ala Asp Glu Gly Trp Phe Ile Thr Glu Asn Arg Glu Met Leu
                325                 330                 335
Pro Phe Trp Met Asn Ser Thr Gly Arg Arg Glu Gly Trp Gln Arg Gly
            340                 345                 350
Trp His Gly Tyr Asp Asn Glu Leu Met Asp Met Arg Gly Ile Phe Leu
        355                 360                 365
Ala Ser Asp Leu Ile Ser Asn Pro Thr Ser Glu Leu Leu Leu Ser Gly
    370                 375                 380

Arg Trp Thr Ser Thr Met Ser Cys Ala Met Trp Trp Ala Ser Pro Arg
385                 390                 395                 400
Cys Pro Thr Thr Asp Pro Gly Pro Gly
                405

<210> 2
<211> 1550
<212> DNA
<213> Homo sapiens
<220>
<221> CDS
<222> (37)..(1263)
<400> 2
gctgtgccag ccgggctctg gcaggctcct ggcagc atg gca gtg aag ctt ggg     54
                                        Met Ala Val Lys Leu Gly
                                        1

                        5
acc ctc ctg ctg gcc ctt gcc ctg ggc ctg gcc cag cca gcc tct gcc     102
Thr Leu Leu Leu Ala Leu Ala Leu Gly Leu Ala Gln Pro Ala Ser Ala
            10                  15                  20
cgc cgg aag ctg ctg gtg ttt ctg ctg gat ggt ttt cgc tca gac tac     150
Arg Arg Lys Leu Leu Val Phe Leu Leu Asp Gly Phe Arg Ser Asp Tyr
        25                  30                  35
atc agt gat gag gcg ctg gag tca ttg cct ggt ttc aaa gag att gtg     198
Ile Ser Asp Glu Ala Leu Glu Ser Leu Pro Gly Phe Lys Glu Ile Val
    40                  45                  50
agc agg gga gta aaa gtg gat tac ttg act cca gac ttc cct agt ctc     246
Ser Arg Gly Val Lys Val Asp Tyr Leu Thr Pro Asp Phe Pro Ser Leu
55                  60                  65                  70
tcg tat ccc aat tat tat acc cta atg act ggc cgc cat tgt gaa gtc     294
Ser Tyr Pro Asn Tyr Tyr Thr Leu Met Thr Gly Arg His Cys Glu Val
                75                  80                  85

cat cag atg atc ggg aac tac atg tgg gac ccc acc acc aac aag tcc 342
His Gln Met Ile Gly Asn Tyr Met Trp Asp Pro Thr Thr Asn Lys Ser
            90                  95                  100
ttt gac att ggc gtc aac aaa gac agc cta atg cct ctc tgg tgg aat 390
Phe Asp Ile Gly Val Asn Lys Asp Ser Leu Met Pro Leu Trp Trp Asn
        105                 110                 115
gga tca gaa cct ctg tgg gtc act ctg acc aag gcc aaa agg aag gtc 438
Gly Ser Glu Pro Leu Trp Val Thr Leu Thr Lys Ala Lys Arg Lys Val
    120                 125                 130
tac atg tac tac tgg cca ggc tgt gag gtt gag att ctg ggt gtc aga 486
Tyr Met Tyr Tyr Trp Pro Gly Cys Glu Val Glu Ile Leu Gly Val Arg
135                 140                 145                 150
ccc acc tac tgc cta gaa tat aaa aat gtc cca acg gat atc aat ttt 534
Pro Thr Tyr Cys Leu Glu Tyr Lys Asn Val Pro Thr Asp Ile Asn Phe
                155                 160                 165
gcc aat gca gtc agc gat gct ctt gac tcc ttc aag agt ggc cgg gcc 582
Ala Asn Ala Val Ser Asp Ala Leu Asp Ser Phe Lys Ser Gly Arg Ala
            170                 175                 180
gac ctg gca gcc ata tac cat gag cgc att gac gtg gaa ggc cac cac 630
Asp Leu Ala Ala Ile Tyr His Glu Arg Ile Asp Val Glu Gly His His
        185                 190                 195
tac ggg cct gca tct ccg cag agg aaa gat gcc ctc aag gct gta gac 678
Tyr Gly Pro Ala Ser Pro Gln Arg Lys Asp Ala Leu Lys Ala Val Asp
    200                 205                 210
act gtc ctg aag tac atg acc aag tgg atc cag gag cgg ggc ctg cag 726
Thr Val Leu Lys Tyr Met Thr Lys Trp Ile Gln Glu Arg Gly Leu Gln
215                 220                 225                 230
gac cgc ctg aac gtc att att ttc tcg gat cac gga atg acc gac att 774
Asp Arg Leu Asn Val Ile Ile Phe Ser Asp His Gly Met Thr Asp Ile
                235                 240                 245
ttc tgg atg gac aaa gtg att gag ctg aat aag tac atc agc ctg aat 822
Phe Trp Met Asp Lys Val Ile Glu Leu Asn Lys Tyr Ile Ser Leu Asn

            250                 255                 260
gac ctg cag caa gtg aag gac cgc ggg cct gtt gtg agc ctt tgg ccg 870
Asp Leu Gln Gln Val Lys Asp Arg Gly Pro Val Val Ser Leu Trp Pro
        265                 270                 275
gcc cct ggg aaa cac tct gag ata tat aac aaa ctg agc aca gtg gaa 918
Ala Pro Gly Lys His Ser Glu Ile Tyr Asn Lys Leu Ser Thr Val Glu
    280                 285                 290
cac atg act gtc tac gag aaa gaa gcc atc cca agc agg ttc tat tac 966
His Met Thr Val Tyr Glu Lys Glu Ala Ile Pro Ser Arg Phe Tyr Tyr
295                 300                 305                 310
aag aaa gga aag ttt gtc tct cct ttg act tta gtg gct gat gaa ggc 1014
Lys Lys Gly Lys Phe Val Ser Pro Leu Thr Leu Val Ala Asp Glu Gly
                315                 320                 325
tgg ttc ata act gag aat cga gag atg ctt ccg ttt tgg atg aac agc 1062
Trp Phe Ile Thr Glu Asn Arg Glu Met Leu Pro Phe Trp Met Asn Ser
            330                 335                 340
acc ggc agg cgg gaa ggt tgg cag cgt gga tgg cac ggc tac gac aac 1110
Thr Gly Arg Arg Glu Gly Trp Gln Arg Gly Trp His Gly Tyr Asp Asn
        345                 350                 355
gag ctc atg gac atg cgg ggc atc ttc ctg gct tcg gac ctg att tca 1158
Glu Leu Met Asp Met Arg Gly Ile Phe Leu Ala Ser Asp Leu Ile Ser
    360                 365                 370
aat cca act tca gag ctg ctc cta tca ggt cgg tgg acg tct aca atg 1206
Asn Pro Thr Ser Glu Leu Leu Leu Ser Gly Arg Trp Thr Ser Thr Met
375                 380                 385                 390
tca tgt gca atg tgg tgg gca tca ccc cgc tgc cca aca acg gat cct 1254
Ser Cys Ala Met Trp Trp Ala Ser Pro Arg Cys Pro Thr Thr Asp Pro
                395                 400                 405
ggt cca ggg tgatgtgcat gctgaagggc cgcgccggca ctgccccgcc 1303
Gly Pro Gly
tgtctggccc agccactgtg ccctggcact gattcttctc ttcctgcttg cataactgat 1363
catattgctt gtctcagaaa aaaacaccat cagcaaagtg ggcctccaaa gccagatgat 1423

tttcatttta tgtgtgaata atagcttcat taacacaatc aagaccatgc acattgtaaa 1483
tacattattc ttggataatt ctatacataa aagttcctac ttgttaaaaa aaaaaaaaaa 1543
aaaaaaa 1550

<210> 3
<211> 1227
<212> DNA
<213> Homo sapiens
<220>
<221> CDS
<222> (1)..(1227)
<400> 3
atg gca gtg aag ctt ggg acc ctc ctg ctg gcc ctt gcc ctg ggc ctg 48
Met Ala Val Lys Leu Gly Thr Leu Leu Leu Ala Leu Ala Leu Gly Leu
1               5                   10                  15
gcc cag cca gcc tct gcc cgc cgg aag ctg ctg gtg ttt ctg ctg gat 96
Ala Gln Pro Ala Ser Ala Arg Arg Lys Leu Leu Val Phe Leu Leu Asp
            20                  25                  30
ggt ttt cgc tca gac tac atc agt gat gag gcg ctg gag tca ttg cct 144
Gly Phe Arg Ser Asp Tyr Ile Ser Asp Glu Ala Leu Glu Ser Leu Pro
        35                  40                  45
ggt ttc aaa gag att gtg agc agg gga gta aaa gtg gat tac ttg act 192
Gly Phe Lys Glu Ile Val Ser Arg Gly Val Lys Val Asp Tyr Leu Thr
    50                  55                  60
cca gac ttc cct agt ctc tcg tat ccc aat tat tat acc cta atg act 240
Pro Asp Phe Pro Ser Leu Ser Tyr Pro Asn Tyr Tyr Thr Leu Met Thr
65                  70                  75                  80
ggc cgc cat tgt gaa gtc cat cag atg atc ggg aac tac atg tgg gac 288
Gly Arg His Cys Glu Val His Gln Met Ile Gly Asn Tyr Met Trp Asp
                85                  90                  95
ccc acc acc aac aag tcc ttt gac att ggc gtc aac aaa gac agc cta 336
Pro Thr Thr Asn Lys Ser Phe Asp Ile Gly Val Asn Lys Asp Ser Leu

            100                 105                 110
atg cct ctc tgg tgg aat gga tca gaa cct ctg tgg gtc act ctg acc 384
Met Pro Leu Trp Trp Asn Gly Ser Glu Pro Leu Trp Val Thr Leu Thr
        115                 120                 125
aag gcc aaa agg aag gtc tac atg tac tac tgg cca ggc tgt gag gtt 432
Lys Ala Lys Arg Lys Val Tyr Met Tyr Tyr Trp Pro Gly Cys Glu Val
    130                 135                 140
gag att ctg ggt gtc aga ccc acc tac tgc cta gaa tat aaa aat gtc 480
Glu Ile Leu Gly Val Arg Pro Thr Tyr Cys Leu Glu Tyr Lys Asn Val
145                 150                 155                 160
cca acg gat atc aat ttt gcc aat gca gtc agc gat gct ctt gac tcc 528
Pro Thr Asp Ile Asn Phe Ala Asn Ala Val Ser Asp Ala Leu Asp Ser
                165                 170                 175
ttc aag agt ggc cgg gcc gac ctg gca gcc ata tac cat gag cgc att 576
Phe Lys Ser Gly Arg Ala Asp Leu Ala Ala Ile Tyr His Glu Arg Ile
            180                 185                 190
gac gtg gaa ggc cac cac tac ggg cct gca tct ccg cag agg aaa gat 624
Asp Val Glu Gly His His Tyr Gly Pro Ala Ser Pro Gln Arg Lys Asp
        195                 200                 205
gcc ctc aag gct gta gac act gtc ctg aag tac atg acc aag tgg atc 672
Ala Leu Lys Ala Val Asp Thr Val Leu Lys Tyr Met Thr Lys Trp Ile
    210                 215                 220
cag gag cgg ggc ctg cag gac cgc ctg aac gtc att att ttc tcg gat 720
Gln Glu Arg Gly Leu Gln Asp Arg Leu Asn Val Ile Ile Phe Ser Asp
225                 230                 235                 240
cac gga atg acc gac att ttc tgg atg gac aaa gtg att gag ctg aat 768
His Gly Met Thr Asp Ile Phe Trp Met Asp Lys Val Ile Glu Leu Asn
                245                 250                 255
aag tac atc agc ctg aat gac ctg cag caa gtg aag gac cgc ggg cct 816
Lys Tyr Ile Ser Leu Asn Asp Leu Gln Gln Val Lys Asp Arg Gly Pro
            260                 265                 270
gtt gtg agc ctt tgg ccg gcc cct ggg aaa cac tct gag ata tat aac 864

Val Val Ser Leu Trp Pro Ala Pro Gly Lys His Ser Glu Ile Tyr Asn
        275                 280                 285
aaa ctg agc aca gtg gaa cac atg act gtc tac gag aaa gaa gcc atc 912
Lys Leu Ser Thr Val Glu His Met Thr Val Tyr Glu Lys Glu Ala Ile
    290                 295                 300
cca agc agg ttc tat tac aag aaa gga aag ttt gtc tct cct ttg act 960
Pro Ser Arg Phe Tyr Tyr Lys Lys Gly Lys Phe Val Ser Pro Leu Thr
305                 310                 315                 320
tta gtg gct gat gaa ggc tgg ttc ata act gag aat cga gag atg ctt 1008
Leu Val Ala Asp Glu Gly Trp Phe Ile Thr Glu Asn Arg Glu Met Leu
                325                 330                 335
ccg ttt tgg atg aac agc acc ggc agg cgg gaa ggt tgg cag cgt gga 1056
Pro Phe Trp Met Asn Ser Thr Gly Arg Arg Glu Gly Trp Gln Arg Gly
            340                 345                 350
tgg cac ggc tac gac aac gag ctc atg gac atg cgg ggc atc ttc ctg 1104
Trp His Gly Tyr Asp Asn Glu Leu Met Asp Met Arg Gly Ile Phe Leu
        355                 360                 365
gct tcg gac ctg att tca aat cca act tca gag ctg ctc cta tca ggt 1152
Ala Ser Asp Leu IlESer Asn Pro Thr Ser Glu Leu Leu Leu Ser Gly
370 375 380
cgg tgg acg tct aca atg tca tgt gca atg tgg tgg gca tca ccc cgc 1200
Arg Trp Thr Ser Thr Met Ser Cys Ala Met Trp Trp Ala Ser Pro Arg
385 390 395 400
tgc cca aca acg gat cct ggt cca ggg 1227
Cys Pro Thr Thr Asp Pro Gly Pro Gly
405

<210> 4
<211> 20
<212> DNA
<213> Artificial Sequence
<220>

<223> forward primer for amplification of TRDH-091 DNA
<400> 4
acagctgtgc cagctcactc                                                    20

<210> 5
<211> 22
<212> DNA
<213> Artificial Sequence
<220>
<223> reverse primer for amplification of TRDH-091 DNA
<400> 5
aggcaataag ttggtctgac ac                                                 22

<210> 6
<211> 19
<212> DNA
<213> Artificial Sequence
<220>
<223> forward primer for amplification of G3PDH DNA
<400> 6
atcaccatct tccaggagc                                                     19

<210> 7
<211> 21
<212> DNA
<213> Artificial Sequence
<220>
<223> reverse primer for amplification of G3PDH DNA
<400> 7
caccttcttg atgtcatcat a                                                  21

<210> 8

<211> 19
<212> DNA
<213> Artificial Sequence
<220>
<223> forward primer for amplification of rat OP-1 DNA
<400> 8
cacagcttcg tggcgctct                                                    19

<210> 9
<211> 21
<212> DNA
<213> Artificial Sequence
<220>
<223> reverse primer for amplification of rat OP-1 DNA
<400> 9
aacttggggt tgatgctctg c                                                 21

<210> 10
<211> 23
<212> DNA
<213> Artificial Sequence
<220>
<223> forward primer for amplification of 5' region of full-length TRDH-
091 cDNA
<400> 10
aattaaccct cactaaaggg aac                                               23

<210> 11
<211> 22
<212> DNA
<213> Artificial Sequence
<220>

<223> reverse primer for amplification of 3' region of full-length TRDH-
091 cDNA
<400> 11
gtaatacgac tcactatagg gc                                                22

<210> 12
<211> 66
<212> DNA
<213> Artificial Sequence
<220>
<223> primer with T7 promoter and poly thymidylate Sequence
<400> 12
aaacgacggc cagtgaattg taatacgact cactataggg cgtttttttt tttttttttt 60
tttttt                                                                  66

<210> 13
<211> 423
<212> DNA
<213> Homo sapiens
<220>
<221> CDS
<400> 13
gattgtgtta atgaagctat tattcacaca taaaatgaaa atcatctggc tttggaggcc 60
cactttgctg atggtgtttt tttctgagac aagcaatatg atcagttatg caagcaggaa 120
gagaagaatc agtgccaggg caangtcggc tcgggcagac aggcggggca gtgccggcgc 180
ggccttcagc atgcacatca ccctggacca ggatccgttg ttgggcagcg gggtgatgcc 240
caccacattg cacatgacat tgtagacgtc caccgacctg ataggagcag ctctgaagtt 300
ggatttgaaa tcaggtccga agccaaggaa gatgccccgc atgtccatcg agctcgttgt 360
cgtagccgtg ccatccacgc tgccaacctt cccgcctgcc ggtgctgttc atccaaaacg 420
gaa                                                                     423

<210> 14

<211> 404
<212> DNA
<213> Homo sapiens
<220>
<221> CDS
<400> 14
acattggcgt caacaaagac agcctaatgc ctctctggtg gaatggatca gaacctctgt 60
gggtcactct gaccaaggcc aaaaggaagg tctacatgta ctactggcca ggctgtgagg 120
ttgagattct gggtgtcaga cccacctact gcctagaata taaaaatgtc ccaacnngat 180
atcaattttn ccaatgcagt cagcgatgct cttgactcct tcaagagtng ccgggccgac 240
ctggcagcca tataccatga gcgcattgac gtggaaggcc accactaccg ggcctgcatc 300
tccgcagagg aaagatgccc tcaaggctgg tagacactgt cctgaagtac atgaccaagt 360
ggatccagga gcggggcctg caggaccgcc tgaacgtcat tatt 404

<210> 15
<211> 395
<212> PRT
<213> Rattus norvegicus
<400> 15
Met Ala Gly Lys Leu Trp Thr Phe Leu Leu Leu Phe Gly Phe Ser Trp
1               5                   10                  15
Val Trp Pro Ala Ser Ala His Arg Lys Leu Leu Val Leu Leu Leu Asp
            20                  25                  30
Gly Phe Arg Ser Asp Tyr Ile Ser Glu Asp Ala Leu Ala Ser Leu Pro
        35                  40                  45
Gly Phe Arg Glu Ile Val Asn Arg Gly Val Lys Val Asp Tyr Leu Thr
    50                  55                  60
Pro Asp Phe Pro Ser Leu Ser Tyr Pro Asn Tyr Tyr Thr Leu Met Thr
65                  70                  75                  80
Gly Arg His Cys Glu Val His Gln Met Ile Gly Asn Tyr Met Trp Asp
                85                  90                  95
Pro Arg Thr Asn Lys Ser Phe Asp Ile Gly Val Asn Arg Asp Ser Leu

            100                 105                 110
Met Pro Leu Trp Trp Asn Gly Ser Glu Pro Leu Trp Ile Thr Leu Met
        115                 120                 125
Lys Ala Arg Arg Lys Val Tyr Met Tyr Tyr Trp Pro Gly Cys Glu Val
    130                 135                 140
Glu Ile Leu Gly Val Arg Pro Thr Tyr Cys Leu Glu Tyr Lys Asn Val
145                 150                 155                 160
Pro Thr Asp Ile Asn Phe Ala Asn Ala Val Ser Asp Ala Leu Asp Ser
                165                 170                 175
Leu Lys Ser Gly Arg Ala Asp Leu Ala Ala Ile Tyr His Glu Arg Ile
            180                 185                 190
Asp Val Glu Gly His His Tyr Gly Pro Ser Ser Pro Gln Arg Lys Asp
        195                 200                 205
Ala Leu Lys Ala Val Asp Thr Val Leu Lys Tyr Met Thr Gln Trp Ile
    210                 215                 220
Gln Glu Arg Gly Leu Gln Asn Asp Leu Asn Val Ile Leu Phe Ser Asp
225                 230                 235                 240
His Gly Met Thr Asp Ile Phe Trp Met Asp Lys Val Ile Glu Leu Ser
                245                 250                 255
Lys Tyr Ile Ser Leu Asp Asp Leu Gln Gln Val Lys Asp Gln Gly Pro
            260                 265                 270
Val Val Ser Leu Trp Pro Val Pro Glu Lys His Ser Glu Ile Tyr His
        275                 280                 285
Lys Leu Arg Thr Val Glu His Met Thr Val Tyr Glu Lys Glu Ala Ile
    290                 295                 300
Pro Asn Arg Phe Tyr Tyr Lys Lys Gly Lys Phe Val Ser Pro Leu Thr
305                 310                 315                 320
Leu Val Ala Asp Glu Gly Trp Phe Ile Ala Glu Ser Arg Glu Ala Leu
                325                 330                 335
Pro Phe Trp Met Asn Ser Thr Gly Lys Arg Glu Gly Trp Gln His Gly
            340                 345                 350
Trp His Gly Tyr Asp Asn Glu Leu Met Asp Met Arg Gly Ile Phe Leu

355                 360                 365
Ala Ser Asp Leu Ile Ser Ser Arg Thr Ser Glu Leu Leu Gln Ser Asp
    370                 375                 380
Leu Trp Met Ser Thr Thr Ser Cys Val Met Ser
385                 390                 395

<210> 16
<211> 1526
<212> DNA
<213> Rattus norvegicus
<220>
<221> CDS
<222> (57)..(1241)
<400> 16
ctcagtcttt gcaagggcac agctgtgcca gctcactcca gcagactcct ggcagc atg 59
                                                              Met
                                                              1
gca gga aag ctc tgg acc ttc ctg ctg ctg ttt ggg ttc agc tgg gtt    107
Ala Gly Lys Leu Trp Thr Phe Leu Leu Leu Phe Gly Phe Ser Trp Val
            5                   10                  15
tgg cca gct tct gcc cac cgg aag ctc ctg gtg ttg ctc ctg gat ggt    155
Trp Pro Ala Ser Ala His Arg Lys Leu Leu Val Leu Leu Leu Asp Gly
        20                  25                  30
ttt cgc tca gac tac atc agt gag gat gcc ctg gca tcc ttg cct ggt    203
Phe Arg Ser Asp Tyr Ile Ser Glu Asp Ala Leu Ala Ser Leu Pro Gly
    35                  40                  45
ttc aga gag atc gtg aac aga gga gtc aaa gtg gat tac ttg act cca    251
Phe Arg Glu Ile Val Asn Arg Gly Val Lys Val Asp Tyr Leu Thr Pro
50                  55                  60                  65
gac ttc ccc agc ctc tcc tat ccc aat tac tac acc ctc atg acc ggc    299
Asp Phe Pro Ser Leu Ser Tyr Pro Asn Tyr Tyr Thr Leu Met Thr Gly
                70                  75                  80

cgc cac tgt gag gtc cac cag atg atc ggg aac tac atg tgg gat ccc 347
Arg His Cys Glu Val His Gln Met Ile Gly Asn Tyr Met Trp Asp Pro
            85                  90                  95
aga acc aac aag tca ttc gac atc ggg gtc aac cga gac agc ctg atg 395
Arg Thr Asn Lys Ser Phe Asp Ile Gly Val Asn Arg Asp Ser Leu Met
        100                 105                 110
ccc ctc tgg tgg aat ggg tca gaa ccc ttg tgg atc act ctg atg aaa 443
Pro Leu Trp Trp Asn Gly Ser Glu Pro Leu Trp Ile Thr Leu Met Lys
    115                 120                 125
gcc agg agg aag gtc tac atg tac tac tgg cca ggc tgt gaa gtt gag 491
Ala Arg Arg Lys Val Tyr Met Tyr Tyr Trp Pro Gly Cys Glu Val Glu
130                 135                 140                 145
att ctt ggt gtc aga cca act tat tgc cta gaa tac aaa aat gtc cca 539
Ile Leu Gly Val Arg Pro Thr Tyr Cys Leu Glu Tyr Lys Asn Val Pro
                150                 155                 160
aca gac atc aac ttt gcg aat gca gtt agc gat gct ctc gac tca tta 587
Thr Asp Ile Asn Phe Ala Asn Ala Val Ser Asp Ala Leu Asp Ser Leu
            165                 170                 175
aag agt ggc cga gcg gat cta gca gcc ata tac cac gaa cgc att gat 635
Lys Ser Gly Arg Ala Asp Leu Ala Ala Ile Tyr His Glu Arg Ile Asp
        180                 185                 190
gta gaa ggc cat cac tat ggc ccc tca tca cct cag aga aaa gat gct 683
Val Glu Gly His His Tyr Gly Pro Ser Ser Pro Gln Arg Lys Asp Ala
    195                 200                 205
ctc aaa gct gtg gac act gtc ctg aag tat atg acc cag tgg att cag 731
Leu Lys Ala Val Asp Thr Val Leu Lys Tyr Met Thr Gln Trp Ile Gln
210                 215                 220                 225
gaa cga ggc ttg cag aat gac cta aac gtc atc ctt ttc tca gac cat 779
Glu Arg Gly Leu Gln Asn Asp Leu Asn Val Ile Leu Phe Ser Asp His
                230                 235                 240
ggg atg act gac atc ttc tgg atg gat aaa gtg att gag ttg agc aaa 827
Gly Met Thr Asp Ile Phe Trp Met Asp Lys Val Ile Glu Leu Ser Lys

            245                 250                 255
tac atc agc ctg gat gac ctg cag caa gtg aag gac caa ggg ccc gtt 875
Tyr Ile Ser Leu Asp Asp Leu Gln Gln Val Lys Asp Gln Gly Pro Val
        260                 265                 270
gtg agc ctg tgg ccc gtc cca gaa aaa cac tct gag ata tat cac aaa 923
Val Ser Leu Trp Pro Val Pro Glu Lys His Ser Glu Ile Tyr His Lys
    275                 280                 285
ctc cgc acc gta gaa cac atg aca gtg tat gag aaa gaa gca ata ccc 971
Leu Arg Thr Val Glu His Met Thr Val Tyr Glu Lys Glu Ala Ile Pro
290                 295                 300                 305
aac agg ttc tat tac aag aaa ggg aaa ttt gtc tct cct ttg acc ttg 1019
Asn Arg Phe Tyr Tyr Lys Lys Gly Lys Phe Val Ser Pro Leu Thr Leu
                310                 315                 320
gtg gct gat gaa ggg tgg ttc ata gca gag agt cga gag gcg ctt ccg 1067
Val Ala Asp Glu Gly Trp Phe Ile Ala Glu Ser Arg Glu Ala Leu Pro
            325                 330                 335
ttt tgg atg aac agc acc ggc aag cgc gaa ggc tgg cag cac gga tgg 1115
Phe Trp Met Asn Ser Thr Gly Lys Arg Glu Gly Trp Gln His Gly Trp
        340                 345                 350
cat gga tat gac aac gag ctc atg gac atg aga ggg atc ttc ctg gct 1163
His Gly Tyr Asp Asn Glu Leu Met Asp Met Arg Gly Ile Phe Leu Ala
    355                 360                 365
tcg gac ctg att tca agt cga act tca gag ctg ctc caa tca gat ctg 1211
Ser Asp Leu Ile Ser Ser Arg Thr Ser Glu Leu Leu Gln Ser Asp Leu
370                 375                 380                 385
tgg atg tct aca aca tca tgt gta atg tcg taggcatcac cccactgccc 1261
Trp Met Ser Thr Thr Ser Cys Val Met Ser
                390                 395
aacaatgggt cctggtccag ggtggtatgc atgctgaaga gtcaaaccag ctcgtctcca 1321
tccatcccgc cgaacagttg tgcgctggtc ttgattctcc tcttatactt tgtatagctc 1381
gccatagggc tcattccaaa gcactcctcc aacatggagt agttttcatt ttccttatga 1441
ataatagctc tattaacaca atcaaggcca ttaaagttgt aaacatatta ttcttggatg 1501

atcctaaaaa aaaaaaaaaa aaaaa 1526

<210> 17
<211> 1185
<212> DNA
<213> Rattus norvegicus
<220>
<221> CDS
<222> (1)..(1185)
<400> 17
atg gca gga aag ctc tgg acc ttc ctg ctg ctg ttt ggg ttc agc tgg 48
Met Ala Gly Lys Leu Trp Thr Phe Leu Leu Leu Phe Gly Phe Ser Trp
1               5                   10                  15
gtt tgg cca gct tct gcc cac cgg aag ctc ctg gtg ttg ctc ctg gat 96
Val Trp Pro Ala Ser Ala His Arg Lys Leu Leu Val Leu Leu Leu Asp
            20                  25                  30
ggt ttt cgc tca gac tac atc agt gag gat gcc ctg gca tcc ttg cct 144
Gly Phe Arg Ser Asp Tyr Ile Ser Glu Asp Ala Leu Ala Ser Leu Pro
        35                  40                  45
ggt ttc aga gag atc gtg aac aga gga gtc aaa gtg gat tac ttg act 192
Gly Phe Arg Glu Ile Val Asn Arg Gly Val Lys Val Asp Tyr Leu Thr
    50                  55                  60
cca gac ttc ccc agc ctc tcc tat ccc aat tac tac acc ctc atg acc 240
Pro Asp Phe Pro Ser Leu Ser Tyr Pro Asn Tyr Tyr Thr Leu Met Thr
65                  70                  75                  80
ggc cgc cac tgt gag gtc cac cag atg atc ggg aac tac atg tgg gat 288
Gly Arg His Cys Glu Val His Gln Met Ile Gly Asn Tyr Met Trp Asp
                85                  90                  95
ccc aga acc aac aag tca ttc gac atc ggg gtc aac cga gac agc ctg 336
Pro Arg Thr Asn Lys Ser Phe Asp Ile Gly Val Asn Arg Asp Ser Leu
            100                 105                 110
atg ccc ctc tgg tgg aat ggg tca gaa ccc ttg tgg atc act ctg atg 384

Met Pro Leu Trp Trp Asn Gly Ser Glu Pro Leu Trp Ile Thr Leu Met
        115                 120                 125
aaa gcc agg agg aag gtc tac atg tac tac tgg cca ggc tgt gaa gtt 432
Lys Ala Arg Arg Lys Val Tyr Met Tyr Tyr Trp Pro Gly Cys Glu Val
    130                 135                 140
gag att ctt ggt gtc aga cca act tat tgc cta gaa tac aaa aat gtc 480
Glu Ile Leu Gly Val Arg Pro Thr Tyr Cys Leu Glu Tyr Lys Asn Val
145                 150                 155                 160
cca aca gac atc aac ttt gcg aat gca gtt agc gat gct ctc gac tca 528
Pro Thr Asp Ile Asn Phe Ala Asn Ala Val Ser Asp Ala Leu Asp Ser
                165                 170                 175
tta aag agt ggc cga gcg gat cta gca gcc ata tac cac gaa cgc att 576
Leu Lys Ser Gly Arg Ala Asp Leu Ala Ala Ile Tyr His Glu Arg Ile
            180                 185                 190
gat gta gaa ggc cat cac tat ggc ccc tca tca cct cag aga aaa gat 624
Asp Val Glu Gly His His Tyr Gly Pro Ser Ser Pro Gln Arg Lys Asp
        195                 200                 205
gct ctc aaa gct gtg gac act gtc ctg aag tat atg acc cag tgg att 672
Ala Leu Lys Ala Val Asp Thr Val Leu Lys Tyr Met Thr Gln Trp Ile
    210                 215                 220
cag gaa cga ggc ttg cag aat gac cta aac gtc atc ctt ttc tca gac 720
Gln Glu Arg Gly Leu Gln Asn Asp Leu Asn Val Ile Leu Phe Ser Asp
225                 230                 235                 240
cat ggg atg act gac atc ttc tgg atg gat aaa gtg att gag ttg agc 768
His Gly Met Thr Asp Ile Phe Trp Met Asp Lys Val Ile Glu Leu Ser
                245                 250                 255
aaa tac atc agc ctg gat gac ctg cag caa gtg aag gac caa ggg ccc 816
Lys Tyr Ile Ser Leu Asp Asp Leu Gln Gln Val Lys Asp Gln Gly Pro
            260                 265                 270
gtt gtg agc ctg tgg ccc gtc cca gaa aaa cac tct gag ata tat cac 864
Val Val Ser Leu Trp Pro Val Pro Glu Lys His Ser Glu Ile Tyr His
        275                 280                 285

aaa ctc cgc acc gta gaa cac atg aca gtg tat gag aaa gaa gca ata 912
Lys Leu Arg Thr Val Glu His Met Thr Val Tyr Glu Lys Glu Ala Ile
    290                 295                 300
ccc aac agg ttc tat tac aag aaa ggg aaa ttt gtc tct cct ttg acc 960
Pro Asn Arg Phe Tyr Tyr Lys Lys Gly Lys Phe Val Ser Pro Leu Thr
305                 310                 315                 320
ttg gtg gct gat gaa ggg tgg ttc ata gca gag agt cga gag gcg ctt 1008
Leu Val Ala Asp Glu Gly Trp Phe Ile Ala Glu Ser Arg Glu Ala Leu
                325                 330                 335
ccg ttt tgg atg aac agc acc ggc aag cgc gaa ggc tgg cag cac gga 1056
Pro Phe Trp Met Asn Ser Thr Gly Lys Arg Glu Gly Trp Gln His Gly
            340                 345                 350
tgg cat gga tat gac aac gag ctc atg gac atg aga ggg atc ttc ctg 1104
Trp His Gly Tyr Asp Asn Glu Leu Met Asp Met Arg Gly Ile Phe Leu
        355                 360                 365
gct tcg gac ctg att tca agt cga act tca gag ctg ctc caa tca gat 1152
Ala Ser Asp Leu Ile Ser Ser Arg Thr Ser Glu Leu Leu Gln Ser Asp
    370                 375                 380
ctg tgg atg tct aca aca tca tgt gta atg tcg 1185
Leu Trp Met Ser Thr Thr Ser Cys Val Met Ser
385                 390                 395

<210> 18
<211> 20
<212> DNA
<213> Artificial Sequence
<220>
<223> forward primer for amplification of human KRGF-1 DNA
<400> 18
ccaggctgtg aggttgagat 20

<210> 19

<211> 20
<212> DNA
<213> Artificial Sequence
<220>
<223> reverse primer for amplification of human KRGF-1 DNA
<400> 19
gtgctgttca tccaaaacgg                                                    20

<210> 20
<211> 17
<212> PRT
<213> Artificial Sequence
<220>
<221> ACETYLATION
<222> (17)
<220>
<223> a peptide consisting of 126-142 of amino acid sequence of rat KRGF-1
and acetylated at its N-terminal.
<400> 20
Thr Leu Met Lys Ala Arg Arg Lys Val Tyr Met Tyr Tyr Trp Pro Gly
 1               5                  10                  15

Cys

<210> 21
<211> 20
<212> PRT
<213> Artificial Sequence
<220>
<221> AMIDATION
<222> (1)
<220>
<223> a peptide consisting of N-terminal added cystein and 298-316 of amino
acid sequence of rat KRGF-1 and amidated at its C-terminal.
<400> 21
Cys Val Tyr Glu Lys Glu Ala Ile Pro Asn Arg Phe Tyr Tyr Lys Lys
 1               5                  10                  15

Gly Lys Phe Val

<210> 22
<211> 20
<212> PRT
<213> Artificial Sequence
<220>
<221> AMIDATION
<222> (1)
<220>
<223> a peptide consisting of N-terminal added cystein and 341-359 of amino
acid sequence of rat KRGF-1 and amidated at its C-terminal.
<400> 22
Cys Asn Ser Thr Gly Lys Arg Glu Gly Trp Gln His Gly Trp His Gly
 1               5                   10                  15
Tyr Asp Asn Glu

<210> 23
<211> 27
<212> DNA
<213> Artificial Sequence
<220>
<223> a forward primer for amplification of a coding sequence of rat mature
KRGF and creation of an EcoRI site at its 5' end.
<400> 23
aaagaattcc accggaagct cctggtg                                          27

<210> 24
<211> 31
<212> DNA
<213> Artificial Sequence
<220>
<223> a reverse primer for amplification of a coding sequence of rat mature
KRGF and creation of a HindIII site at its 3' end.
<400> 24
aaatttaagc ttcacgacat tacacatgat g                                     31

<210> 25

<211> 33
<212> DNA
<213> Artificial Sequence
<220>
<223> a forward primer for amplification of a coding sequence of rat KRGF
with its signal peptide and creation of an EcoRI site and Kozak sequence at
its 5' end.
<400> 25
aaagaattcg ccaccatggc aggaaagctc tgg                                    33

<210> 26
<211> 30
<212> DNA
<213> Artificial Sequence
<220>
<223> a reverse primer for amplification of a coding sequence of rat KRGF
with its signal peptide and creation of a HindIII site at its 3' end.
<400> 26
aaatttaagc ttgcgacatt acacatgatg                                        30